# FASCICULE DE BREVET EUROPEEN

(11) **EP 1 495 023 B1**
(45) Date de publication et mention de la délivrance du brevet: **07.09.2005**
(21) Numéro de dépôt: 03730302.1
(22) Date de dépôt: 02.04.2003
(51) Int. Cl.: C07D 471/04, A61K 31/437, A61P 35/00, A61P 9/00, A61P 29/00

(54) **NOUVEAUX DERIVES D INDOLIZINE 1,2,3 SUBSTITUEE, INHIBITEURS DES FGFs, LEUR PROCEDE DE PREPARATION ET LES COMPOSITIONS PHARMACEUTIQUES LES CONTENANT**
1,2,3-SUBSTITUIERTE INDOLIZINDERIVATE, FGF INHIBITOREN, VERFAHREN ZU IHRER HERSTELLUNG UND DIESE ENTHALTENDE PHARMAZEUTISCHE ZUBEREITUNGEN
NOVEL 1,2,3-SUBSTITUTED INDOLIZINE DERIVATIVES, INHIBITORS OF FGFS, METHOD FOR MAKING SAME AND PHARMACEUTICAL COMPOSITIONS CONTAINING SAME

(30) Priorité: 04.04.2002 FR 0204220
(43) Date de publication de la demande: 12.01.2005
(73) Titulaire: Sanofi-Aventis, 75013 Paris (FR)
(72) Inventeur: BADORC, Alain, F-31120 Roquettes (FR); BONO, Françoise, F-31300 Toulouse (FR); BORDES, Marie-Françoise, F-31860 Labarthe sur Leze (FR); GUILLO, Nathalie, F-31000 Toulouse (FR); HERBERT, Jean-Marc, F-31170 Tournefeuille (FR)
(74) Mandataire: Tsitini-Souleau, Maria
(86) Numéro de dépôt international: PCT/FR2003/001030
(87) Numéro de publication internationale: WO 2003/084956

(56) Documents cités:
- EP-A- 0 097 636
- EP-A- 0 235 111
- WO-A-00/71129

## Description

La présente invention a pour objet de nouveaux dérivés d'indolizine 1,2,3 substituée, qui sont des inhibiteurs des FGFs (basic Fibroblast Growth factor), leur procédé de préparation et les compositions pharmaceutiques les contenant.

Les FGF sont une famille de polypeptides synthétisés par un grand nombre de cellules lors du développement embryonnaire et par des cellules des tissus adultes dans diverses conditions pathologiques.

On connaît certains dérivés de naphtyridine diamines et des urées correspondantes qui sont des inhibiteurs sélectifs de FGF-1 (Batley B. et al., *Life Sciences*, (1998), vol 62 n°2, pp143-150 ; Thompson A. et al., *J. Med. Chem*., (2000), vol 43, pp4200-4211).

Certains dérivés d'indolizine sont décrits dans les demandes de brevets et brevets US 4 378 362, FR 2 341 578, GB 2 064 536, EP 0 097 636, EP 302 792, EP 0 382 628, et EP 0 235 111. Ces composés sont utiles dans le traitement de l'angine de poitrine et de l'arythmie. Des propriétés inhibitrices de la translocation calcique sont décrites pour certains de ces composés.

La demande de brevet EP 0 022 762 décrit également certains dérivés d'indolizine qui possèdent une activité inhibitrice de la xanthine oxydase et de l'adénosine désaminase ainsi qu'une activité uricosurique. Ces composés peuvent être utilisés dans le traitement des désordres physiologiques consécutifs à un excès d'acide urique, des perturbations du système immunitaire et aux agents parasitaires.

Il a été maintenant trouvé que certains composés, dérivés d'indolizine, sont des puissants antagonistes de la liaison des FGFs à ses récepteurs.

Ainsi, la présente invention a pour objet des nouveaux dérivés d'indolizine de formule I, 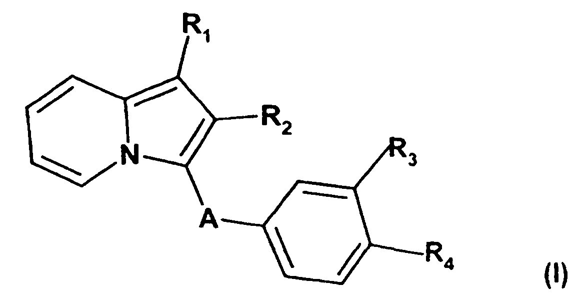 dans laquelle
- **R**_{**1**} représente un radical hydroxy, un radical alcoxy linéaire ou ramifié de 1 à 5 atomes de carbone, un radical carboxy, un radical alcoxycarbonyle de 2 à 6 atomes de carbone ou un radical de formule :
   - -NR₅R₆
   - -NH-SO₂-Alk
   - -NH-SO₂-Ph
   - -NH-CO-Ph
   - -N(Alk)-CO-Ph
   - -NH-CO-NH-Ph
   - -NH-CO-Alk
   - -NN-CO₂-Alk
   - -O-(CH₂)ₙ-cAlk
   - -O-Alk-COOR₇
   - -O-Alk-O-R₈
   - -O-Alk-OH
   - -O-Alk-C(NH₂):NOH
   - -O-Alk-NR₅R₆
   - -O-Alk-CN
   - -O-(CH₂)ₙ-Ph
   - -O-Alk-CO-NR₅R₆
   - -CO-NH-(CH₂)ₘ-COOR₇
   - -CO-NH-Alk
   dans lesquelles
   - Alk représente un radical alkyle ou un radical alkylène linéaire ou ramifié de 1 à 5 atomes de carbone,
   - cAlk représente un radical cycloalkyle de 3 à 6 atomes de carbone,
   - n représente un nombre entier de 0 à 5,
   - m représente un nombre entier de 1 à 5,
   - R₅ et R₆ identiques ou différents représentent chacun un atome d'hydrogène, un radical alkyle linéaire ou ramifié de 1 à 5 atomes de carbone ou un radical benzyle,
   - R₇ représente un atome d'hydrogène ou un radical alkyle de 1 à 5 atomes de carbone,
   - R₈ représente un radical alkyle de 1 à 5 atomes de carbone ou un radical -CO-Alk,
   - Ph représente un radical phényle éventuellement substitué par un ou plusieurs atomes d'halogène, par un ou plusieurs radicaux alcoxy de 1 à 5 atomes de carbone, par un ou plusieurs radicaux carboxy ou par un ou plusieurs radicaux alcoxycarbonyle de 2 à 6 atomes de carbone,
- **R**_{**2**} représente un atome d'hydrogène, un radical alkyle de 1 à 5 atomes de carbone, un radical d'halogenure d'alkyle de 1 à 5 atomes de carbone comportant 3 à 5 atomes d'halogène, un radical cycloalkyle de 3 à 6 atomes de carbone ou un radical phényle éventuellement substitué par un ou plusieurs atomes d'halogène, par un ou plusieurs radicaux alcoxy de 1 à 5 atomes de carbone, un ou plusieurs radicaux carboxy ou par un ou plusieurs radicaux alcoxycarbonyle de 2 à 6 atomes de carbone,
- **A** représente un radical -CO-, -SO- ou -SO₂-,
- **R**_{**3**} et **R**_{**4**} identiques ou différents représentent chacun un atome d'hydrogène, un radical alcoxy de 1 à 5 atomes de carbone, un radical amino, un radical carboxy, un radical alcoxycarbonyle de 2 à 6 atomes de carbone, un radical hydroxy, un radical nitro, un radical hydroxyamino, un radical de formule
   - -Alk-COOR₇
   - -NR₅R₆
   - -NH-Alk-COOR₇
   - -NH-COO-Alk
   - -N(R₁₁)-SO₂-Alk-NR₉R₁₀
   - -N(R₁₁)-SO₂-Alk
   - -N(R₁₁)-Alk-NR₅R₆
   - -N(R₁₁)-CO-Alk-NR₉R₁₀
   - -N(R₁₁)-CO-Alk
   - -N(R₁₁)-CO-CF₃
   - -NH-Alk-HetN
   - -O-Alk-NR₉R₁₀
   - -O-Alk-CO-NR₅R₆
   - -O-Alk-HetN
   dans lesquels n, m, Alk, R₅, R₆, et R₇, ont la signification donnée précédemment pour R₁ et
   - R₉ et R₁₀ identiques ou différents représentent chacun un atome d'hydrogène ou un radical alkyle de 1 à 5 atomes de carbone,
   - R₁₁ représente un atome d'hydrogène ou un radical -Alk-COOR₁₂ où R₁₂ représente un atome d'hydrogène, un radical alkyle de 1 à 5 atomes de carbone ou un radical benzyle,
   - HetN représente un hétérocycle à 5 ou 6 chaînons comportant au moins un atome d'azote et éventuellement un autre hétéroatome choisi parmi l'azote et l'oxygène
ou R₃ et R₄ forment ensemble un hétérocycle insaturé de 5 à 6 chaînons, à condition toutefois que lorsque R₃ représente un radical alcoxy et R₄ représente un radical -O-Alk-NR₉R₁₀ ou un radical hydroxy, R₁ ne représente pas un atome d'hydrogène ou un radical alcoxy,
éventuellement sous la forme de l'un de leurs sels pharmaceutiquement acceptables.

On préfère un composé de formule I dans laquelle
- **R**_{**1**} représente un radical hydroxy, un radical alcoxy linéaire ou ramifié de 1 à 5 atomes de carbone, un radical carboxy, un radical alcoxycarbonyle de 2 à 6 atomes de carbone ou un radical de formule :
   - -NR₅R₆
   - -NH-SO₂-Alk
   - -NH-SO₂-Ph
   - -NH-CO-Ph
   - -N(Alk)-CO-Ph
   - -NH-CO-NH-Ph
   - -NH-CO-Alk
   - -NH-CO₂-Alk
   - -O-(CH₂)ₙ-cAlk
   - -O-Alk-COOR₇
   - -O-Alk-O-R₈
   - -O-Alk-OH
   - -O-Alk-NR₅R₆
   - -O-Alk-CN
   - -O-(CH₂)ₙ-Ph
   - -O-Alk-CO-NR₅R₆
   - -CO-NH-(CH₂)ₘ-COOR₇
   - -CO-NH-Alk
   dans lesquelles
   - Alk représente un radical alkyle ou un radical alkylène linéaire ou ramifié de 1 à 5 atomes de carbone,
   - cAlk représente un radical cycloalkyle de 3 à 6 atomes de carbone,
   - n représente un nombre entier de 0 à 5,
   - m représente un nombre entier de 1 à 5,
   - R₅ et R₆ identiques ou différents représentent chacun un atome d'hydrogène, un radical alkyle linéaire ou ramifié de 1 à 5 atomes de carbone ou un radical benzyle,
   - R₇ représente un atome d'hydrogène ou un radical alkyle de 1 à 5 atomes de carbone,
   - R₈ représente un radical alkyle de 1 à 5 atomes de carbone ou un radical -CO-Alk
   - Ph représente un radical phényle éventuellement substitué par un ou plusieurs atomes d'halogène, par un ou plusieurs radicaux alcoxy de 1 à 5 atomes de carbone, un ou plusieurs radicaux carboxy ou par un ou plusieurs radicaux alcoxycarbonyle de 2 à 6 atomes de carbone,
- **R**_{**2**} représente un radical alkyle de 1 à 5 atomes de carbone, un radical trifluorométhyle, un radical cycloalkyle de 3 à 6 atomes de carbone ou un radical phényle éventuellement substitué par un ou plusieurs atomes d'halogène, par un ou plusieurs radicaux alcoxy de 1 à 5 atomes de carbone, par un ou plusieurs radicaux carboxy ou par un ou plusieurs radicaux alcoxycarbonyle de 2 à 6 atomes de carbone,
- **A** représente un radical -CO-, -SO₂-,
- **R**_{**3**} et **R**_{**4**} identiques ou différents représentent chacun un atome d'hydrogène, un radical alcoxy de 1 à 5 atomes de carbone, un radical amino, un radical carboxy, un radical alcoxycarbonyle de 2 à 6 atomes de carbone, un radical nitro, un radical hydroxyamino, un radical de formule
   - -Alk-COOR₇
   - -NR₅R₆
   - -NH-Alk-COOR₇
   - -NH-COO-Alk
   - -N(R₁₁)-SO₂-Alk-NR₉R₁₀
   - -N(R₁₁)-SO₂-Alk
   - -N(R₁₁)-Alk-NR₅R₆
   - -N(R₁₁)-CO-Alk-NR₉R₁₀
   - -N(R₁₁)-CO-Alk
   - -N(R₁₁)-CO-CF₃
   - -NH-Alk-HetN
   dans lesquels n, m, Alk, R₅, R₆, et R₇, ont la signification donnée précédemment pour R₁ et
   - R₉ et R₁₀ identiques ou différents représentent chacun un atome d'hydrogène ou un radical alkyle de 1 à 5 atomes de carbone,
   - R₁₁ représente un atome d'hydrogène ou un radical -Alk-COOR₁₂ où R₁₂ représente un atome d'hydrogène, un radical alkyle de 1 à 5 atomes de carbone ou un radical benzyle,
   - HetN représente un hétérocycle à 5 ou 6 chaînons comportant au moins un atome d'azote et éventuellement un autre hétéroatome choisi parmi l'azote et l'oxygène
éventuellement sous la forme de l'un de leurs sels pharmaceutiquement acceptables.

On préfère en particulier un composé de formule I dans laquelle
- **R**_{**1**} représente un radical alcoxy de 1 à 5 atomes de carbone, un radical carboxy, un radical -O-Alk-COOH dans laquelle Alk représente un radical alkylène linéaire ou ramifié de 1 à 5 atomes de carbone, un radical de formule -O-Alk-Ph dans laquelle Alk représente un radical alkylène de 1 à 5 atomes de carbone et Ph représente un radical phényle éventuellement substitué par un ou plusieurs atomes d'halogène ou par un ou plusieurs radicaux alcoxy de 1 à 5 atomes de carbone ou par un ou plusieurs radicaux carboxy, un radical de formule -NH-CO-Ph, un radical de formule -NH-SO₂-Ph ou un radical de formule -NH-CO-NH-Ph,
   - **R**_{**2**} représente un radical alkyle de 1 à 5 atomes de carbone,
   - **A** représente un radical -CO-,
   - **R**_{**3**} et **R**_{**4**} différents représentent chacun un atome d'hydrogène, un radical alcoxy de 1 à 5 atomes de carbone, un radical amino, un radical carboxy un radical alcoxycarbonyle de 2 à 6 atomes de carbone,
éventuellement sous la forme de l'un de ses sels phannaceutiquement acceptables.

Parmi les composés de l'invention les composés particulièrement préférés sont les suivants :
- (4-amino -3-méthoxy phényl) (1-méthoxy 2-méthyl indolizin-3-yl) méthanone
- acide 3-(4-amino -3-méthoxy benzoyl) 2-méthyl indolizin-1-yl carboxylique
- acide 2-{[3-(4-amino -3-méthoxybenzoyl) 2-méthyl indolizin-1-yl] oxy} acétique
- (4-amino -3-méthoxy phényl) {1-[(4-chlorobenzyl) oxy] 2-méthylindolizin-3-yl} méthanone
- (4-amino -3-méthoxy phényl) {1-[(3-méthoxybenzyl) oxy] 2-méthylindolizin-3-yl} méthanone
- acide 4-({[3-(4-amino -3-méthoxy benzoyl) 2-méthyl indolizin-1-yl] oxy} méthyl) benzoique
- acide 3-(4-carboxybenzoyl) 2-méthyl indolizin-1-yl carboxylique
- 3-[(1-méthoxy -2-méthyl indolizin-3-yl carbonyl] benzoate de méthyle
- acide 4-[(1-méthoxy -2-méthyl indolizin-3-yl) carbonyl] benzoïque
- acide 2-amino-5-[(1-méthoxy-2-méthylindolizin-3-yl) carbonyl] benzoïque
- acide 2-amino-5-({1-[(3-méthoxybenzoyl) amino]-2-méthylindolizin-3-yl} carbonyl) benzoïque
- acide 2-amino-5-({2-méthyl-1-[(3,4,5-triméthoxybenzoyl)amino] indolizin-3-yl} carbonyl) benzoïque
- acide 2-amino-5-({1-{[(3-méthoxyphényl) sulfonyl] amino}-2-méthylindolizin-3-yl} carbonyl) benzoïque
éventuellement sous la forme de l'un de ses sels pharmaceutiquement acceptables.

La présente invention concerne également un procédé de préparation des composés de formule I caractérisé en ce que
**A)** on condense un dérivé d'indolizine de formule II, 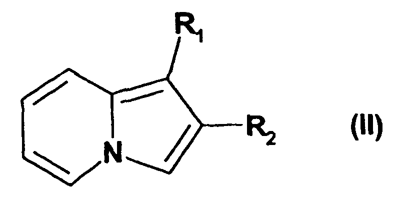 dans laquelle R₁ et R₂ ont la signification donnée pour la formule I mais R₂ ne représente pas un atome d'hydrogène ou un radical d'halogenure d'alkyle,
   avec un dérivé de formule III, 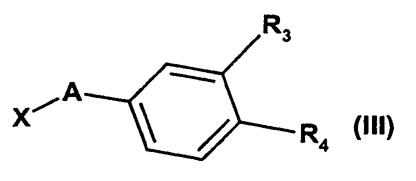 dans laquelle X représente un atome d'halogène et R₃ ou R₄ identiques ou différents représentent chacun un atome d'hydrogène, un radical nitro, un radical trifluoroacétamido, ou un radical alcoxycarbonyle de 2 à 6 atomes de carbone, pour obtenir les composés de formule Ia, Id ou Ik, 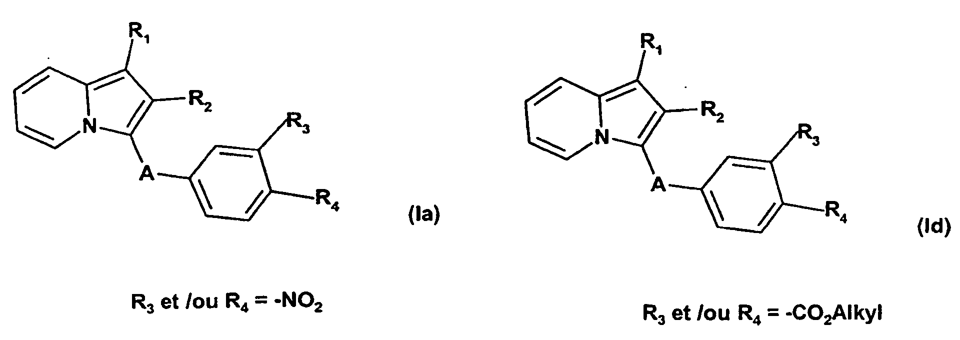 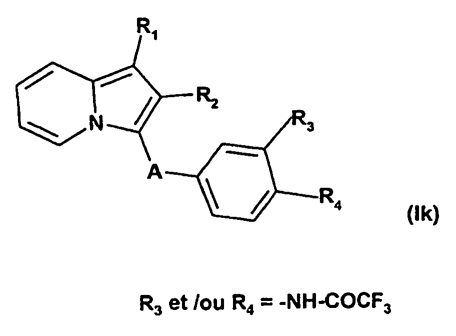 et ensuite,
   **a)** on soumet les composés de formule Ia à une réduction pour obtenir les composés de formule Ib, 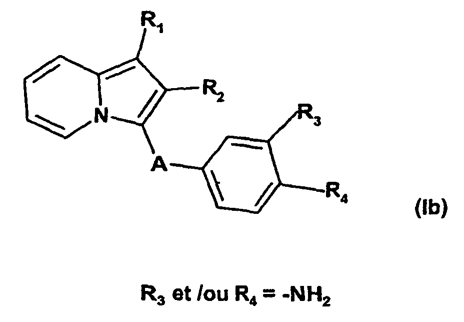 dans laquelle R₃ et / ou R₄ représentent un radical amino, lesquels ensuite composés de formule Ib
      - sont soumis à l'action d'un halogénure d'alkyle pour obtenir les composés de la formule I pour lesquels R₄ et / ou R₃ représentent un radical -NR₅R₆ (dans lequel R₅ représente un atome d'hydrogène et R₆ représente un radical alkyle de 1 à 5 atomes de carbone) un radical -NH-Alk-NR₅R₆ ou un radical -NH-Alk-COOR₇ (dans lequel R₇ ne représente pas un atome d'hydrogène) à partir duquel par une saponification ultérieure on obtient les composés de formule I pour lesquels R₄ et / ou R₃ représentent un radical -NH-Alk-COOR₇ dans laquelle R₇ représente un atome d'hydrogène,
      ou
      - sont soumis à une acylation pour obtenir les composés de formule I pour lesquels R₄ et / ou R₃ représentent un radical -NH-CO-Alk, ou un radical -NH-CO-Alk-NR₉R₁₀, lesquels ensuite sont soumis à une alkylation pour obtenir un radical -N(R₁₁)-CO-Alk ou un radical -N(R₁₁)-CO-Alk-NR₉R₁₀ où R₁₁ représente un radical -Alk-COOR₁₂ dans lequel R₁₂ ne représente pas un atome d'hydrogène, ces derniers composés sont ensuite éventuellement soumis à une saponification pour obtenir les composés de formule I pour lesquels R₄ et / ou R₃ représentent un radical -N(R₁₁)-CO-Alk ou un radical -N(R₁₁)-CO-Alk-NR₉R₁₀ où R₁₁ représente un radical -Alk-COOH,
      ou
      - sont soumis à une sulfonylation, pour obtenir les composés de formule I pour lesquels R₄ et / ou R₃ représentent un radical -NH-SO₂-Alk ou un radical -NH-SO₂-Alk-NR₉R₁₀, lesquels ensuite sont soumis à une alkylation pour obtenir un radical -N(R₁₁)-SO₂-Alk ou un radical -N(R₁₁)-SO₂-Alk-NR₉R₁₀ où R₁₁ représente un radical -Alk-COOR₁₂ dans lequel R₁₂ ne représente pas un atome d'hydrogène, ces derniers composés sont ensuite éventuellement soumis à une saponification pour obtenir les composés de formule I pour lesquels R₄ et / ou R₃ représentent un radical -N(R₁₁)-SO₂-Alk ou un radical -N(R₁₁)- SO₂-Alk -NR₉R₁₀ où R₁₁ représente un radical -Alk-COOH
   **b)** on soumet les composés de formule Id dans laquelle R₃ et / ou R₄ représentent un radical alcoxycarbonyle à une saponification pour obtenir les composés de formule I dans laquelle R₃ et / ou R₄ représente un radical carboxy,
      **ou**
   **c)** on soumet lorsque R₁ représente un radical benzyloxy les composés de formule Ia à l'action de l'acide trifluoroacétique ou les composés de formule Id à une hydrogénation, pour obtenir les composés de formule If, 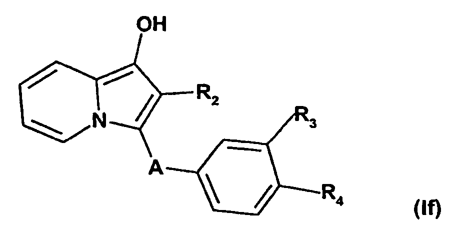 dans laquelle R₃ et / ou R₄ ont les significations données ci-dessus,
      et ensuite que l'on soumet les composés de formule If à une O-alkylation pour obtenir les composés de formule Ig, 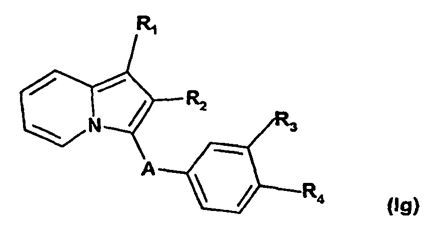 dans laquelle R₃ et / ou R₄ ont les significations données ci-dessus, et R₁ représente un radical alcoxy linéaire ou ramifié de 1 à 5 atomes de carbone, un radical -O-(CH₂)ₙ-cAlk, un radical -O-Alk-COOR₇, un radical -O-Alk-NR₅R₆ un radical -O-(CH₂)ₙ-Ph un radical -O-Alk-O-R₈, - qui lorsque R₈ représente un radical -COCH₃ peut donner par saponification ultérieure un radical -O-Alk-OH - ou un radical -O-Alk-CN qui par traitement avec de l'hydroxylamine conduit à un radical -O-Alk-C(NH₂)=NOH,
      **ou**
   **d)** on soumet lorsque R₁ représente un radical alcoxycarbonyle les composés de formule Ia à une saponification pour obtenir les composés de formule Ih, 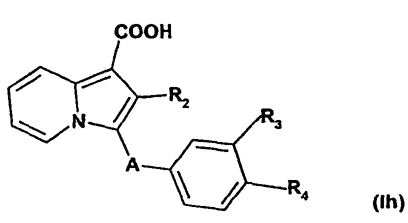 dans laquelle R₃ et / ou R₄ ont les significations données ci-dessus, lesquels ensuite sont soumis à l'action d'un dérivé d'amine pour obtenir les composés de formule I dans laquelle R₁ représente un radical -CO-NH-Alk ou à l'action d'un dérivé d'aminoacide pour obtenir les composés de formule I dans laquelle R₁ représente un radical -CO-NH-(CH₂)ₘ-COOR₇
      **ou**
   **e)** on soumet lorsque R₁ représente un radical -NH-CO₂tButyle les composés de formule Ia ou Id
      - soit à une alkylation suivie d'une déprotection et d'une éventuelle deuxième alkylation pour obtenir les composés de formule Ii,
      - soit à une déprotection suivie d'une acylation pour obtenir les composés de formule Ij dans laquelle R₅ représente un atome d'hydrogène, suivie d'une éventuelle alkylation pour obtenir les composés de formule Ij dans laquelle R₅ représente un radical alkyle 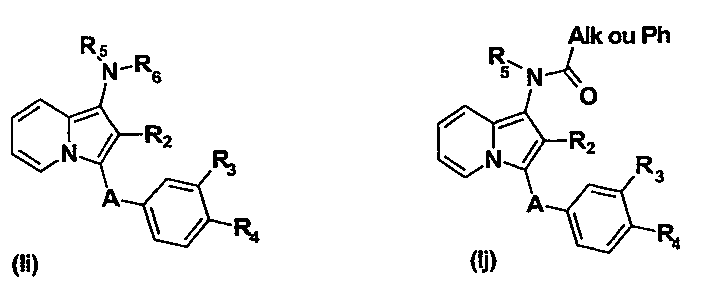
      **ou**
   **f)** on soumet lorsque R₁ représente un radical -NH-CO₂tButyle les composés de formule **Ik**
      - soit à une déprotection suivie d'une acylation pour obtenir les composés de formule Il 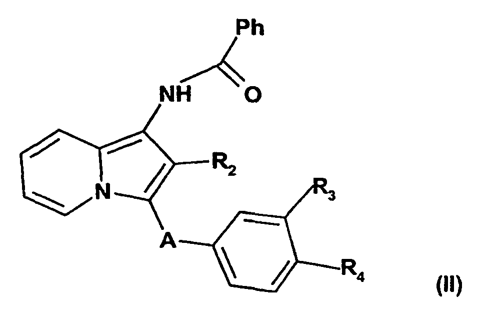
      - soit à une déprotection suivie d'une sulfonylation pour obtenir les composés de formule Im 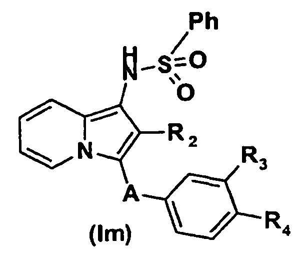
      - soit à une déprotection suivie d'un traitement par un phénylisocyanate pour obtenir les composés de formule In 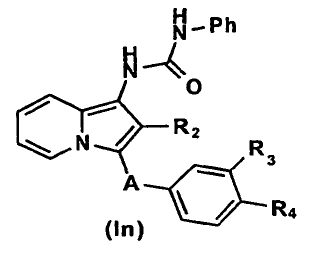
   **OU**
**B)** lorsque R₁ représente un groupement électro-attracteur, R₂ représente un atome d'hydrogène ou un radical d'halogenure d'alkyle et A représente un radical -CO- on fait réagir la pyridine avec une bromoacétophénone de formule IV, 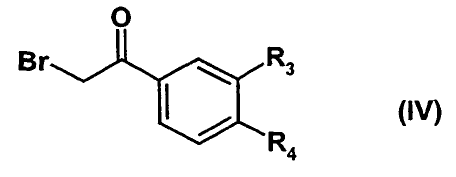 pour obtenir les composés de formule V, 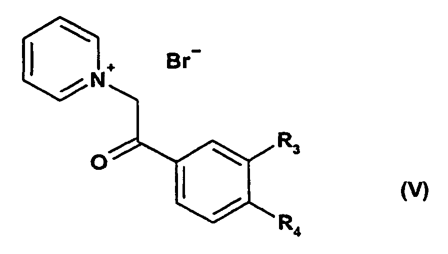 lesquels ensuite sont soumis à une cycloaddition 1,3-dipolaire avec l'acrylate d'éthyle ou un dérivé halogéné de crotonate d'éthyle en présence d'un oxydant pour obtenir les composés de formule la dans laquelle **R**_{**1**} représente un radical éthoxycarbonyle et R₂ représente un atome d'hydrogène ou un radical un radical d'halogenure d'alkyle.

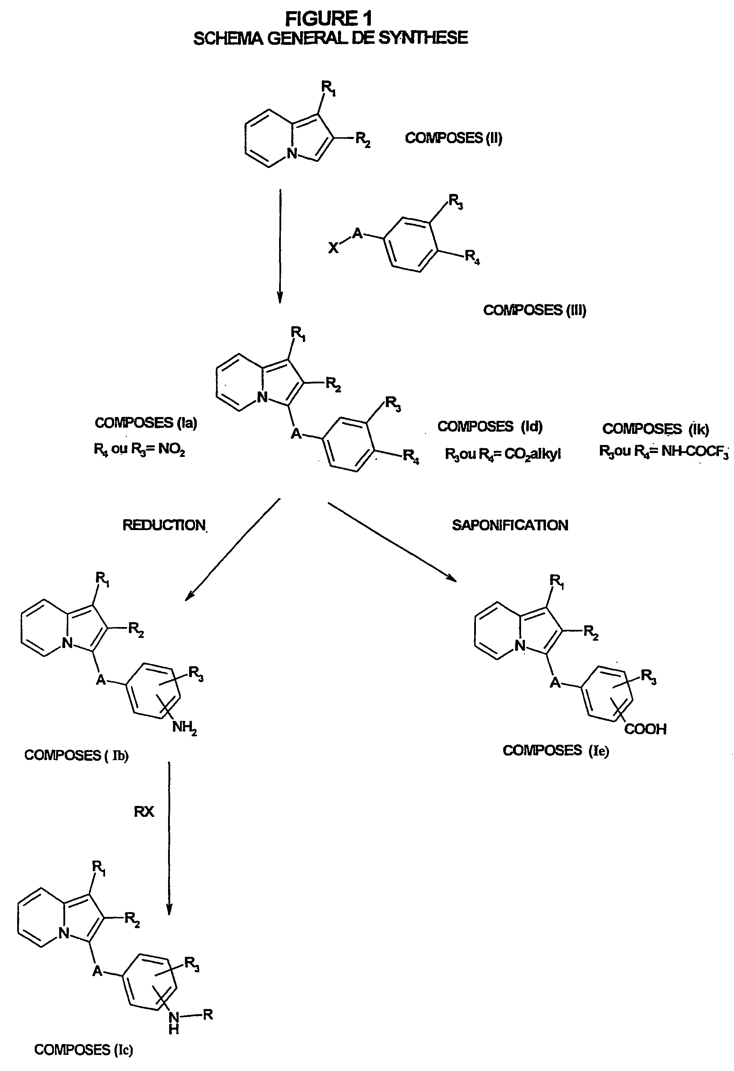

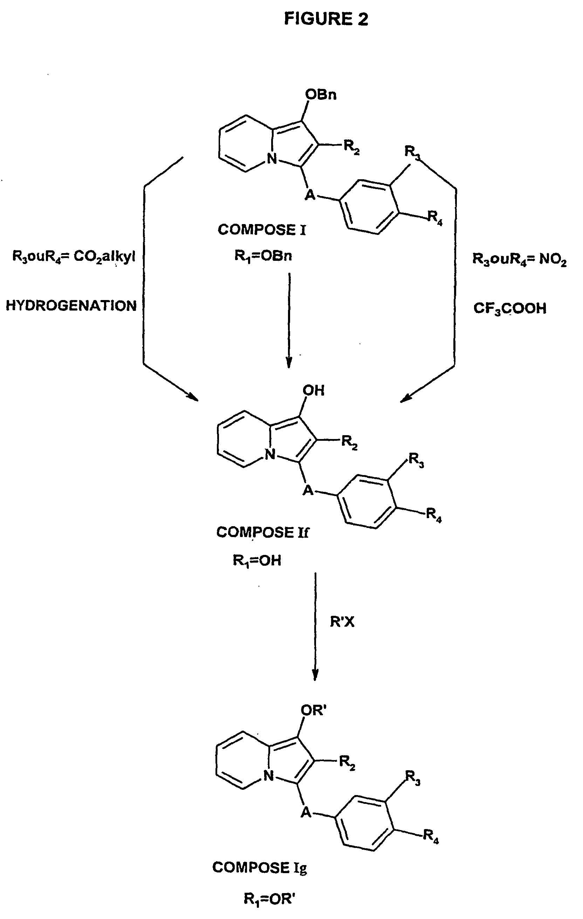

Les figures 1 et 2 donnent le schéma de synthèse des produits Ia à Ig et Ik.

Les composés de formule Ia dans lesquels R₂ représente un atome d'hydrogène ou un radical un radical d'halogenure d'alkyle, A représente un radical -CO- et R₁ est un groupement électro-attracteur tel que alcoxycarbonyl sont préparés selon des méthodes connues de cycloaddition [*J. Heterocyclic Chem.,* (2001), **38**, 853-857].

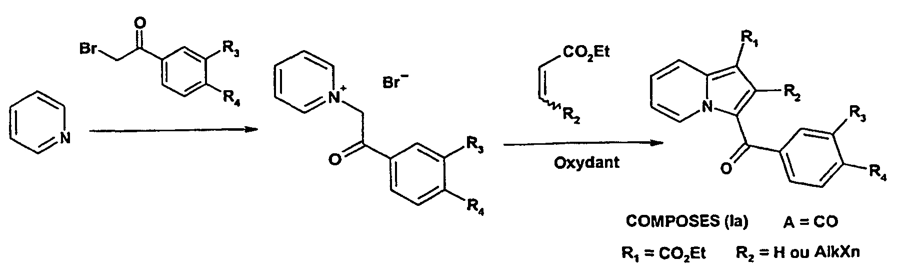

La quaternization de la pyridine par une bromoacétophénone convenablement substituée conduit au pyridinium. La cycloaddition 1,3-dipolaire de ce dernier est réalisé en présence d'un oxydant comme le tétrapyridinecobalt (II) dichromate dans un solvant polaire tel que le diméthylforinarnide.

Les composés selon l'invention, quand R₃ et/ou R₄ représentent un radical nitro sont préparés avec des méthodes connues de benzoylation (*Eur. J. Med. Chem. Chim. Ther,* (1983), 18(4), pp339-346) à partir d'un dérivé d'indolizine de formule II, et un dérivé de chlorure de nitrobenzoyle ou un dérivé de chlorure de nitrobenzène sulfonyle, composés qui correspondent à un composé de formule III. On obtient ainsi les composés de formule Ia.

A partir des composés de la formule la par réduction de la fonction nitro on obtient les composés de la formule Ib dans laquelle R₃ et/ou R₄ représente un radical amino. En soumettant les composés de formule Ib à l'action d'un halogénure d'alkyle on obtient les composés de la formule Ic pour lesquels R₃ et/ou R₄ représente un radical -NR₅R₆ (dans lequel R₅ représente un atome d'hydrogène et R₆ a les significations données ci-dessus), un radical -NH-Alk-NR₅R₆ ou un radical -NH-Alk-COOR₇ dans lequel R₇ ne représente pas un atome d'hydrogène. A partir de ces derniers composés, en les soumettant à une saponification ultérieure, on obtient les composés pour lesquels R₇ représente un atome d'hydrogène.

Par acylation des composés de formule Ib on obtient les composés de formule Ic pour lesquels R₃ et/ou R₄ représente un radical NH-CO-Alk, ou un radical NH-CO-Alk-N R₉R₁₀.

En soumettant ces composés pour lesquels R₃ et/ou R₄ représente un radical -NH-CO-Alk, ou un radical -NH-CO-Alk-NR₉R₁₀ à une alkylation avec un dérivé contenant un reste alcoxycarbonyle on obtient les composés de formule I pour lesquels R₃ et/ou R₄ représente un radical -N(R₁₁)-CO-Alk, ou un radical -N(R₁₁)-CO-Alk-NR₉R₁₀ où R₁₁ représente un radical -Alk-COOR₁₂ où R₁₂ ne représente pas un atome d'hydrogène. En soumettant ces derniers produits à une saponification, on obtient des composés pour lesquels R₃ et/ou R₄ représente un radical -N(R₁₁)-CO-Alk, ou un radical -N(R₁₁)-CO-Alk-NR₉R₁₀ où R₁₁ représente un radical -Alk-COOH.

Par sulfonylation des composés de formule Ib on obtient les composés de formule Ic pour lesquels R₃ et/ou R₄ représente un radical -NH-SO₂-Alk ou un radical NH-SO₂-Alk- NR₉R₁₀.

En soumettant ces composés pour lesquels R₃ et/ou R₄ représente un radical -NH-SO₂-Alk ou un radical NH-SO₂-Alk- NR₉R₁₀ à une alkylation avec un dérivé contenant un reste alcoxycarbonyle on obtient les composés de formule I pour lesquels R₃ et/ou R₄ représente un radical -N(R₁₁)-SO₂-Alk, ou un radical -N(R₁₁)-SO₂-Alk-NR₉R₁₀ où R₁₁ représente un radical -Alk-COOR₁₂ où R₁₂ ne représente pas un atome d'hydrogène. En soumettant ces derniers produits à une saponification, on obtient des composés pour lesquels R₃ et/ou R₄ représente un radical -N(R₁₁)-SO₂-Alk, ou un radical -N(R₁₁)-SO₂-Alk-NR₉R₁₀ où R₁₁ représente un radical -Alk-COOH.

En faisant réagir un dérivé d'indolizine de formule II avec un dérivé de chlorure d'alcoxycarbonylbenzoyle de formule III, on obtient les composés de formule Id dans laquelle R₃ et/ou R₄ représente un radical alcoxycarbonyle. En soumettant ces derniers composés à une saponification on obtient les composés de formule le dans laquelle R₃ et/ou R₄ représente un radical carboxy.

En faisant réagir un dérivé d'indolizine de formule II avec un dérivé de chlorure de trifluoroacétamidobenzoyle de formule III, on obtient les composés de formule **Ik** dans laquelle R₃ et/ou R₄ représente un radical trifluoroacétamide. En soumettant ces derniers composés à une hydrolyse basique on obtient les composés de formule **Ik** dans laquelle R₃ et/ou R₄ représente un radical carboxy et/ou amino.

Comme représenté dans la figure 2, à partir des composés de formule I dans laquelle R₁ représente un radical benzyloxy et R₃ ou R₄ représentent un radical alcoxycarbonyle, on peut obtenir, en soumettant ces composés à une hydrogénation, les composés de formule If. Quand R₃ ou R₄ représentent un radical nitro, les composés de la formule If sont obtenus par action de l'acide trifluoroacétique.

En soumettant les composés de formule If à une O-alkylation, on obtient les composés de formule Ig dans laquelle R₁ représente un radical alcoxy linéaire ou ramifié de 1 à 5 atomes de carbone, un radical -O-(CH₂)ₙ-cAlk, un radical -O-Alk-COOR₇, un radical -O-Alk-NR₅R₆, un radical -O-(CH₂)ₙ-Ph, un radical -O-Alk-O-R₈ -qui lorsque R₈ représente un radical -COCH₃ peut donner par saponification le radical -O-Alk-OH -, un radical -O-Alk-CN qui peut conduire au radical -O-Alk-C(NH₂):NOH par traitement avec de l'hydroxylamine.

Pour obtenir les composés de formule Ih dans laquelle R₁ est un radical carboxy et A est un radical -CO- ou un radical -SO₂ on soumet les composés de formule Ia dans laquelle R₁ est un radical alcoxycarbonyl à une saponification. Les dérivés de l'acide indolizin-1-yl carboxylique de formule Ih ainsi obtenus peuvent ensuite être soumis à l'action d'une amine pour préparer les composés de formule Ia dans laquelle R₁ représente un radical -CO-NH-Alk, ou à l'action d'un dérivé d'aminoacide pour obtenir les composés de formule I dans laquelle R₁ représente un radical -CO-NH-(CH₂)ₘ-COOR₇.

Les composés de formule II, lorsque R₁ représente un radical -NH-COO*t*Butyle ou un radical -N(CH₃)CH₂C₆H₅ sont préparés selon les schémas de synthèse suivants en utilisant la réaction de Tschitschibabin (*Synthesis,* (1975), p209) pour préparer les indolizines.

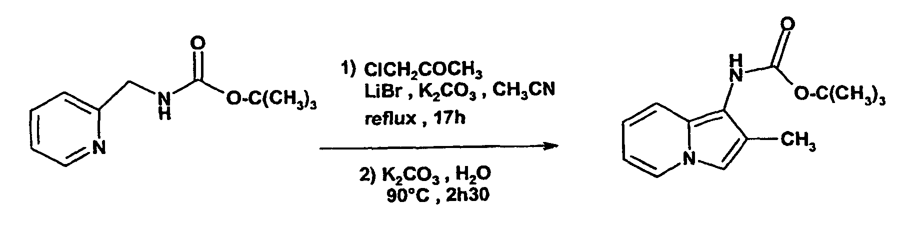

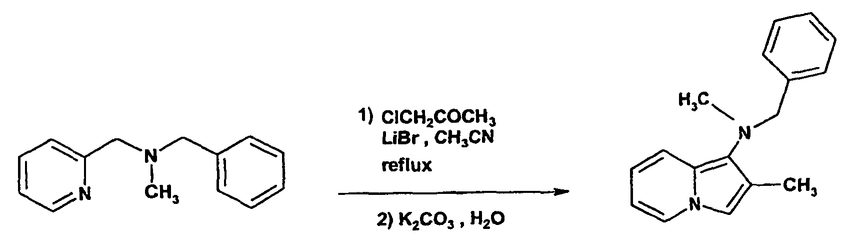

Les composés de formule II, lorsque R₁ représente un radical -OCH₃ ou un radical -OCH₂C₆H₅ sont préparés également en utilisant la réaction de Tschitschibabin selon les schéma de synthèse suivant :

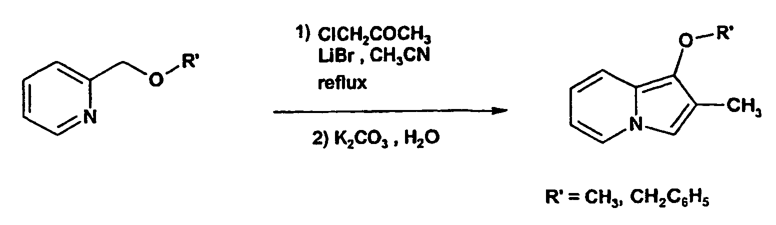

Les composés de la formule I sont des antagonistes puissants de FGF1 et 2. Leurs capacités d'inhiber à la fois la formation de nouveaux vaisseaux à partir de cellules endothéliales différenciées et de bloquer la différentiation de cellules de moelle osseuse humaine adulte CD34+ CD133+ en cellules endothéliales a été démontrée *in vitro.* De plus, leur capacité à inhiber l'angiogénèse pathologique a été démontrée *in vivo.* Par ailleurs, il a été démontré que les composés de formule I sont des antagonistes puissants du récepteur FGFR1

De manière générale, les FGFs sont impliqués de façon importante par l'intermédiaire de sécrétions autocrines, paracrines ou juxtacrines dans les phénomènes de dérégulation de la stimulation de la croissance des cellules cancéreuses. De plus, les FGFs affectent l'angiogénèse tumorale qui joue un rôle prépondérant à la fois sur la croissance de la tumeur mais aussi sur les phénomènes de métastasisation.

L'angiogénèse est un processus de génération de nouveaux vaisseaux capillaires à partir de vaisseaux préexistants ou par mobilisation et différentiation de cellules de la moelle osseuse. Ainsi, à la fois une prolifération incontrôlée des cellules endothéliales et une mobilisation d' angioblastes à partir de la moelle osseuse sont observées dans les processus de néovascularisation des tumeurs. Il a été montré *in vitro* et *in vivo* que plusieurs facteurs de croissance stimulent la prolifération endothéliale, et notamment le FGF1 ou a-FGF et le FGF2 ou b-FGF. Ces deux facteurs induisent la prolifération, la migration et la production de protéases par les cellules endothéliales en culture et la néovascularisation *in vivo*. Les a-FGF et b-FGF interagissent avec les cellules endothéliales par l'intermédiaire de deux classes de récepteurs, les récepteurs de haute affinité à activité tyrosine kinase (FGFRs) et les récepteurs de basse affinité de type héparan sulfate protéoglycane (HSPGs) situé à la surface des cellules et dans les matrices extracellulaires. Alors que le rôle paracrine de ces deux facteurs sur les cellules endothéliales est largement décrit, les a-FGF et b-FGF pourraient également intervenir sur ces cellules à travers un processus autocrine. Ainsi, les a-FGF et b-FGF et leurs récepteurs représentent des cibles très pertinentes pour les thérapies visant à inhiber les processus d'angiogénèse (Keshet E, Ben-Sasson SA.,. *J. Clin. Invest,* (1999), vol 501, pp104-1497 ; Presta M, Rusnati M, Dell'Era P, Tanghetti E, Urbinati C, Giuliani R et al, *New York: Plenum Publishers,* (2000), pp7-34, Billottet C, Janji B, Thiery J.P., Jouanneau J, Oncogene, (2002) vol 21, pp8128-8139).

Par ailleurs, des études systématiques visant à déterminer l'expression due aux a-FGF et b-FGF et de leurs récepteurs (FGFR) sur différents types de cellules tumorales mettent en évidence qu'une réponse cellulaire à ces deux facteursF est fonctionnelle dans une grande majorité de lignées tumorales humaines étudiées. Ces résultats supportent l'hypothèse qu'un antagoniste des a-FGF et b-FGF pourrait également inhiber la prolifération des cellules tumorales (Chandler LA, Sosnowski BA, Greenlees L, Aukerman SL, Baird A, Pierce GF., *Int.J.Cancer,* (1999), vol 58, pp81-451).

Les a-FGF et b-FGF jouent un rôle important dans la croissance et le maintien des cellules de la prostate. Il a été montré à la fois dans des modèles animaux et chez l'homme qu'une altération de la réponse cellulaire à ces facteurs joue un rôle primordial dans la progression du cancer de la prostate. En effet dans ces pathologies on enregistre à la fois une augmentation de la production des a-FGF et b-FGF par les fibroblastes et les cellules endothéliales présentes au niveau de la tumeur et une augmentation de l'expression des récepteurs FGFRs sur les cellules tumorales. Ainsi une stimulation paracrine des cellules cancéreuses de la prostate s'opère, et ce processus serait un composant majeur de cette pathologie. Un composé possédant une activité antagoniste des récepteurs FGFRs tels que les composés de la présente invention peut représenter une thérapie de choix dans ces pathologies (Giri D, Ropiquet F., *Clin*.*Cancer Res*., (1999), vol 71, pp5-1063; Doll JA, Reiher FK, Crawford SE, Pins MR, Campbell SC, Bouck NP., *Prostate,* (2001), vol 305, pp 49-293).

Plusieurs travaux montrent la présence de a-FGF et b-FGF et de leurs récepteurs FGFRs à la fois dans les lignées tumorales humaines du sein (notamment MCF7) et dans des biopsies de tumeurs. Ces facteurs seraient responsables dans cette pathologie de l'apparition de phénotype très agressif et induisant une forte métastasisation. Ainsi un composé possédant une activité antagoniste des récepteurs FGFRs, comme les composés de la formule I, peut représenter une thérapie de choix dans ces pathologies (Vercoutter-Edouart A-S, Czeszak X, Crépin M, Lemoine J, Boilly B, Le Bourhis X et al., *Exp.Cell Res.,* (2001), vol 262, pp59-68).

Les mélanomes cancéreux sont des tumeurs qui induisent avec une fréquence importante des métastases et qui sont très résistantes aux différents traitements de chimiothérapie. Les processus d'angiogénèse joue un rôle prépondérant dans la progression d'un mélanome cancéreux. De plus, il a été montré que la probabilité d'apparition de métastases augmente très fortement avec l'augmentation de la vascularisation de la tumeur primaire. Les cellules de mélanomes produisent et sécrètent différents facteurs angiogéniques dont le a-FGF et le b-FGF. Par ailleurs, il a été montré qu'une inhibition de l'effet cellulaire de ces deux facteurs par le FGFR1 soluble bloque *in vitro* la prolifération et la survie des cellules tumorales de mélanome et bloque *in vivo* la progression tumorale. Ainsi un composé possédant une activité antagoniste des récepteurs FGFRs comme les composés de la présente invention peut représenter une thérapie de choix dans ces pathologies (Rofstad EK, Halsor EF., *Cancer Res.,* (2000) ; Yayon A, Ma Y-S, Safran M, Klagsbrun M, Halaban R., *Oncogene,* (1997), vol 14, pp 2999-3009).

Les cellules de gliome produisent *in vitro* et *in vivo* du a-FGF et du b-FGF et possèdent à leur surface différents FGFRs. Cela suggère donc que ces deux facteurs par un effet autocrine et paracrine jouent un rôle pivotal dans la progression de ce type de tumeur. De plus, comme la plupart des tumeurs solides, la progression des gliomes et leur capacité à induire des métastases est très dépendante des processus angiogéniques dans la tumeur primaire. Il a également été montré que des antisens du FGFR1 bloquent la prolifération d'astrocytomes humains. De plus, des dérivés des naphthalenesulfonates sont décrites pour inhiber les effets cellulaires des a-FGF et b-FGF *in vitro* et l'angiogénèse induite par ces facteurs de croissance *in vivo*. Une injection intracérébrale de ces composés induit une augmentation très significative de l'apoptose et une diminution importante de l'angiogénèse se traduisant par une régression considérable de gliomes chez le rat. Ainsi un composé possédant une activité antagoniste des a-FGF et / ou b-FGF et / ou des récepteurs FGFRs, comme les composés de la présente invention, peut représenter une thérapie de choix dans ces pathologies (Yamada SM, Yamaguchi F, Brown R, Berger MS, Morrison RS, *Glia* , (1999), vol 76, pp28-66 ; Auguste P, Gürsel DB, Lemière S, Reimers D, Cuevas P, Carceller F et al., *Cancer Res.,* (2001), vol 26, pp 61-1717).

Plus récemment le rôle potentiel d'agents pro angiogéniques dans les leucémies et lymphomes a été documenté. En effet de manière générale il a été rapporté que des clones cellulaires dans ces pathologies peuvent être soit détruits naturellement par le système immunitaire soit basculer dans un phénotype angiogénique qui favorise leur survie puis leur prolifération. Ce changement de phénotype est induit par une sur expression de facteurs angiogéniques notamment par les macrophages et / ou une mobilisation de ces facteurs à partir de la matrice extracellulaire (Thomas DA, Giles FJ, Cortes J, Albitar M, Kantarjian HM., *Acta Haematol,* (2001), vol 207, pp106-190). Parmi les facteurs angiogéniques, le b-FGF a été détecté dans de nombreuses lignées cellulaires tumorales lymphoblastiques et hématopoiétiques. Les récepteurs FGFRs sont également présents sur la majorité de ces lignées suggérant un possible effet cellulaire autocrine des a-FGF et b-FGF induisant la prolifération de ces cellules. Par ailleurs il a été rapporté que l'angiogénèse de la moelle osseuse par des effets paracrines était corrélée à la progression de certaines de ces pathologies.

De manière plus particulière il a été montré dans les cellules CLL (chronic lymphocytic leukemia) que le b-FGF induit une augmentation de l'expression de protéine anti apoptotique (Bc12) conduisant à une augmentation de la survie de ces cellules et participe donc de manière importante à leur cancérisation. De plus, les taux de b-FGF mesurés dans ces cellules sont très bien corrélés avec le stade d'avancement clinique de la maladie et la résistance à la chimiothérapie appliquée dans cette pathologie (fludarabine). Ainsi, un composé possédant une activité antagoniste des récepteurs FGFRs, comme les composés de la présente invention, peut représenter une thérapie de choix soit seul soit en association avec la fludarabine ou d'autres produits actifs dans cette pathologie (Thomas DA, Giles FJ, Cortes J, Albitar M, Kantarjian HM., *Acta Haematol,* (2001), vol 207, pp106-190 ; Gabrilove JL, *Oncologist,* (2001), vol 6, pp4-7).

Il existe une corrélation entre le processus d'angiogénèse de la moelle osseuse et les "extramedullar disease" dans les CML (chronic myelomonocytic leukemia). Différentes études démontrent que l'inhibition de l'angiogénèse, en particulier par un composé possédant une activité antagoniste des récepteurs FGFRs, pourrait représenter une thérapeutique de choix dans cette pathologie.

La prolifération et la migration de cellules musculaires lisses vasculaires contribuent à l'hypertrophie intimale des artères et joue ainsi un rôle prépondérant dans l'athérosclérose et dans la resténose après angioplastie et endoarterectomie.

Des études *in vivo* montrent, après lésion de la carotide par "balloon injury", une production locale de a-FGF et de b-FGF. Dans ce même modèle un anticorps neutralisant anti FGF2 inhibe la prolifération des cellules musculaires lisses vasculaires et diminue ainsi l'hypertrophie intimale.
Une protéine chimérique FGF2 liée à une molécule telle que la saponine bloque la liaison du b-FGF à ses récepteurs FGFRs, inhibe la prolifération des cellules musculaires lisses vasculaires *in vitro* et l'hypertrophie intimale *in vivo* (Epstein CE, Siegall CB, Biro S, Fu YM, FitzGerald D., *Circulation,* (1991), vol 87, pp84-778 ; Waltenberger J., *Circulation,* (1997), pp96-4083).
Ainsi, les antagonistes des récepteurs FGFRs, tels que les composés de la présente invention représentent une thérapie de choix, soit seul, soit en association avec des composés antagonistes d'autres facteurs de croissance impliqués dans ces pathologies comme le PDGF, dans le traitement des pathologies liées à la prolifération des cellules musculaires lisses vasculaires telles que l'athérosclérose, la resténose post-angioplastie ou suite à la pose de prothèses endovasculaires (stents) ou lors de pontages aorto-coronariens.

L'hypertrophie cardiaque intervient en réponse à un stress de la paroi ventriculaire induit par une surcharge en terme de pression ou de volume. Cette surcharge peut être la conséquence de nombreux états physio pathologiques comme l'hypertension, l'AC (aortic coarctation), l'infarctus du myocarde, et différents troubles vasculaires. Les conséquences de cette pathologie sont des changements morphologiques, moléculaires et fonctionnels comme l'hypertrophie des myocytes cardiaques, l'accumulation de protéines matricielles et la ré-expression de gènes foetaux. Le b-FGF est impliqué dans cette pathologie. En effet l'addition de b-FGF à des cultures de cardiomyocytes de rat nouveau-né modifie le profil des gènes correspondants aux protéines contractiles conduisant à un profil de gènes de type foetaux. De manière complémentaire des myocytes de rat adulte montrent une réponse hypertrophique sous l'effet du b-FGF, cette réponse etant bloquée par des anticorps neutralisants anti b-FGF. Des expériences réalisées *in vivo* sur des souris transgéniques "knock-out" pour le b-FGF, montrent que le b-FGF est le facteur stimulant majeur de l'hypertrophie des myocyte cardiaque dans cette pathologie (Schultz JeJ, Witt SA, Nieman ML, Reiser PJ, Engle SJ, Zhou M et al., *J.Clin. Invest*., (1999), vol 19, pp 104-709).
Ainsi un composé, comme les composés de la présente invention, possédant une activité antagoniste des récepteurs FGFRs représente une thérapie de choix dans le traitement de l'insuffisance cardiaque et toute autre pathologie associée à une dégénérescence du tissu cardiaque. Ce traitement pourrait être réalisé seul ou en association avec les traitements courants (beta-bloquants, diurétiques, antagonistes d'angiotensine, antiarrythmiques, anti-calciques, anti-thrombotiques etc...)

Les troubles vasculaires dus au diabète se caractérisent par une altération de la réactivité vasculaire et du flux sanguin, une hyperperméabilité, une réponse proliférative exacerbée et une augmentation des dépôts de protéines matricielles. De manière plus précise le a-FGF et le b-FGF sont présents dans les membranes pré rétiniennes de patients ayant des rétinopathies diabétiques, dans les membranes des capillaires sous jacents et dans l'humeur vitrée de malades souffrants de rétinopathies prolifératives. Un récepteur du FGF soluble capable de lier à la fois le a-FGF et le b-FGF est développé dans les troubles vasculaires liés au diabète (Tilton RG, Dixon RAF, Brock TA., *Exp. Opin. Invest. Drugs,* (1997), vol 84, pp6-1671). Ainsi un composé comme les composés de formule I possédant une activité antagoniste des récepteurs FGFRs représente une thérapie de choix soit seul soit en association avec des composés antagonistes d'autres facteurs de croissance impliqués dans ces pathologies comme le VEGF.

L'arthrite rhumatoïde (RA) est une maladie chronique avec une étiologie inconnue. Alors qu'elle affecte de nombreux organes, la forme la plus sévère de RA est une inflammation synoviale des articulations progressive aboutissant à la destruction. L'angiogénèse semble affecter de manière importante la progression de cette pathologie. Ainsi le a-FGF et le b-FGF ont été détectés dans le tissu synovial et dans le fluide articulaire de patients atteints de RA, indiquant que ce facteur de croissance intervient dans l'initiation et / ou la progression de cette pathologie. Dans des modèles de AIA (adjuvant-induced model of arthritis) chez le rat, il a été montré que la sur-expression de b-FGF augmente la sévérité de la maladie alors qu'un anticorps neutralisant anti b-FGF bloque la progression de la RA (Yamashita A, Yonemitsu Y, Okano S, Nakagawa K, Nakashima Y, Irisa T et al., *J*.*Immunol*., (2002), vol 57, pp 168-450 ; Manabe N, Oda H, Nakamura K, Kuga Y, Uchida S, Kawaguchi H, *Rheumatol,* (1999), vol.20, pp38-714). Ainsi les composés selon l'invention représentent une thérapie de choix dans cette pathologie.

Les IBD (inflammatory bowel disease) comprennent deux formes de maladies inflammatoires chroniques de l'intestin : les UC (ulcerative colitis) et la maladie de Crohn's (CD). Les IBD sont caractérisées par une dysfonction immunitaire se traduisant par une production inappropriée de cytokines inflammatoires induisant l'établissement d'un système micro-vasculaire local. Cette angiogénèse d'origine inflammatoire a pour conséquence une ischémie intestinale induite par vasoconstriction. Des taux circulants et locaux de b-FGF importants ont été mesurés chez des patients atteints de ces pathologies (Kanazawa S, Tsunoda T, Onuma E, Majima T, Kagiyama M, Kkuchi K., *American Journal of Gastroenterology,* (2001), vol 28, pp 96-822 ; Thom M, Raab Y, Larsson A, Gerdin B, Hallgren R., *Scandinavian Journal of Gastroenterology,* (2000), vol 12, pp35-408). Les composés de l'invention présentant une activité anti angiogénique importante dans un modèle d'angiogénèse inflammatoire représentent une thérapie de choix dans ces pathologies.

Les FGFR1 2 et 3 sont impliqués dans les processus de chronogénèse et osteogénèse. Des mutations conduisant à l'expression de FGFRs toujours activés ont été reliées à un grand nombre de maladies génétiques humaines se traduisant par des malformations du squelette comme les syndromes de Pfeiffer, Crouzon, Apert, Jackson-Weiss et Bear-Stevenson cutis gyrata. Certaines de ces mutations affectant plus particulièrement le FGFR3 conduisent notamment à des achondroplasies (ACH), des hypochondroplasies (HCH) et des TD (Thanatophoric dysplasia); ACH étant la forme la plus courante de nanisme. D'un point de vue biochimique l'activation soutenue de ces récepteurs s'effectue par une dimérisation du récepteur en absence de ligand (Chen L., Adar R., Yang X. Monsonego E.O., LI C., Hauschka P.V, Yagon A. and Deng C.X., (1999), *The Journ. Of Clin. Invest.,* vol 104 n° 11, pp 1517-1525). Ainsi les composés de l'invention présentant une activité antagoniste de la liaison du b-FGF au FGFR et inhibant ainsi la dimérisation du récepteur représentent une thérapie de choix dans ces pathologies.

Grâce à leur faible toxicité et leurs propriétés pharmacologiques et biologiques, les composés de la présente invention trouvent leur application dans le traitement de tout carcinome ayant un degré de vascularisation important (poumon, sein, prostate, oesophage) ou induisant des métastases (colon, estomac, mélanome) ou étant sensibles au a-FGF ou au b-FGF de manière autocrine ou enfin dans des pathologies de type lymphomes et leucémies. Ces composés représentent une thérapie de choix soit seul soit en association avec une chimiothérapie adaptée. Les composés selon l'invention trouvent également leur application dans le traitement de maladies cardiovasculaires comme l'athérosclérose, la resténose post angioplastie dans le traitement des maladies liés aux complications apparaissant suite à la pose de prothèses endovasculaires et/ou de pontages aorto-coronariens ou d'autres greffes vasculaires et l'hypertrophie cardiaque ou de complications vasculaires du diabète comme les rétinopathies diabétiques. Les composés selon l'invention trouvent également leur application dans le traitement de maladies inflammatoires chroniques comme l'arthrite rhumatoïde ou les IBD. Enfin les composés selon l'invention peuvent être utilisés dans le traitement des achondroplasies (ACH), des hypochondroplasies (HCH) et des TD (Thanatophoric dysplasia).

Selon un autre de ses aspects, la présente invention a donc pour objet une composition pharmaceutique contenant, en tant que principe actif, un composé de formule I selon l'invention ou un de ses sels pharmaceutiquement acceptables, éventuellement en association avec un ou plusieurs excipients inertes et appropriés.

Lesdits excipients sont choisis selon la forme pharmaceutique et le mode d'administration souhaitée : orale, sublinguale, sous-cutanée, intramusculaire, intraveineuse, transdermique, transmuqueux, locale ou rectale.

Les compositions pharmaceutiques selon la présente invention sont administrées de préférence par voie orale.

Dans les compositions pharmaceutiques de la présente invention pour l'administration orale, les principes actifs peuvent être administrés sous forme unitaire d'administration, en mélange avec des supports pharmaceutiques classiques. Les formes unitaires d'administration appropriées comprennent par exemple les comprimés éventuellement sécables, les gélules, les poudres, les granules et les solutions ou suspensions orales.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues.

On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants ou des agents de mise en suspension, comme la polyvinylpyrrolidone, de même qu'avec des édulcorants ou des correcteurs du goût.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

La quantité de principe actif à administrer dépend, comme toujours, du degré de progression de la maladie ainsi que de l'âge et du poids du patient.

Les compositions selon l'invention, pour une administration orale, contiennent donc des doses recommandées de 0,01 à 700 mg.

Les exemples suivants, donnés à titre non limitatif, illustrent la présente invention.

### PREPARATIONS

### Préparation I

### Synthèse de tert-butyl-2-méthylindolizin-1-yl carbamate

A 10g (48m.moles) de tert-butyl [(pyridin-2-yl) méthyl] carbamate dans 50ml d'acétonitrile, on ajoute 11.7g (62.4m.moles) de carbonate de potassium et 6.3g (72m.moles) de bromure de lithium puis 5ml (62.4m.moles) de chloroacétone et on chauffe à reflux pendant une nuit.

On refroidit et on ajoute 40ml d'eau et 11.7g (62.4m.moles) de carbonate de potassium puis on chauffe à 90°C pendant 2h30. Le milieu réactionnel est refroidi et extrait à l'acétate d'éthyle.

La phase organique est décantée, lavée avec une solution aqueuse saturée de chlorure de sodium, séchée sur sulfate de sodium et concentrée sous pression réduite. Le produit est purifié par chromatographie flash sur colonne de silice en éluant avec un mélange de toluène / acétate d'éthyle (95 : 5). On recueille 6.27g d'une poudre blanche.
Rendement : 53%
Point de Fusion : 111°C

### Préparation II

### Synthèse de : N-benzyl N-méthyl N-(2-méthylindolizin-1-yl) amine

Ce composé est obtenu selon le même mode opératoire que le composé de la préparation I en utilisant la réaction de Tschitschibabin et en partant de 2.47g de *N*-benzyl *N*-méthyl *N*-[(pyridin-2-yl) méthyl] amine et de chloroacétone. On obtient 970mg d'une huile jaune.
Rendement : 34%
Spectrométrie de masse (Mode ES+) : MH+ = 251

### Préparation III

### Synthèse de la 1-méthoxy 2-méthylindolizine

Ce composé est obtenu en partant de la 2-(méthoxyméthyl)pyridine et de la chloroacétone en utilisant la réaction de Tschitschibabin. Le produit est isolé sous forme d'une huile jaune qui cristallise au congélateur.
Rendement : 77.5%
Spectrométrie de Masse (Mode ES+) : MH+ = 161.8

### Préparation IV

### Synthèse de la 1-benzyloxy-2-méthylindolizine.

Ce composé est obtenu selon le même procédé que celui décrit dans la Préparation I en utilisant la réaction de Tschitschibabin.
Le produit est isolé sous forme d'une huile jaune.
Rendement : 39%.

### Préparation V

### Synthése du 5-(chlorocarbonyl)-2-[(2,2,2-trifluoroacétyl) amino] benzoate de méthyle

### Etape A

Synthèse de **l'acide 4-amino-3-(méthoxycarbonyl) benzoïque** A 2.5g (12.1 m.moles) d'acide 2,4-dioxo-1,4-dihydro-2*H*-3,1-benzoxazine-6-carboxylique [décrit dans *J.Med. Chem*.; (1981), **24**(6), 735-742] en solution dans 10ml de diméthylformamide et 10ml de méthanol, on ajoute 150mg (1.21 m.mole) de 4-diméthylaminopyridine, et chauffe le mélange à 60°C pendant 3 heures. On concentre le milieu réactionnel sous pression réduite. On reprend le résidu dans de l'eau et on extrait avec de l'acétate d'éthyle. La phase organique est lavée avec une solution saturée de chlorure de sodium, séchée sur sulfate de sodium et concentrée sous pression réduite. Le solide obtenu est repris dans de l'acétate d'éthyle, filtré et séché. On obtient 1.98g d'une poudre blanche.
Rendement : 84%
Point de fusion : 224.5°C

### Etape B

### Synthèse de l'acide 3-(méthoxycarbonyl)-4-[(2,2,2-trifluoroacétyl)amino] benzoate de méthyle

A 1.0g (5.12 m.moles) d'acide 4-amino-3-(méthoxycarbonyl)benzoïque en suspension dans 15ml de dichlorométhane, on ajoute rapidement 868µl (6.15 m.moles) d'anhydride trifluoroacétique. On agite la solution pendant 30 minutes à température ambiante. On concentre la solution à sec et on reprend le solide obtenu dans un mélange pentane/éther éthylique puis on filtre. On obtient 1.48g d'une poudre blanche après séchage.
Rendement : 99%
Point de fusion : 239°C

### Etape C

A 784mg (2.69 m.moles) d'acide 3-(méthoxycarbonyl)-4-[(2,2,2-trifluoroacétyl) amino] benzoate de méthyle en solution dans 9ml de dichlorométhane, on ajoute 530µl (7.27m.moles) de chlorure de thionyle et 3 gouttes de diméthylformamide puis chauffe à reflux 90 minutes. Le milieu est évaporé à sec, entraîne l'excès de chlorure de thionyle par co-évaporation avec du toluène. On obtient 834mg de chlorure d'acide sous forme d'un solide jaune, utilisé tel quel sans autre purification dans les étapes de benzoylation des indolizines.
Rendement : quantitatif.

### EXEMPLES

### Exemple 1

### (1-methoxy 2-méthyl indolizin-3-yl)(3-méthoxy-4-nitrophényl) méthanone

A 3g (0.0186mole) de 1-méthoxy 2-méthylindolizine dont la préparation est décrite dans la Préparation III dissout dans 50ml de 1,2-dichloroéthane, on ajoute 4.21g (0.0195 moles) de chlorure de 3-méthoxy 4-nitrobenzoyle et on agite à température ambiante pendant 4 heures.

Le milieu réactionnel est versé sur de l'eau. La phase organique est décantée, lavée avec une solution aqueuse de bicarbonate de sodium puis à l'eau, séchée sur sulfate de sodium et concentrée sous vide.

Le résidu est purifié par chromatographie sur colonne de silice en éluant avec du dichlorométhane. Après évaporation on obtient 6.05g d'un solide jaune.
Rendement : 95%
Point de fusion : 287°C

### Exemples 2 à 28

En procédant selon la préparation décrite ci-dessus, on synthétise les composés de formule I, pour lesquels A représente un radical -CO-, décrits dans le Tableau I ci-dessous, par benzoylation de la position 3 des indolizines diversement substituées en position 1 et 2 avec les chlorures de benzoyle substitués adéquats.

**TABLEAU I**

| **Exemple** | **R**_{**1**} | **R**_{**2**} | **R**_{**3**} | **R**_{**4**} | **Rendement (%)** | **Point de Fusion (°C)** ou **Spectro de Masse (MH+)** |
|---|---|---|---|---|---|---|
| 2 | OBn | Ph | OMe | NO₂ | 94 | 186°C |
| 3 | OBn | Me | OMe | NO₂ | 95 | 153°C |
| 4 | OBn | Me | H | CO₂Me | 70.5 | 110°C |
| 5 | OMe | cPr | OMe | NO₂ | 81 | 112°C |
| 6 | OMe | Ph | OMe | NO₂ | 82 | 65°C |
| 7 | OMe | Me | H | NO₂ | 88 | 146°C |
| 8 | OMe | Me | H | CO₂Me | 92 | 143°C |
| 9 | OMe | Me | CO₂Me | H | 75 | 121°C |
| 10 | OMe | Me | NO₂ | CO₂Me | 57 | 138°C |
| 11 | OMe | Me | OMe | CO₂Me | 88.5 | 145°C |
| 12 | OMe | Me | H | CH₂CO₂Me | 75 | 94°C |
| 13 | CO₂Et | Me | OMe | -NO₂ | 91 | 137°C |
| 14 | CO₂Et | Me | OMe | CO₂Me | 45.5 | 141°C |
| 15 | CO₂Et | Ph | OMe | NO₂ | 85 | 151°C |
| 16 | CO₂Et | Me | H | CO₂Me | 98 | 139°C |
| 17 | N(Me)Bn | Me | OMe | NO₂ | 90 | MH+ = 430.3 |
| 18 | NHBOC | Me | OMe | NO₂ | 76 | MH+ = 426.5 |
| 19 | CO₂Et | Me | CO₂Me | NO₂ | 92 | 137°C |
| 20 | OMe | Me | CO₂Me | NO₂ | 100 | 150°C |
| 21 | OMe | Me | NO₂ | OMe | 90 | 135°C |
| 22 | CO₂Et | Me | NO₂ | OMe | 30 | 60°C |
| 23 | CO₂Et | Me | CO₂Me | NO₂ | 92 | 137°C |
| 24 | OMe | Me | CO₂Me | OMe | 69 | 119°C |
| 25 | CO₂Et | Me | CO₂Me | OMe | 12 | 110°C |
| 26 | OMe | cPr | CO₂Me | NO₂ | 34 | MH⁺ = 395.2 |
| 27 | NH-BOC | Me | CO₂Me | NO₂ | 81 | 92°C |
| 28 | NH-BOC | Me | CO₂Me | NHCOCF₃ | 81 | 226°C |
| Bn = benzyle | | | | | | |
| Me = méthyle | | | | | | |
| Et = éthyle | | | | | | |
| BOC = *t*butoxycarbonyle | | | | | | |

### Exemple 29

### (1-amino-2-méthyl indolizin-3-yl)(3-méthoxy-4-nitrophényl) méthanone

A une solution de 643mg (1.51 m.mole) de *tert*-butyl 3-(3-méthoxy 4-nitrobenzoyl) 2-méthyl indolizin-1-yl carbamate dans 20ml de dichlorométhane, refroidie à 0°C, on ajoute goutte à goutte 2.32ml d'acide trifluoroacétique. L'introduction terminée, on laisse revenir à température ambiante et on agite 4 heures. Le milieu réactionnel est versé sur une solution aqueuse saturée de carbonate de potassium et extrait à l'acétate d'éthyle. La phase organique est décantée, lavée avec une solution aqueuse saturée de chlorure de sodium, séchée sur sulfate de sodium et concentrée sous pression réduite. Les cristaux obtenus sont repris à l'éther isopropylique, filtrés, lavés à l'éther isopropylique puis séchés. On obtient 425mg d'un solide brun. Rendement : 87%
Spectrométrie de masse (Mode ES+) MH⁺ = 326.3

En procédant selon la préparation décrite ci-dessus, on synthétise les composés de formule I pour lesquels A représente un radical -CO-, décrits dans le Tableau II ci-dessous, par déprotection de l'amine en position 1 des indolizines à l'aide d'acide trifluoroacétique

**TABLEAU II**

| **Exemple** | **R**_{**1**} | **R**_{**2**} | **R**_{**3**} | **R**_{**4**} | **Rendement (%)** | **Point de Fusion (°C)** |
|---|---|---|---|---|---|---|
| 30 | NH₂ | Me | CO₂Me | NO₂ | 91 | 162°C |
| 31 | NH₂ | Me | CO₂Me | NHCOCF₃ | 88 | 231°C |

### Exemple 32

### N-[3-(3-methoxy 4-nitrobenzoyl) 2-méthyl indolizin-1-yl] méthane sulfonamide

A une solution de 350mg (1.08 m.mole) du composé de l'exemple 29 dans 3ml de pyridine, on ajoute 0.292ml (3.78m.moles) de chlorure de mésyle et agite à température ambiante pendant 4 heures. Le milieu réactionnel est concentré sous pression réduite. Le résidu est repris par de l'acide chlorhydrique 1N et extrait au dichlorométhane. La phase organique est décantée, lavée avec une solution aqueuse saturée de chlorure de sodium, séchée sur sulfate de sodium et concentrée sous pression réduite. Le résidu est cristallisé dans l'éthanol. On obtient 327mg de cristaux jaunes.
Rendement : 75%
Spectrométrie de masse (Mode ES+) MH⁺ = 404.3

### Exemple 33

### 5-[(1-{[(3-méthoxyphényl) sulfonyl] amino}-2-méthylindolizin-3-yl) carbonyl]-2-[(2,2,2-trifluoroacétyl) amino] benzoate de méthyle

Ce composé est préparé selon le même procédé que l'exemple précédent par sulfonylation du 5-[(1-amino-2-méthylindolizin-3-yl)carbonyl]-2-[2,2,2-trifluoroacétyl)amino]benzoate de méthyle par le chlorure de 3-méthoxybenzene sulfonyle. On obtient 466mg d'une poudre jaune.
Rendement : 83%
Point de fusion : 220.5°C

### Exemple 34

### 5-[(1-{[(3-méthoxyanilino)carbonyl]amino}-2-méthylindolizin-3-yl)carbonyl]-2-[(2,2,2-trifluoroacétyl)amino]benzoate de méthyle

A 400mg (0.95 m.mole) de 5-[(1-amino-2-méthylindolizin-3-yl)carbonyl]-2-[2,2,2-trifluoroacétyl)amino]benzoate de méthyle dissous dans 13ml de tétrahydrofurane, on ajoute 140µl (1.05 m.mole) de 3-méthoxyphénylisocyanate. On chauffe le mélange réactionnel à 40°C pendant 20 heures et on concentre sous pression réduite. On reprend le résidu obtenu dans de l'acétone, on filtre le solide et on le lave avec de l'acétone puis de l'éther éthylique. On obtient 442mg d'une poudre jaune.
Rendement : 82%
Point de fusion : 314°C

### Exemple 35

### (3-méthoxy 4-nitrophényl) [2-méthyl 1-(méthylamino) indolizin-3-yl] méthanone

### ETAPE A

### Synthèse du tert-butyl 3-(3-méthoxy 4-nitrobenzoyl) 2-méthyl indolizin-1-yl (méthyl) carbamate

A 315mg (7.9m.moles) d'hydrure de sodium (à 60% en dispersion dans l'huile) en suspension dans 10ml de tétrahydrofurane refroidie à 0°C, on ajoute goutte à goutte 3.05g (7.2 m.moles) de *tert*-butyl 3- (3-méthoxy 4-nitrobenzoyl) 2-méthyl-indolizin-1-yl carbamate en solution dans 50ml de tétrahydrofurane. Après 1 heure d'agitation à 0°C, on ajoute 0.59ml (9.5 m.moles) d'iodure de méthyle en maintenant à 0°C. On laisse revenir à température ambiante et on agite 1 heure. Le milieu réactionnel est versé sur une solution aqueuse saturée de bicarbonate de sodium et extrait à l'acétate d'éthyle. La phase organique est décantée, lavée avec une solution aqueuse saturée de chlorure de sodium, séchée sur sulfate de sodium et concentrée sous pression réduite. On obtient 3.47g d'une mousse orangée.
Rendement : 96%
Spectrométrie de masse (Mode ES+) MH⁺ = 440.3.

### ETAPE B

A une solution de 3.38g (7.7 m.moles) du produit obtenu à l'étape A dans 60ml de dichlorométhane, refroidie à 0°C, on ajoute goutte à goutte 13 ml d'acide trifluoroacétique. L'introduction terminée, on laisse revenir à température ambiante et on agite 3 heures.

Le milieu réactionnel est versé sur une solution aqueuse saturée de bicarbonate de sodium et extrait à l'acétate d'éthyle. La phase organique est décantée, lavée avec une solution aqueuse saturée de chlorure de sodium, séchée sur sulfate de sodium et concentrée sous pression réduite.

Le résidu est purifié par chromatographie flash sur colonne de silice en éluant avec un mélange toluène/ acétate d'éthyle (9/1). Après évaporation on obtient 2.2g d'une poudre rouge.
Rendement : 76%
Spectrométrie de masse (Mode ES+) MH⁺ = 340.2

### Exemple 36 et 37

En procédant selon l'exemple 35-Etape A, on synthétise les composés de formule I, pour lesquels A représente un radical -CO-, décrits dans le Tableau ci-dessous, par alkylation du carbamate de tert-butyl en position 1 des indolizines avec le chlorure de 3-méthoxybenzyle en présence d'hydrure de sodium dans un solvant tel que le tétrahydrofurane ou le diméthylformamide

**TABLEAU III**

| **Exemple** | **R**_{**1**} | **R**_{**2**} | **R**_{**3**} | **R**_{**4**} | **Rendement (%)** | **Point de Fusion (°C) ou Spectro de Masse (MH+)** |
|---|---|---|---|---|---|---|
| 36 | N(BOC)Bn-3-OMe | Me | OMe | NO₂ | 91 | MH+ = 546.4 |
| 37 | N(BOC)Bn-3-OMe | Me | CO₂Me | NO2 | 80 | 65°C |

### Exemples 38 et 39

En procédant selon l'exemple 35-Etape B, on synthétise les composés de formule I, pour lesquels A représente un radical -CO-, décrits dans le Tableau IV ci-dessous, par déprotection de l'amine en position 1 des indolizines à l'aide d'acide trifluoroacétique.

**TABLEAU IV**

| **Exemple** | **R**_{**1**} | **R**_{**2**} | **R**_{**3**} | **R**_{**4**} | **Rendement (%)** | **Spectro de Masse (MH+)** |
|---|---|---|---|---|---|---|
| 38 | NHBn-3-OMe | Me | OMe | NO₂ | 89 | MH+ = 446.3 |
| 39 | NHBn-3-OMe | Me | CO₂Me | NH₂ | 99 | MH+ = 444.4 |

### Exemple 40

### [1 - (diméthylamino) 2-méthyl indolizin-3-yl] (3-méthoxy -4- nitrophényl) méthanone

A 44mg (1.1 m.mole) d'hydrure de sodium (à 60% en dispersion dans l'huile) en suspension dans 5ml de tétrahydrofurane refroidie à 0°C, on ajoute goutte à goutte 382mg (1.1 m.mole) du composé de l'exemple 21 en solution dans 10ml de tétrahydrofurane. L'introduction terminée, on laisse revenir à température ambiante 1 heure, puis on ajoute 69µl (1.1 m.mole) d'iodure de méthyle et on agite à température ambiante 17 heures.

Le milieu réactionnel est versé sur une solution aqueuse saturée de chlorure de sodium et extrait à l'acétate d'éthyle. La phase organique est décantée, lavée avec une solution aqueuse saturée de chlorure de sodium, séchée sur sulfate de sodium et concentrée sous pression réduite.

Le résidu est purifié par chromatographie sur colonne de silice en éluant avec un mélange toluène/acétate d'éthyle (95/5). On obtient 143mg d'une mousse orangée.
Rendement : 37%

### Exemple 41

### {1-[(3-méthoxybenzyl)(méthyl)amino]-2-méthylindolizin-3-yl}(3-méthoxy-4-nitrophényl)méthanone

A 542mg (1.22 m.mole) de {1-[(3-méthoxybenzyl)amino]-2-méthylindolizin-3-yl}(3-méthoxy-4-nitrophénylméthanone en solution dans 15ml de diméthylformamide, on ajoute 595mg (1.83 m.mole) de carbonate de césium et 83µl (1.34 m.mole) d'iodure de méthyle.

On chauffe le mélange réactionnel à 40°C pendant 21 heures.

On verse le mélange dans une solution de chlorure de sodium saturée et on extrait avec de l'acétate d'éthyle. La phase organique est séchée sur sulfate de sodium et concentrée sous pression réduite.

On purifie le produit par chromatographie sur gel de silice en éluant avec un mélange toluène/acétate d'éthyle (95/5).
On obtient une gomme rouge.
Rendement : 96%
Spectrométrie de masse (Mode ES+) MH+ = 460.3

### Exemple 42

### 2-amino-5-({1-[(3-méthoxybenzyl) (méthyl) amino]-2-méthylindolizin-3-yl} carbonyl) benzoate de méthyle

Ce composé est préparé selon le même procédé que celui décrit dans l'exemple ci-dessus à partir de 340mg (0.76 m.mole) de 2-amino-5-({1-[(3-méthoxybenzyl)amino]-2-méthylindolizin-3-yl}carbonyl)benzoate de méthyle. On obtient 260mg d'un solide orange.
Rendement : 80%
Point de fusion : 60°C

### Exemple 43

### 5-({1-[(3-méthoxybenzoyl)amino]-2-méthylindolizin-3-yl}carbonyl)-2-[2,2,2-trifluoroacétyl)amino]benzoate de méthyle

A 1.16g (7.6 m.moles) d'acide 3-méthoxybenzoïque dissous dans 30ml de diméthylformamide et 60ml de dichlorométhane, on ajoute 3.37g (7.6 m.moles) de benzotriazol-1-yloxy-tris(diméthylamino)phosphonium hexafluorophosphate (BOP) et 2.1ml de triéthylamine.

On agite à température ambiante pendant 15 minutes puis on ajoute 3.04g (7.2 m.moles) de 5-[(1-amino-2-méthylindolizin-3-yl)carbonyl]-2-[2,2,2-trifluoroacétyl) amino]benzoate de méthyle.

Après 16 heures d'agitation à température ambiante, on filtre le précipité jaune obtenu dans le milieu réactionnel et on le lave avec du dichlorométhane. On obtient 2.38g d'une poudre jaune.
Rendement : 60%
Point de fusion : 239°C

### Exemple 44 à 61

En procédant selon la procédure décrite ci-dessus, on synthétise les composés décrits dans le tableau V ci-dessous par couplage du (1-amino-2-méthylindolizin-3-yl)(3-méthoxy-4-nitrophényl)méthanone ou du 5-[(1-amino-2-méthylindolizin-3-yl)carbonyl]-2-[2,2,2-trifluoroacétyl)amino]benzoate de méthyle avec l'acide carboxylique approprié en présence de benzotriazol-1-yloxy-tris (diméthylamino) phosphonium hexafluorophosphate (BOP).

**TABLEAU V**

| **Exemple** | **R1** | **R2** | **R3** | **R4** | **Rendement (%)** | **Point de Fusion (°C)** |
|---|---|---|---|---|---|---|
| 44 | NHCOPh-4-Cl | Me | OMe | NO₂ | 76 | 255°C |
| 45 | NHCOPh-4-CO₂Me | Me | OMe | NO₂ | 88 | 274°C |
| 46 | NHCOPh-3-OMe | Me | OMe | NO₂ | 86 | 180°C |
| 47 | NHCOPh-3-OMe-4-NO₂ | Me | OMe | NO₂ | 60 | 286°C |
| 48 | NHCOPh-2,3-OMe | Me | CO₂Me | NHCOCF₃ | 87 | 215°C |
| 49 | NHCOPh-2,5-OMe | Me | CO₂Me | NHCOCF₃ | 83 | 214°C |
| 50 | NHCOPh-3,4-OMe | Me | CO₂Me | NHCOCF₃ | 76 | 260°C |
| 51 | NHCOPh-3,4,5-OMe | Me | CO₂Me | NHCOCF₃ | 83 | 259 °C |
| 52 | NHCOPh-3,5-OMe | Me | CO₂Me | NHCOCF₃ | 74 | 223°C |
| 53 | NHCOPh-3-OMe 4-Me | Me | CO₂Me | NHCOCF₃ | 82 | 251 °C |
| 54 | NHCOPh-3,4-méthylènediox | Me | CO₂Me | NHCOCF₃ | 79 | 245°C |
| 55 | NHCOPh-3-OMe 4-Cl | Me | CO₂Me | NHCOCF₃ | 82 | 264°C |
| 56 | NHCOPh-3-OMe 4-F | Me | CO₂Me | NHCOCF₃ | 27 | 260°C |
| 57 | NHCOPh-2,5-OMe 4-Cl | Me | CO₂Me | NHCOCF₃ | 82 | 242°C |
| 58 | NHCO-5-indolyl | Me | CO₂Me | NHCOCF₃ | 76 | 186°C |
| 59 | NHCOPh-3-OMe 4-CO₂Me | Me | CO₂Me | NHCOCF₃ | 77 | 191°C |
| 60 | NHCOPh-3-OCF₃ | Me | CO₂Me | NHCOCF₃ | 60 | 258°C |
| 61 | NHCOPh-2,4,5-OMe | Me | CO₂Me | NHCOCF₃ | 64 | 253°C |

### Exemple 62

### N-[3-(3-méthoxy-4-nitrobenzoyl)-2-méthylindolizin-1-yl] acétamide

A 410mg (1.26 m.mole) de (1-amino-2-méthylindolizin-3-yl)(3-méthoxy-4-nitrophényl)méthanone dissous dans 10ml de dichlorométhane, on ajoute 1.20ml (12.60 m.moles) d'anhydride acétique.

On agite le mélange réactionnel à température ambiante pendant 15 minutes. Le précipité obtenu est filtré et lavé avec de l'éther éthylique puis séché pour donner 295mg d'une poudre orange.
Rendement : 63%
Point de fusion : 238°C

### Exemple 63

### 3-méthoxy-N-[3-(3-méthoxy-4-nitrobenzoyl)-2-méthylindolizin-1-yl]-N-méthylbenzamide

A 466mg (1.01 m.mole) de 3-méthoxy-*N*-[3-(3-méthoxy-4-nitrobenzoyl)-2-méthylindolizin-1-yl]benzamide en solution dans 19ml de tétrahydrofurane, on ajoute 51mg d'hydrure de sodium (suspension dans l'huile 60%).

On agite à température ambiante pendant 10 minutes puis on ajoute 65µl d'iodure de méthyle. Après agitation pendant 2 heures, on ajoute de l'eau au milieu réactionnel puis on extrait avec de l'acétate d'éthyle. La phase organique est lavée avec une solution de chlorure de sodium saturée, séchée sur sulfate de sodium et concentrée sous pression réduite.

On purifie le produit par chromatographie sur gel de silice en éluant avec du dichlorométhane. On obtient 435mg d'un solide jaune.
Rendement : 91%
Point de fusion : 190°C

### Exemple 64

### 5-({1-[(3-méthoxybenzoyl)(méthyl)amino]-2-méthylindolizin-3-yl}carbonyl)-2-nitrobenzoate de méthyle

Ce composé est préparé selon le protocole décrit pour l'exemple ci-dessus par méthylation de 1.9g (3.9 m.moles) de 5-({1-[(3-méthoxybenzoyl)amino]-2-méthylindolizin-3-yl}carbonyl)-2-nitrobenzoate de méthyle avec l'iodure de méthyle. On obtient 1.85g d'un solide rouge.
Rendement : 84%
Point de fusion : 158.5°C

### Exemple 65

### [3-(3-méthoxy-4-nitrobenzoyl)-2-(trifluorométhyl)indolizin-1-yl]carboxylate d'éthyle

### ETAPE A

### Synthèse du bromure de 1-[2-(3-méthoxy-4-nitrophényl)-2-oxoéthyl] pyridinium

A 1.32g (4.82m.moles) de 2-bromo-1-(3-méthoxy-4-nitrophényl)-1-éthanone, décrit dans Bull. Soc. Chim. Fr., (1962), 2255-2261, en solution dans 13ml d'acétonitrile on ajoute 467µl (5.78m.moles) de pyridine et on agite à température ambiante pendant 5 heures.

Le milieu réactionnel est précipité.
On ajoute de l'éther éthylique, on filtre, on lave les cristaux à l'éther éthylique puis on sèche. On obtient 1.65g de cristaux jaunes.
Rendement : 97%
Point de fusion: 216°C.

### ETAPE B

A 219µl (1.56m.mole) de triéthylamine dans 4.5ml de diméthylformamide, on ajoute par portions 500mg (1.42m.mole) de bromure de 1-[2-(3-méthoxy-4-nitrophényl)-2-oxoéthyl] pyridinium puis 1.06ml (7.08m.moles) de 4,4,4-trifluorocrotonate d'éthyle et 561mg (0.92m.mole) de tétrapyridinecobalt (II) dichromate.

On porte le milieu réactionnel à 90°C pendant 6 heures. Le milieu réactionnel est refroidi puis versé sur de l'acide chlorhydrique 1N et le produit ainsi obtenu est extrait à l'acétate d'éthyle.

La phase organique est décantée, lavée à l'eau puis avec une solution aqueuse saturée de chlorure de sodium, séchée sur sulfate de sodium et concentrée sous pression réduite.

Le produit obtenu est purifié par chromatographie sur colonne de silice en éluant avec du dichlorométhane. On obtient 437mg d'une poudre jaune.
Rendement : 71%
Point de fusion : 63°C.

### Exemple 66

### [3-(3-méthoxy-4-nitrobenzoyl)indolizin-1-yl]carboxylate d'éthyle

Ce composé est obtenu selon le même procédé que l'exemple précédent à l'étape B par cycloaddition 1,3-dipolaire du bromure de 1-[2-(3-méthoxy-4-nitrophényl)-2-oxoéthyl]pyridinium (obtenu à l'étape A de l'exemple précédent) avec l'acrylate d'éthyle On obtient après purification par chromatographie flash sur colonne de silice en éluant au dichlorométhane une poudre jaune.
Rendement : 78%
Point de fusion : 168°C.

### Exemple 67

### (1-hydroxy 2-méthyl indolizin-3-yl) (3-méthoxy 4-nitrophényl) méthanone

Une solution de 5g (12m.moles) de [1-(benzyloxy) 2-méthyl indolizin-3-yl] (3-méthoxy 4-nitrophényl) méthanone composé de l'exemple 3, dans 30ml d'acide trifluoroacétique est portée au reflux pendant 2 heures.

Le milieu réactionnel est évaporé sous pression réduite. Le résidu est repris à l'acétate d'éthyle, lavé avec une solution aqueuse de bicarbonate de sodium et à l'eau puis la phase organique est séchée sur sulfate de sodium et évaporée sous pression réduite.

Le produit obtenu est purifié par chromatographie sur colonne de silice en éluant avec un mélange de dichlorométhane/méthanol (99/1). On obtient 2.93g d'une poudre orange.
Rendement : 75%
Point de fusion : 193°C

### Exemple 68

### 4-({[3-(3-méthoxy 4-nitrobenzoyl) 2-méthyl indolizin-1yl] oxy} méthyl) benzoate de méthyle

A une solution de 1g (3.06mmoles) de (1-hydroxy-2-méthyl-3-indolizinyl) (3-méthoxy-4-nitrophényl) méthanone dans 16ml de diméthylformamide en présence de 508mg (3.68mmoles) de carbonate de potassium, on ajoute 812mg (3.37mmoles) de 4-(bromométhyl)benzoate de méthyle et on chauffe à 90°C pendant 4 heures.

Le milieu réactionnel est versé sur de l'eau et extrait à l'acétate d'éthyle.

La phase organique est lavée à l'eau, séchée sur sulfate de sodium et évaporée à sec. Le produit obtenu est purifié par chromatographie sur colonne de silice en éluant avec un mélange de toluène/acétate d'éthyle (9/1).On obtient 880mg d'une poudre jaune.
Rendement : 60.5%
Point de fusion: 154°C.

### Exemples 69 à 84

En procédant selon le procédé décrit dans l'exemple 68, on synthétise les composés décrits dans le Tableau VI ci-dessous, par alkylation du (1-hydroxy 2-méthyl indolizin-3-yl) (3-méthoxy 4-nitrophényl) méthanone avec les dérivés halogénés convenablement choisis. Pour obtenir le composé de l'exemple 80 on soumet le composé de l'exemple 79 à une saponification.

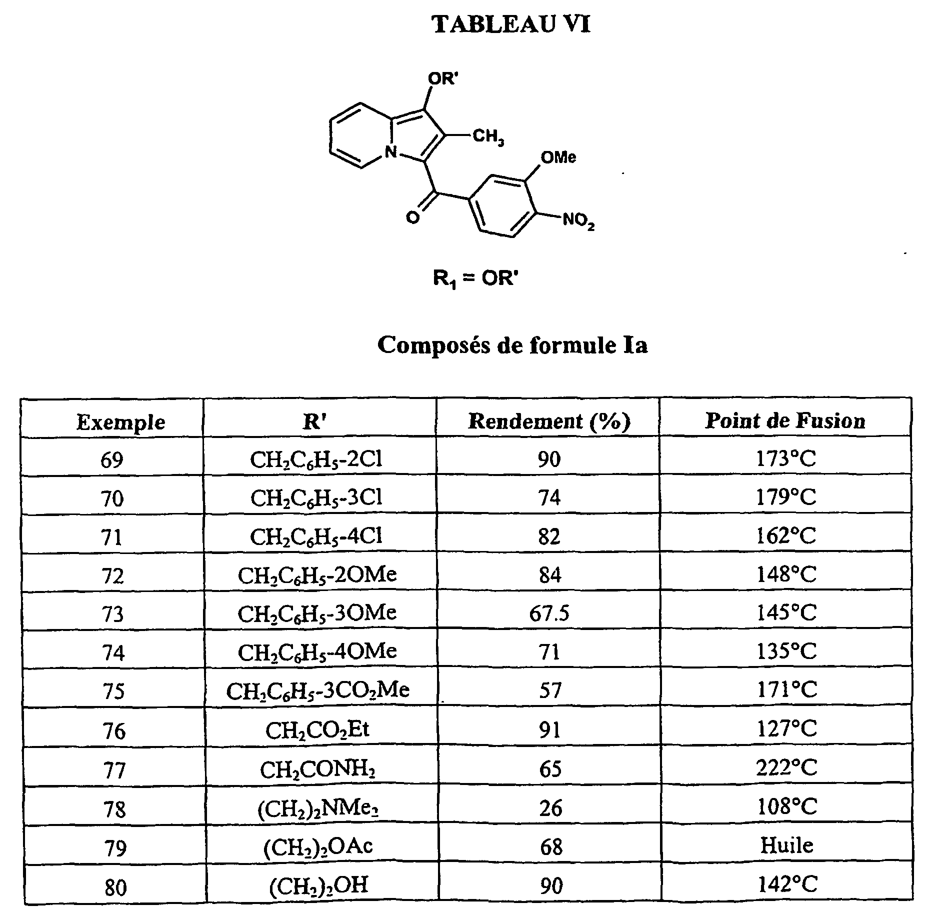

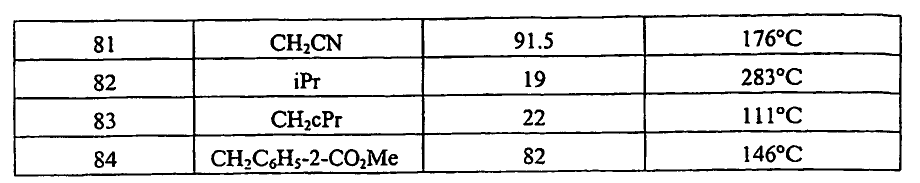

### Exemple 85

### 4-[(1-hydroxy 2-méthyl indolizin-3-yl) carbonyl] benzoate de méthyle

A 3.45g (8.64mmoles) de 4-[(1-(benzyloxy) 2-méthyl indolizin-3-yl) carbonyl] benzoate de méthyle dans 40ml d'éthanol en présence de 690mg de Pd/C à 10% on ajoute 8.75ml (86.37mmoles) de cyclohexène et on chauffe à reflux pendant une heure.

Le milieu réactionnel est refroidi à température ambiante, le catalyseur est éliminé par filtration sur talc. Le filtrat est sous pression réduite.

Le produit obtenu est purifié par chromatographie sur colonne de silice en éluant avec un mélange de dichlorométhane/méthanol (98/2). On obtient 2.5g d'une poudre orange.
Rendement : 93.5%
Point de fusion: 192°C

### Exemple 86

### 4-{[1-(2-ethoxy 2-oxoéthoxy) 2-méthyl indolizin-3-yl]carbonyl} benzoate de méthyle

A 500mg (1.62mmole) de 4-[(1-hydroxy 2-méthyl indolizin-3-yl) carbonyl] benzoate de méthyle, composé de l'exemple 85, dans 10ml de diméthylformamide en présence de 268mg (1.94mmole) de carbonate de potassium, on ajoute 202µl (1.78mmole) de bromoacétate d'éthyle et on chauffe à 90°C pendant une heure.

On refroidit, on verse le milieu réactionnel sur de l'eau et on extrait à l'acétate d'éthyle puis on décante. La phase organique est lavée à l'eau, séchée sur sulfate de sodium et évaporée sous pression réduite. Le produit obtenu est purifié par chromatographie sur colonne de silice en éluant avec un mélange de toluène/acétate d'éthyle (9/1).

On obtient 570mg d'une poudre jaune.
Rendement : 89%
Point de fusion : 84.5°C

### Exemple 87

### 4-({1-[(3-méthoxybenzyl)oxy] 2-méthyl indolizin-3-yl} carbonyl) benzoate de méthyle

Ce composé est obtenu selon le même mode opératoire que celui de l'exemple 86, par O-alkylation du 4-[(1-hydroxy 2-méthylindolizin-3-yl) carbonyl] benzoate de méthyle avec le bromure de 3-méthoxybenzyle. On obtient une poudre jaune qui fond à 106°C.
Rendement : 76%.

### Exemple 88

### Acide 3-(3-méthoxy 4-nitrobenzoyl) 2-méthyl indolizin-1-yl carboxylique

A 5g (13.1 m.moles) de 3-(3-méthoxy 4-nitrobenzoyl) 2-méthyl indolizin-1-yl carboxylate d'éthyle, composé de l'exemple 13 - préparé selon le mode opératoire de l'exemple 1 par benzoylation du (2-méthylindolizin-1-yl) carboxylate d'éthyle décrit dans *J. Chem. Soc.*,(1963), pp3277-3280 -, en suspension dans 50ml de dioxanne on ajoute 26.2ml de soude 1N et chauffe à reflux 17 heures. Le milieu réactionnel est concentré sous pression réduite. Le résidu est repris à l'eau et lavé à l'éther éthylique, décanté. La phase aqueuse est acidifiée à pH 6 avec une solution d'hydrogénosulfate de potassium et extraite à l'acétate d'éthyle. La phase organique est lavée à l'eau, séchée sur sulfate de sodium et concentrée sous pression réduite. On obtient 4.9g d'une poudre orange. Rendement quantitatif.
Point de fusion: 215°C

### Exemple 89

### N-éthyl 3-(3-méthoxy 4-nitrobenzoyl) 2-méthyl indolizin-1-yl carboxamide

A une solution de 750mg (2.12m.moles) d'acide de l'exemple 88 dans 12ml de diméthylformamide, on ajoute 0.61ml (4.34m.moles) de triéthylamine et par portions 983mg (2.22m.moles) de benzotriazol-1-yl oxy-tris (diméthylamino) phosphonium hexafluorophosphate. On agite 5mn à température ambiante puis on ajoute 182mg (2.22 m.moles) de chlorhydrate d'éthylamine.

Le milieu réactionnel est agité une nuit à température ambiante, versé sur de l'eau et extrait à l'acétate d'éthyle. La phase organique est décantée, lavée à l'eau, séchée sur sulfate de sodium et concentrée sous pression réduite.

Le produit est purifié par chromatographie sur colonne de silice en éluant avec dichlorométhane/méthanol (98/2). On obtient 700mg d'une poudre jaune.
Rendement : 87%
Point de fusion: 188°C

### Exemple 90

### Ethyl 2-({[3-(3-méthoxy 4-nitrobenzoyl) 2-méthylindolizin-1-yl] carbonyl} amino) acétate

Ce composé est obtenu selon le même procédé que le composé précédent, par couplage de l'acide 3-(3-méthoxy 4-nitrobenzoyl) 2-méthyl indolizin-1-yl carboxylique avec le chlorhydrate du glycinate d'éthyle.

Le produit est purifié par chromatographie sur colonne de silice en éluant avec dichlorométhane/méthanol (93/7). On obtient une poudre jaune.
Rendement : 86%
Point de fusion : 191°C

### Exemple 91

### 1-méthoxy 2-méthyl 3-[(4-nitrophényl) sulfonyl] indolizine

A 500mg (3.1m.moles) de 1-méthoxy 2-méthylindolizine dissout dans 8ml de 1,2-dichloroéthane, on ajoute 690mg (3.1m.moles) de chlorure de 4-nitrobenzène sulfonyle en solution dans 4ml de 1,2-dichloroéthane et agite à température ambiante pendant 20 heures. Le milieu réactionnel est versé sur de l'eau et du dichlorométhane. La phase organique est décantée, lavée à l'eau, séchée sur sulfate de sodium et concentrée sous pression réduite.

Le produit est purifié par chromatographie sur colonne de silice en éluant avec cyclohexane/acétate d' éthyle (9/1). On obtient 330mg d'une huile jaune.
Rendement: 31%

### Exemple 92

### 1-méthoxy 2-méthyl 3-[(3-nitrophényl) sulfonyl] indolizine

Ce composé est préparé selon le protocole décrit pour l'exemple ci-dessus par sulfonylation de 1g (6.2 m.moles) de 1-méthoxy 2-méthylindolizine avec le chlorure de 3-nitrobenzène sulfonyle. On obtient 540mg d'une huile jaune.
Rendement : 98%

### Exemple 93

### Sel de sodium de l'acide 4-[(1-méthoxy 2-méthyl indolizin-3-yl)sulfonyl) benzoïque

Ce composé est obtenu selon le même opératoire que le composé de l'exemple 91, par sulfonylation de la 1-méthoxy 2-méthylindolizine avec l'acide 4-chlorosulfonyl benzoïque. Le produit est purifié par chromatographie flash sur colonne de silice en éluant avec dichlorométhane/acétone (9/1). On obtient 120mg d'une poudre jaune.
Rendement : 11%.

Le produit dissout dans le méthanol est salifié par ajout d'un équivalent de soude 1N. Le méthanol est évaporé et le résidu est cristallisé dans l'acétone. Le produit est filtré, lavé à l'acétone puis à l'éther éthylique et séché. On obtient 100mg de sel de sodium sous forme d'une poudre jaune.
Point de fusion : 175°C.

### Exemple 94

### (4-amino 3-méthoxyphényl)(1-méthoxy 2-méthyl indolizin-3-yl) méthanone

A 6g (0.0176mole) de (1-methoxy-2-méthyl-3-indolizinyl)(3-méthoxy-4-nitrophényl) méthanone, composé de l'exemple 1, dans 100ml d'éthanol, on ajoute 700mg de Pd/C à 10% puis 35.71ml (0.352mole) de cyclohexène et on chauffe à reflux 2 heures. Le milieu réactionnel est refroidi, filtré sur talc et lave le catalyseur au dichlorométhane. Le filtrat est concentré sous pression réduite. Le résidu est repris au dichlorométhane. On lave la phase organique à la soude 1N puis à l'eau, on sèche sur sulfate de sodium et on concentre sous pression réduite. On recueille 5.05g d'une poudre jaune.

Le produit est salifié par dissolution de la poudre précédemment obtenue dans 60ml de dichlorométhane plus 20ml de méthanol puis l'ajout de 21ml d'acide chlorhydrique 1N dans l'éther éthylique. Après addition d'éther éthylique, le précipité obtenu est filtré, lavé à l'éther éthylique puis séché. On recueille 5.4g d'une poudre jaune sous forme de chlorhydrate.
Rendement : 88%
Point de fusion : 198°C

### Exemples 95 à 117

En procédant selon la préparation décrite ci-dessus, on synthétise les composés décrits dans le Tableau VII ci-dessous, par réduction de la fonction nitro des composés de formule Ia avec le cyclohexène en présence de Pd/C à 10% comme catalyseur.

**TABLEAU VII**

| **Exemple** | **R**_{**1**} | **R**_{**2**} | **R**_{**3**} | **R**_{**4**} | **Rdt (%)** | **Sels** | **Point de fusion ou Spectro de Masse (MH**^{**+**}**)** |
|---|---|---|---|---|---|---|---|
| 95 | OMe | C₆H₅ | OMe | NH₂ | 90 | HCl, 0.45H₂O | 209°C |
| 96 | OMe | cPr | OMe | NH₂ | 95 | HCl, 0.15H₂O | 191°C |
| 97 | CO₂Et | Me | OMe | NH₂ | 91 | HCl | 194°C |
| 98 | OCH₂CO₂Et | Me | OMe | NH₂ | 99 | HCl | 182°C |
| 99 | OCH₂CONH₂ | Me | OMe | NH₂ | 87 | HCl | MH⁺=354.1 |
| 100 | O(CH₂)₂OH | Me | OMe | NH₂ | 89 | HCl, 0.5H₂O | 205°C |
| 101 | OMe | Me | H | NH₂ | 86 | HCl, 0.2H₂O | 221°C |
| 102 | CONHEt | Me | OMe | NH₂ | 72 | HCl, 0.45H₂O | 221°C |
| 103 | CONHCH₂CO₂Et | Me | OMe | NH₂ | 91 | HCl, 1.05H₂O | 196°C |
| 104 | OMe | Me | CO₂Me | NH₂ | 87 | 0.4H₂O | 297°C |
| 105 | CO₂Et | Me | CO₂Me | NH₂ | 95 | - | 172°C |
| 106 | OMe | Me | NH₂ | OMe | 82 | HCl | 209°C |
| 107 | CO₂Et | C₆H₅ | OMe | NH₂ | 86 | - | 180°C |
| 108 | CO₂Et | Me | NH₂ | OMe | 85 | | 162°C |
| 109 | CO₂Et | CF₃ | OMe | NH₂ | 81 | - | 75°C |
| 110 | CO₂Et | H | OMe | NH₂ | 89 | - | 143°C |
| 111 | NHCOPh-4-CO₂Me | Me | OMe | NH₂ | 72 | HCl | 275°C |
| 112 | NHCOPh-3-OMe | Me | OMe | NH₂ | 77 | HCl, 0.4H₂O | 209°C |
| 113 | NHCOPh(3-OMe)4-NH₂ | Me | OMe | NH₂ | 82 | 2HCl, 1H₂O | 178°C |
| 114 | NHAc | Me | OMe | NH₂ | 57 | HCl, 0.35H₂O | 253°C |
| 115 | N(Me)COPh-3-OMe | Me | OMe | NH₂ | 98 | HCl | 113°C |
| 116 | N(Me)COPh-3-OMe | Me | CO₂Me | NH₂ | 99 | - | 91°C |
| 117 | N(BOC)COPh-3-OMe | Me | CO₂Me | NH₂ | 98 | - | 82°C |

### Exemple 118

### Chlorhydrate de (4-amino 3-méthoxyphényl) {1-[(2-chlorobenzyl) oxy] 2-méthyl indolizin-3-yl} méthanone

A 470mg (1.04m.mole) de {1-[(2-chlorobenzyl)oxy] 2-méthyl indolizin-3-yl}(3-méthoxy 4-nitrophényl) méthanone dans 5ml de méthanol et 10ml de dichlorométhane, on ajoute 47mg de Pd/C à 10% puis 253µl (5.21m.mole) d'hydrate d'hydrazine et on agite à température ambiante une nuit. Le milieu réactionnel est filtré sur talc et lave le catalyseur au méthanol.

Le filtrat est concentré sous pression réduite. Le résidu est repris à l'acétate d'éthyle, on lave la phase organique avec une solution aqueuse saturée de chlorure de sodium, on sèche sur sulfate de sodium et concentre sous pression réduite. On recueille 460mg d'une poudre jaune.

Le produit est salifié par dissolution de la poudre précédemment obtenue dans un mélange d'acétate d'éthyle et de méthanol puis on ajoute 1.25ml (1.2 équivalent) d'acide chlorhydrique 1N dans l'éther éthylique. Après addition d'éther éthylique, le précipité obtenu est filtré, lavé à l'éther éthylique puis séché. On recueille 440mg d'une poudre jaune sous forme de chlorhydrate 0.65H₂O.
Rendement : 90%
Point de fusion : 177°C

### Exemple 119 à 140

En procédant selon la préparation décrite à l'exemple 118, on synthétise les composés décrits dans le Tableau VIII ci-dessous, par réduction de la fonction nitro des composés de formule la avec l'hydrate d'hydrazine en présence de Pd/C à 10% comme catalyseur.

**TABLEAU VIII**

| **Exemple** | **A** | **R**_{**1**} | **R**_{**2**} | **R**_{**3**} | **R**_{**4**} | **Rdt (%)** | **Sels** | **Point de fusion ou Spectro de Masse (MH**^{**+**}**)** |
|---|---|---|---|---|---|---|---|---|
| 119 | CO | OBn | C₆H₅ | OMe | NH₂ | 94 | HCl, 0.2H₂O | 207°C |
| 120 | CO | O(CH₂)₂NMe₂ | Me | OMe | NH₂ | 31 | 2HCl, 2H₂O | 246°C |
| 121 | CO | OBn-4-Cl | Me | OMe | NH₂ | 99 | HCl | 177°C |
| 122 | CO | OBn-3-OMe | Me | OMe | NH₂ | 95 | HCl | 181°C |
| 123 | CO | OBn-4-OMe | Me | OMe | NH₂ | 99 | HCl, 0.3H₂O | 128°C |
| 124 | CO | OBn-2-OMe | Me | OMe | NH₂ | 99 | HCl | 164°C |
| 125 | CO | OBn-3-CO₂Me | Me | OMe | NH₂ | 75 | HCl | 185°C |
| 126 | CO | OBn-4-CO₂Me | Me | OMe | NH₂ | 93 | HCl, 1H₂O | 160°C |
| 127 | CO | OBn-3-Cl | Me | OMe | NH₂ | 96 | HCl | 175°C |
| 128 | CO | N(Me)Bn | Me | OMe | NH₂ | 78 | HCl, 1.6H₂O | 114°C |
| 129 | CO | NHBOC | Me | OMe | NH₂ | 95 | base | MH⁺= 396.4 |
| 130 | CO | NHMe | Me | OMe | NH₂ | 88 | HCl, 1.15H₂O | 210°C |
| 131 | CO | NHSO₂Me | Me | OMe | NH₂ | 83 | HCl | 228°C |
| 132 | CO | OMe | Me | NH₂ | CO₂ Me | 72 | - | 135°C |
| 133 | SO₂ | OMe | Me | H | NH₂ | 66 | - | 157°C |
| 134 | SO₂ | OMe | Me | NH₂ | H | 45 | - | 137°C |
| 135 | CO | OCH₂cPr | Me | OMe | NH₂ | 99 | HCl | 181°C |
| 136 | CO | OiBu | Me | OMe | NH₂ | 60 | HCl | 103°C |
| 137 | CO | NMe₂ | Me | OMe | NH₂ | 80 | 2HCl, 0.2H₂O | 171°C |
| 138 | CO | OBn-2-CO₂Et | Me | OMe | NH₂ | 98 | HCl, 0.5H₂O | 185°C |
| 139 | CO | NHBn-3-OMe | Me | OMe | NH₂ | 72 | HCl | 186°C |
| 140 | CO | N(Me)Bn-3-OMe | Me | OMe | NH₂ | 95 | HCl, 1.5H₂O | 161°C |

### Exemple 141

### 4-chloro-N-[3-(4-amino-3-méthoxybenzoyl)-2-méthylindolizin-1-yl] benzamide

Un mélange de 384mg (0.83 m.mole) de 4-chloro-*N*-[3-(3-méthoxy-4-nitrobenzoyl)-2-méthylindolizin-1-yl] benzamide et 115mg d'oxyde de platine dans 9ml de diméthylformamide est agité sous 5 bars d'hydrogène à température ambiante pendant 24 heures, puis filtré sur talc. Le filtrat est concentré sous pression réduite.

Le produit est purifié par chromatographie sur gel de silice en éluant avec toluène/acétone (9/1 à 8/2). A la poudre jaune obtenue mise en suspension dans 5ml de dichlorométhane et 5ml de méthanol, on ajoute 1ml d'acide chlorhydrique 1N dans l'éther éthylique. Le précipité obtenu est filtré, lavé à l'acétone puis dissout dans 2ml de méthanol et 40ml d'eau. Le chlorhydrate ainsi obtenu est lyophilisé. On obtient 162 mg d'une poudre orange.
Rendement : 50%
Point de fusion : 191°C

### Exemple 142

### [1-(2-hydroxyéthoxy) 2-méthyl indolizin-3-yl] (3-méthoxy-4-nitro-phényl) méthanone

A 420mg (1.02 m.mole) d'acétate de 2-{[3-(3-méthoxy 4-nitrobenzoyl) 2-méthyl indolizin-1-yl] oxy} éthyle, composé de l'exemple 79 dissout dans 6ml de dioxanne, on ajoute 1.52ml de soude 1N et agite à température ambiante 6 heures. Le milieu réactionnel est versé sur de l'eau et de l'acétate d'éthyle. La phase organique est décantée, lavée à l'eau, séchée sur sulfate de sodium et concentrée sous pression réduite. On obtient 340mg d'une poudre orange utilisée sans autre purification dans l'étape ultérieure de réduction du nitro.
Rendement : 90%
Point de fusion : 142°C.

### Exemple 143

### Sel de sodium d'acide 4-[(1-méthoxy-2-méthyl-3-indolizinyl) carbonyl] benzoïque

A 720mg (2.23 m.moles) de 4-[(1-méthoxy 2-méthyl indolizin-3-yl) carbonyl] benzoate de méthyle, composé de l'exemple 8) en solution dans 15ml de méthanol plus 15ml de dioxanne on ajoute 2.45ml de soude 1N et on agite à température ambiante une nuit. Le milieu réactionnel est concentré sous pression réduite. Le résidu est repris à l'eau, on lave à l'éther éthylique et on décante. La phase aqueuse est acidifiée avec de l'acide chlorhydrique 1N et extrait au dichlorométhane.

La phase organique est lavée à l'eau séchée sur sulfate de sodium et concentrée sous pression réduite. On obtient 700mg d'une poudre orange que l'on met en suspension dans 20ml de méthanol puis on ajoute un équivalent de soude 1N. La solution obtenue est concentrée sous pression réduite. Le résidu est cristallisé dans l'acétone. Le produit est filtré, lavé à l'acétone puis à l'éther éthylique On sèche et on obtient 680mg de poudre jaune.
Rendement (sel de Na) : 92%
Point de fusion > 400°C.

### Exemples 144 à 157

En procédant selon le procédé décrit dans l'exemple 143 on synthétise les composés décrits dans le Tableau IX ci-dessous, par saponification de la fonction ester des composés de formule Id

**TABLEAU IX**

| **Exemple** | **R**_{**1**} | **R**_{**2**} | **R**_{**3**} | **R**_{**4**} | **Rdt (%)** | **Sels** | **Point de fusion** |
|---|---|---|---|---|---|---|---|
| 144 | OMe | Me | CO₂H | H | 76 | Na | 218°C |
| 145 | OMe | Me | NO₂ | CO₂H | 85 | Na | 265°C |
| 146 | OMe | Me | NH₂ | CO₂H | 77 | Na | 315°C |
| 147 | OBn-3-OMe | Me | H | CO₂H | 81 | Na, 0.7H₂O | 268°C |
| 148 | OMe | Me | OMe | CO₂H | 87 | Na, 1H₂O | .235°C |
| 149 | OMe | Me | H | CH₂CO₂H | 91 | Na, 0.7H₂O | 248°C |
| 150 | OMe | Me | CO₂H | NH₂ | 98 | Na, 1H₂O | 258°C |
| 151 | OMe | Me | CO₂H | NO₂ | 83 | Na, 0.95H₂O | 164°C |
| 152 | OMe | Me | CO₂H | OMe | 92 | Na, 0.65H₂O | 318°C |
| 153 | H | Me | CO₂H | NH₂ | 95 | Na, 1.3H₂O | 300°C |
| 154 | OMe | cPr | CO₂H | NH₂ | 100 | Na, 1.75H₂O | 249°C |
| 155 | N(Me)COPh-3-OMe | Me | CO₂H | NH₂ | 77 | Na, 3.2 H₂O | 230°C |
| 156 | NHBn-3-OMe | Me | CO₂H | NH₂ | 83 | Na, 1.15H₂O | 164°C |
| 157 | N(Me)Bn-3-OMe | Me | CO₂H | NH₂ | 78 | Na, 1.2H₂O | 211°C |

### Exemple 158

### Acide 2-amino-5-({1-[(3-méthoxybenzoyl)amino]-2-méthylindolizin-3-yl} carbonyl) benzoique

A 3.31g (6.0 m.moles) de 5-({1-[(3-méthoxybenzoyl)amino]-2-méthylindolizin-3-yl}carbonyl)-2-[2,2,2-trifluoroacétyl)amino]benzoate de méthyle en suspension dans 40ml de dioxane et 20ml de méthanol, on ajoute 6.6ml d'une solution de soude (2N). On chauffe le milieu réactionnel à reflux pendant 2.5 heures, puis laisse revenir à température ambiante et concentre sous pression réduite. On reprend le résidu obtenu dans une solution aqueuse saturée de bicarbonate de sodium et on extrait avec l'acétate d'éthyle. Après décantation, on acidifie la phase aqueuse avec une solution molaire d'acide chlorhydrique. Le précipité obtenu est filtré, lavé abondamment à l'eau et séché sous vide. On obtient 2.4g d'une poudre jaune.
Rendement : 90%
Point de fusion : 290°C
Sel de Na, monohydrate : Point de fusion : 265°C

### Exemples 159 à 174

En procédant selon la procédure décrite ci-dessus, on synthétise les composés de formule I dans laquelle A représente - CO- décrits dans le tableau X ci-dessous par hydrolyse de l'ester méthylique et du trifluroacétamide de R3 et R4 avec de la soude

**TABLEAU X**

| **Exemple** | **R1** | **R2** | **R3** | **R4** | **Rendement (%)** | **Sels** | **Point de Fusion (°C)** |
|---|---|---|---|---|---|---|---|
| 159 | NHCOPh-2,3-OMe | Me | CO₂H | NH₂ | 75 | Na, 3.0 H₂O | 236°C |
| 160 | NHCOPh-2,5-OMe | Me | CO₂H | NH₂ | 77 | Na, 2.5 H₂O | 265°C |
| 161 | NHCOPh-3,4-OMe | Me | CO₂H | NH₂ | 79 | Na, 2.0 H₂O | 331°C |
| 162 | NHCOPh-3,4,5-OMe | Me | CO₂H | NH₂ | 92 | Na, 1.5 H₂O | 349°C |
| 163 | NHCOPh-3,5-OMe | Me | CO₂H | NH₂ | 71 | Na, 2.0 H₂O | 293°C |
| 164 | NHCOPh-(3-OMe)4-Me | Me | CO₂H | NH₂ | 78 | Na, 1.0 H₂O | 277°C |
| 165 | NHCOPh-3,4-methylènedioxy | Me | CO₂H | NH₂ | 94 | Na, 1.8 H₂O | >400°C |
| 166 | NHCOPh(4-Cl)-3-OMe | Me | CO₂H | NH₂ | 67 | Na, 3.0 H₂O | 320°C |
| 167 | NHCOPh(4-F)3-OMe | Me | CO₂H | NH₂ | 72 | Na, 2.25 H₂O | 276°C |
| 168 | NHCOPh(4-Cl)2.5-OMe | Me | CO₂H | NH₂ | 82 | Na, 2.5 H₂O | 280°C |
| 169 | NHCO-5-indolyl | Me | CO₂H | NH₂ | 86 | Na, 2.8 H₂O | 296°C |
| 170 | NHCOPh(3-OMe)4-CO₂H | Me | CO₂H | NH₂ | 74 | 2Na, 2.5 H₂O | 323°C |
| 171 | NHCOPh-3-OCF₃ | Me | CO₂H | NH₂ | 76 | Na, 1.5 H₂O | 321°C |
| 172 | NHCOPh-2,4,5-OMe | Me | CO₂H | NH₂ | 53 | Na, 2.5 H₂O | 272°C |
| 173 | NHSO₂Ph-3-OMe | Me | CO₂H | NH₂ | 50 | Na, 2 H₂O | 238°C |
| 174 | NHCONHPh-3-OMe | Me | CO₂H | NH₂ | 75 | Na, 1.2 H₂O | 378°C |

### Exemple 175

### Acide 3-(4-amino 3-méthoxybenzoyl) 2-méthylindolizin-1-yl carboxylique

A 2.1g (5.96 m.moles) de 3-(4-amino-3-méthoxybenzoyl)-2-méthyl-1-indolizine carboxylate d'éthyle en solution dans 30ml de dioxanne on ajoute 30ml de soude 2N et on chauffe à reflux pendant 20 heures. Le milieu réactionnel est concentré sous pression réduite.

Le résidu est repris à l'eau, lavé à l'éther éthylique, décanté. La phase aqueuse est acidifiée à pH 6.5 avec une solution aqueuse à 10% d'hydrogénosulfate de potassium et extraite à l'acétate d'éthyle. La phase organique est lavée à l'eau séchée sur sulfate de sodium et concentrée sous pression réduite. On obtient 1.8g d'une poudre jaune.
Rendement : 93%
Deux sels du composé sont ensuite préparés :
Sel de Sodium monohydrate ; Point de fusion : 224°C Chlorhydrate ; Point de fusion : 213°C

### Exemples 176 à 185

En procédant selon la préparation décrite ci-dessus, on synthétise les composés décrits dans le Tableau XI ci-dessous, par saponification de la fonction ester contenue dans le substituant R₁ des composés de formule Id, dans laquelle A représente un radical -CO- avec de la soude.

**TABLEAU XI**

| **Exemple** | **R**_{**1**} | **R**_{**2**} | **R**_{**3**} | **R**_{**4**} | **Rendement (%)** | **Sels** | **Point de Fusion** (°C) |
|---|---|---|---|---|---|---|---|
| 176 | OCH₂CO₂H | Me | 3-OMe | 4-NH₂ | 91 | - | 227°C |
| 177 | CONHCH₂CO₂H | Me | 3-OMe | 4-NH₂ | 90 | Na, 0.95H₂O | 297°C |
| 178 | OBn-3-CO₂H | Me | 3-OMe | 4-NH₂ | 84 | Na,1.25H₂O | 207°C |
| 179 | OBn-4-CO₂H | Me | 3-OMe | 4-NH₂ | 76 | Na, 0.7H₂O | 216°C |
| 180 | CO₂H | C₆H₅ | 3-OMe | 4-NH₂ | 84 | Na, 1.25H₂O | 305°C |
| 181 | OBn-2-CO₂H | Me | 3-OMe | 4-NH₂ | 100 | Na,1.5H₂O | Déc.174 |
| 182 | CO₂H | CF₃ | 3-OMe | 4-NH₂ | 87 | Na, 1H₂O | 330°C |
| 183 | CO₂H | H | 3-OMe | 4-NH₂ | 90 | Na,1.9H₂O | 254°C |
| 184 | CO₂H | Me | 3-NH₂ | 4-OMe | 91 | Na, 1H₂O | 225°C |
| 185 | NHCOPh-4-CO₂H | Me | OMe | NH₂ | 48 | HCl | 256°C |

### Exemple 186

### Sel disodique d'acide 3-(4-carboxybenzoyl) 2-méthyl indolizin-1-yl carboxylique

A 910mg (2.49 m.moles) de 3-[4-(méthoxycarbonyl) benzoyl] 2-méthyl indolizin-1-yl carboxylate d'éthyle en solution dans 20ml de dioxanne plus 20ml d'éthanol, on ajoute 7.47ml de soude 1N et on chauffe à reflux pendant 6 heures. Le milieu réactionnel est concentré sous pression réduite. Le résidu est repris à l'eau, on lave à l'éther éthylique et on décante. La phase aqueuse est acidifiée avec de l'acide chlorhydrique 1N et extraite à l'acétate d'éthyle. La phase organique est lavée à l'eau séchée sur sulfate de sodium et concentrée sous pression réduite. On obtient 650mg d'une poudre jaune que l'on met en suspension dans 20ml de méthanol puis ajoute 4.02ml de soude 1N (2 éq.).

La solution obtenue est concentrée sous pression réduite. Le résidu est cristallisé dans l'acétone. Le produit est filtré, lavé à l'acétone puis à l'éther éthylique et séché. On obtient 700mg de poudre jaune.
Rendement sel disodique dihydraté : 81%
Point de fusion > 400°C.

### Exemples 187 à 191

En procédant selon l'exemple 186, on synthétise les composés décrits dans le Tableau XII ci-dessous, par saponification des fonctions esters contenues dans les substituants R₁ et R₄ des composés de formule I, dans laquelle A représente un radical -CO-, avec de la soude 1N

**TABLEAU XII**

| **Exemple** | **R**_{**1**} | **R**_{**2**} | **R**_{**3**} | **R**_{**4**} | **Rendement (%)** | **Sels** | **Point de Fusion (°C)** |
|---|---|---|---|---|---|---|---|
| 187 | OCH₂CO₂H | Me | H | CO₂H | 90 | 2Na, 2H₂O | >400°C |
| 188 | CO₂H | Me | OMe | CO₂H | 96 | 2Na,1.5H₂O | 323°C |
| 189 | CO₂H | Me | CO₂H | NH₂ | 82 | 2Na, 1.9H₂O | 336°C |
| 190 | CO₂H | Me | CO₂H | NO₂ | 96 | 2Na, 2.5H₂O | 321°C |
| 191 | CO₂H | Me | CO₂H | OMe | 66 | 2Na, 1.4H₂O | 310°C |

### Exemple 192

### Chlorhydrate de (4-{[3-(dibutylamino) propyl]amino} 3-méthoxyphényl) (1-méthoxy 2-méthyl indolizin-3-yl) méthanone

A 278.4mg (2.25m.moles) de tertiobutylate de potassium dans 5ml de tétrahydrofurane, on ajoute 700mg (2.25 m.moles) de (4-amino 3-méthoxyphényl) (1-méthoxy 2-méthylindolizin-3-yl) méthanone, composé décrit à l'exemple 94 dissout dans 5ml de tétrahydrofurane et on agite 15 minutes à température ambiante.

On ajoute ensuite 510.5mg (2.48 m.moles) de chlorure de dibutylaminopropyle dans 5ml de tétrahydrofurane et on porte au reflux pendant une nuit.

Le milieu réactionnel est refroidi et versé sur de l'eau puis extrait à l'acétate d'éthyle. La phase organique est décantée, lavée à l'eau, séchée sur sulfate de sodium et concentrée sous pression réduite.

Le produit est purifié par chromatographie sur colonne de silice en éluant avec un mélange de dichlorométhane / acétone (9/1) puis (1/1).

On obtient 700mg d'une résine orangée que l'on salifie dans l'éther éthylique par ajout d'un équivalent d'acide chlorhydrique 1N dans l'éther éthylique.

Les cristaux obtenus sont filtrés, lavés à l'éther éthylique et séchés. On obtient une poudre orangée sous forme chlorhydrate, 1.25 H₂O.
Rendement : 65%
Point de fusion : 51°C

### Exemple 193

### Chlorhydrate de [3-méthoxy 4-(méthylamino) phényl] (1-méthoxy 2-méthyl indolizin-3-yl) méthanone

Ce composé est obtenu selon le même mode opératoire que celui décrit à l'exemple 192 par alkylation de la (4-amino 3-méthoxyphényl) (1-méthoxy 2-méthylindolizin-3-yl) méthanone avec l'iodure de méthyle. On obtient une poudre jaune.
Rendement : 45%
Point de fusion : 172°C

### Exemple 194

### Dichlorhydrate de (4-([3-(dibutylamino) propyl] amino} 3-méthoayphényl) (1-méthoxy 2-phényl indolizin-3-yl) méthanone

Obtenu selon le même mode opératoire que celui décrit dans l'exemple 192 par alkylation de la (4-amino 3-méthoxyphényl) (1-méthoxy 2-phényl indolizin-3-yl) méthanone, composé de l'exemple 95) avec le chlorure de dibutylaminopropyle. On obtient une poudre orangée (dichlorhydrate, 1.3 H2O.)
Rendement : 37%
Point de fusion: 158°C

### Exemple 195

### 2-{2-méthoxy 4-[(1-méthoxy 2-méthyl indolizin-3-yl) carbonyl] anilino} acétate d'éthyle

Ce composé a été obtenu selon le même mode opératoire que celui décrit dans l'exemple 192 par alkylation de la (4-amino 3-méthoxyphényl) (1-méthoxy 2-méthylindolizin-3-yl) méthanone, composé de l'exemple 94 avec le bromoacétate d'éthyle. On obtient une poudre jaune.
Rendement : 60.5%
Point de fusion : 125°C

### Exemple 196

### Acide 2-{2-méthoxy 4-[(1-méthoxy 2-méthyl indolizin-3-yl) carbonyl] anilino} acétique

A 1g (2.52 m.moles) de 2-{2-méthoxy-4-[(1-méthoxy 2-méthyl indolizin-3-yl) carbonyl] anilino} acétate d'éthyle, composé obtenu à l'exemple 195, en solution dans 10ml d'éthanol on ajoute 3.15ml de soude 1N et on agite à température ambiante pendant une nuit. Le milieu réactionnel est concentré sous pression réduite. Le résidu est repris à l'eau, lavé à l'éther éthylique et décanté. La phase aqueuse est neutralisée avec de l'acide chlorhydrique 1N. Le précipité formé est filtré, lavé à l'eau, séché puis repris à l'éther éthylique, filtré et séché. On obtient une poudre jaune.
Rendement : 48.5%
Point de fusion : 196°C

### Exemple 197

### 2-{2-méthoxy 4-[(1-méthoxy 2-phényl indolizin-3-yl) carbonyl] anilino} acétate d'éthyle

Ce composé a été obtenu selon le procédé décrit dans l'exemple 192 par alkylation de la (4-amino 3-méthoxyphényl) (1-méthoxy 2-phényl indolizin-3-yl) méthanone, composé de l'exemple 95 avec le bromoacétate d'éthyle. On obtient une poudre jaune.
Rendement : 78%
Point de fusion : 132°C

### Exemple 198

### Acide 2-{2-méthoxy 4-[(1-méthoxy 2-phényl indolizin-3-yl) carbonyl] anilino} acétique

Obtenu selon le même mode opératoire que le composé de l'exemple 196 par saponification du 2-{2-méthoxy 4-[(1-méthoxy 2-phényl indolizin-3-yl) carbonyl] anilino} acétate d'éthyle, composé de l'exemple 197 avec la soude 1N. On obtient une poudre jaune.
Rendement : 80%
Point de fusion : 206°C

### Exemple 199

### Chlorhydrate de 3-(dibutylamino) N-{2-méthoxy 4-[(1-méthoxy 2-methyl indolizin-3-yl) carbonyl] phényl} propanamide

A 2.5g (8.06 m.moles) de (4-amino 3-méthoxyphényl) (1-méthoxy 2-méthyl indolizin-3-yl) méthanone, composé de l'exemple 94, dans 20ml de dichlorométhane refroidi à 5°C, on ajoute 2.5ml (17.7m.moles) de triéthylamine puis 846µl (8.86m.moles) de chlorure de 3-chloropropionyle en solution dans 10ml de dichlorométhane et on agite pendant 3 heures à température ambiante. Le milieu réactionnel est lavé à l'eau puis avec une solution aqueuse saturée de chlorure de sodium, séché sur sulfate de sodium et concentré sous pression réduite.

Le résidu obtenu est dissout dans 40ml d'éthanol et on ajoute 1.7g (13.2m.moles) de dibutylamine puis on chauffe à reflux pendant 7 heures. Le milieu réactionnel est concentré sous pression réduite. Le produit est purifié par chromatographie sur colonne de silice en éluant avec un mélange de dichlorométhane / méthanol (98 : 2). On obtient 2.6g de produit qui est salifié par ajout d'acide chlorhydrique 1N dans l'éther éthylique. On obtient une poudre jaune (chlorhydrate, 0.25H₂O).
Rendement : 65%
Point de fusion : 82°C

### Exemple 200

### Chlorhydrate de 3-(dibutylamino) N-{2-méthoxy 4-[(1-méthoxy 2-phényl indolizin-3-yl) carbonyl] phényl} propanamide

Ce composé a été obtenu selon le même mode opératoire que celui décrit dans l'exemple 199 par acylation de la (4-amino 3-méthoxyphényl) (1-méthoxy 2-phényl indolizin-3-yl) méthanone avec le chlorure de 3-chloropropionyle, suivi d'une amination avec la dibutylamine. On obtient une poudre jaune (Chlorhydrate, hémihydrate).
Rendement : 52%
Point de fusion : 190°C

### Exemple 201

### N-{2-méthoxy 4-[(1-méthoxy 2-méthyl indolizin-3-yl) carbonyl] phényl} acétamide

Ce composé a été obtenu selon le même mode opératoire que celui décrit dans l'exemple 199 par acylation de la (4-amino 3-méthoxyphényl) (1-méthoxy 2-méthyl indolizin-3-yl) méthanone composé de l'exemple 94 avec le chlorure d'acétyle. Le produit est purifié par chromatographie flash sur silice en éluant avec un mélange de dichlorométhane / méthanol (99 : 1). On obtient une poudre jaune (0.3 H2O)
Rendement : 73%
Point de fusion 180°C

### Exemple 202

### 2-méthoxy 4-[(1-méthoxy 2-méthyl indolizin-3-yl) carbonyl] phénylcarbamate d'éthyle

Ce composé a été obtenu selon le même mode opératoire que celui décrit dans l'exemple 199 par acylation de la (4-amino 3-méthoxyphényl) (1-méthoxy2-méthyl indolizin-3-yl) méthanone avec le chloroformate d'éthyle. Le produit est purifié par chromatographie flash sur silice en éluant avec du dichlorométhane. On obtient une poudre jaune.
Rendement : 41%
Point de fusion : 140°C

### Exemple 203

### Chlorhydrate de 2-{[3-(dibutylamino) propanoyl] 2-méthoxy 4-[(1-méthoxy 2-méthyl indolizin-3-yl) carbonyl] anilino} acétate d'éthyle

A 89.1mg (2.23 m.moles) d'hydrure de sodium, à 60% en dispersion dans l'huile, dans 10ml de diméthylformamide on ajoute goutte à goutte 1g (2.03 m.moles) de 3-(dibutylamino) *N*-{2-méthoxy 4-[(1-méthoxy 2-methyl indolizin-3-yl) carbonyl] phényl} propanamide, composé de l'exemple 199, en solution dans 10ml de diméthylformamide puis agite à température ambiante 1 heure. On ajoute ensuite 247µl (2.23 m.moles) de bromoacétate d'éthyle et on agite à température ambiante pendant une nuit.

Le milieu réactionnel est versé sur de l'eau et de l'acétate d'éthyle. La phase organique est décantée, lavée à l'eau, séchée sur sulfate de sodium et concentrée sous pression réduite. Le produit est purifié par chromatographie flash sur colonne de silice en éluant avec un mélange de dichlorométhane / méthanol (97:3). On obtient 850mg d'une huile qui est dissoute dans l'éther éthylique et salifiée par ajout d'un équivalent d'acide chlorhydrique 1N dans l'éther éthylique. On obtient des cristaux jaunes (chlorhydrate, hydrate).
Rendement : 72%
Point de fusion : 67°C

### Exemple 204

### Chlorhydrate d'acide 2-{[3-(dibutylamino) propanoyl] 2-méthoxy 4-[(1-méthoxy 2-méthyl indolizin-3-yl) carbonyl] anilino}acétique

A 340mg (0.586 m.moles) de 2-{[3-(dibutylamino) propanoyl] 2-méthoxy 4-[(1-méthoxy 2-méthyl indolizin-3-yl) carbonyl] anilino} acétate d'éthyle obtenu à l'exemple 203, en solution dans 5ml d'éthanol, on ajoute 586µl de soude 1N et on agite à température ambiante une nuit. Le milieu réactionnel est concentré sous pression réduite. Le résidu est repris à l'eau, lavé à l'éther éthylique et décanté. La phase aqueuse est neutralisée avec de l'acide chlorhydrique 1N, et extrait au dichlorométhane. La phase organique est séchée sur sulfate de sodium et concentrée sous pression réduite.

Le produit est purifié par chromatographie flash sur colonne de silice en éluant avec un mélange de dichlorométhane / méthanol (8 : 2). On obtient 230mg d'une huile qui est dissoute dans l'acétate d'éthyle et salifiée par ajout d'un équivalent d'acide chlorhydrique 1N dans l'éther éthylique. On obtient une poudre jaune (Chlorhydrate, 0.6H₂O)
Rendement : 71%
Point de fusion: 151°C

### Exemple 205

### Chlorhydrate de 2-{[3-(dibutylamino) propanoyl] 2-méthoxy 4-[(1-méthoxy 2-phényl-3-indolizinyl) carbonyl] anilino} acétate d'éthyle

Obtenu selon le procédé décrit dans l'exemple 203 par alkylation de 3-(dibutylamino) *N*-{2-méthoxy 4-[(1-méthoxy 2-phényl indolizin-3-yl) carbonyl] phényl} propanamide composé de l'exemple 200, avec le bromoacétate d'éthyle. On obtient après salification avec de l'acide chlorhydrique 1N dans l'éther éthylique une poudre jaune (chlorhydrate).
Rendement : 55%
Point de fusion : 64°C

### Exemple 206

### Chlorhydrate de l'acide 2-{[3-(dibutylamino) propanoyl] 2-méthoxy 4-[(1 méthoxy 2-phényl indolizin-3-yl) carbonyl] anilino} acétique

Obtenu selon le même mode opératoire que celui de l'exemple 204 par saponification de 2-{[3-(dibutylamino) propanoyl] 2-méthoxy 4-[(1-méthoxy-2-phényl indolizin-3-yl)carbonyl] anilino} acétate d'éthyle, composé de l'exemple 205, avec de la soude 1N. On obtient après salification avec de l'acide chlorhydrique 1N dans l'éther éthylique une poudre jaune.
Rendement : 75%
Point de fusion : 113°C

### Exemple 207

### Chlorhydrate de 2-(dibutylamino) N-{2-méthoxy 4-[(1-méthoxy 2-méthyl indolizin-3-yl) carbonyl] phényl} 1-éthanesulfonamide

A 1g (3.22 m.moles) de (4-amino 3-méthoxyphényl) (1-méthoxy-2-méthyl indolizin-3-yl) méthanone dans 15ml de dichlorométhane, on ajoute 471.5µl (3.38m.moles) de triéthylamine puis 346.9µl (3.22m.moles) de chlorure de 2-chloroéthylsulfonyle en solution dans 5ml de dichlorométhane et on agite à température ambiante pendant une nuit. Le milieu réactionnel est lavé à l'eau puis avec une solution aqueuse saturée de chlorure de sodium, séché sur sulfate de sodium et concentré sous pression réduite.

Le résidu obtenu est dissout dans 10ml d'éthanol. On ajoute 375mg (2.9m.moles) de dibutylamine et on chauffe à reflux pendant 4 heures. Le milieu réactionnel est concentré sous pression réduite. Le produit est purifié par chromatographie sur colonne de silice en éluant avec un mélange de dichlorométhane / méthanol (98 : 2). On obtient 1.18g de produit qui est salifié par ajout d'acide chlorhydrique 1N dans l'éther éthylique. On obtient une poudre jaune (chlorhydrate, hémihydrate).
Rendement : 69%
Point de fusion: 91°C

### Exemple 208

### Chlorhydrate 2-(dibutylamino) N-{2-méthoxy 4-[(1-méthoxy 2-phényl indolizin-3-yl) carbonyl] phényl} 1-éthanesulfonamide

Ce composé est obtenu selon le même mode opératoire que le composé de l'exemple 207 par sulfonylation de la (4-amino 3-méthoxyphényl) (1-méthoxy 2-phényl indolizin-3-yl) méthanone avec le chlorure de 2-chloroéthylsulfonyle, suivi d'une amination avec la dibutylamine. On obtient une poudre jaune (chlorhydrate, hémihydrate).
Rendement : 63%
Point de fusion: 111°C

### Exemple 209

### N-{2-méthoxy 4-[(1-méthoxy 2-méthyl indolizin-3-yl) carbonyl] phényl} méthanesulfonamide

Obtenu selon le même mode opératoire que le composé de l'exemple 207 par sulfonylation de la (4-amino 3-méthoxyphényl) (1-méthoxy 2-méthyl indolizin-3-yl) méthanone avec le chlorure de méthanesulfonyle. Le produit est purifié par chromatographie sur colonne de silice en éluant avec un mélange de toluène / acétate d'éthyle (7 : 3). On obtient une poudre jaune.
Rendement : 67%
Point de fusion : 165°C

### Exemple 210

### 3-{3-méthoxy 4-[(méthylsulfonyl) amino] benzoyl} 2-méthyl indolizin-1-yl carboxylate d'éthyle

Ce composé a été obtenu selon le même mode opératoire que celui décrit dans l'exemple 207 par sulfonylation de la 3-(4-amino 3-méthoxybenzoyl) 2-méthyl indolizin-1-yl carboxylate d'éthyle composé de l'exemple 97, avec le chlorure de méthanesulfonyle. Le produit est purifié par chromatographie sur colonne de silice en éluant avec un mélange de toluène / acétate d'éthyle (8 : 2). On obtient une poudre jaune.
Rendement : 57%
Point de fusion: 178°C

### Exemple 211

### Sel de sodium de l'acide 3-{3-méthoxy 4-[(méthylsulfonyl) amino] benzoyl} 2-méthyl indolizin-1-yl carboxylique

A 290mg (0.675m.mole) de 3-{3-méthoxy 4-[(méthylsulfonyl) amino] benzoyl} 2-méthyl indoliz-1-yl carboxylate d'éthyle dans 7ml de dioxanne plus 7ml d'eau, on ajoute 1ml de lessive de soude et on chauffe à reflux pendant 6 heures.

On refroidit, on verse sur de l'eau et on neutralise avec une solution aqueuse d'hydrogénosulfate de potassium puis on extrait à l'acétate d'éthyle. La phase organique est lavée à l'eau, séchée sur sulfate de sodium et concentrée sous pression réduite.

On obtient 220mg d'une poudre jaune. Le produit est salifié par ajout d'un équivalent de soude 1N à une suspension du produit dans l'eau et agitation à température ambiante jusqu'à dissolution.

La solution obtenue est ensuite lyophilisée. On recueille un lyophilisat jaune (sel de Na, 1.85 H₂O)
Rendement : 81%
Spectrométrie de masse (Mode ES+) MH⁺ = 403.2

### Exemple 212

### Chlorhydrate de l'acide 2-{{[2-(dibutylamino) éthyl] sulfonyl} 2-méthoxy 4-[(1-méthoxy 2-méthyl indolizin-3-yl) carbonyl] anilino} acétique

### ETAPE A

### 2-{{[2-(dibutylamino) éthyl] sulfonyl} 2-méthoxy 4-[(1-méthoxy 2-méthyl indolizin-3-yl) carbonyl] anilino} acétate de benzyle

A 400mg (0.755m.mole) de 2-(dibutylamino) N-{2-méthoxy 4-[(1-méthoxy 2-méthyl indolizin-3-yl) carbonyl] phényl} 1-éthanesulfonamide, composé de l'exemple 207, en solution dans 10ml de diméthylformamide, on ajoute 125mg (0.906m.mole) de carbonate de potassium puis 142µl (0.906m.mole) de bromoacétate de benzyle et on chauffe à 60°C pendant 1 heure. Le milieu réactionnel est versé sur de l'eau et de l'acétate d'éthyle.

La phase organique est décantée, lavée à l'eau, séchée sur sulfate de sodium et concentrée sous pression réduite.

Le produit est purifié par chromatographie flash sur colonne de silice en éluant avec un mélange de dichlorométhane / méthanol (98 : 2). On obtient 440mg d'une huile engagée directement dans l'étape suivante.
Rendement : 86%

### ETAPE B

A 430mg (0.634m.mole) de 2-{{[2-(dibutylamino) éthyl] sulfonyl} 2-méthoxy 4-[(1-méthoxy 2-méthyl indolizin-3-yl) carbonyl] anilino} acétate de benzyle dans 5ml d'éthanol, en présence de 100mg de Pd/C à 10%, on ajoute 1.3ml (12.7m.mole) de cyclohexène et on porte au reflux pendant 3 heures. Le milieu réactionnel est refroidi. On filtre le catalyseur et on concentre sous pression réduite le filtrat.

Le produit est purifié par chromatographie flash sur colonne de silice en éluant avec un mélange de dichlorométhane / méthanol (9 : 1). On obtient 250mg d'une huile qui est dissoute dans l'acétate d'éthyle et salifiée par ajout d'un équivalent d'acide chlorhydrique 1N dans l'éther éthylique. On obtient une poudre orange(chlorhydrate, dihydrate)
Rendement : 67%
Point de fusion : 85°C

### Exemple 213

### Chlorhydrate de 2-{{[2-(dibutylamino) éthyl] sulfonyl} 2-méthoxy 4-[(1-méthoxy 2-phényl indolizin-3-yl) carbonyl] anilino} acétate de benzyle

Ce composé a été obtenu selon le même mode opératoire que l'exemple 212 étape A par alkylation de 2-(dibutylamino) N-{2-méthoxy 4-[(1-méthoxy 2-méthyl indolizin-3-yl) carbonyl] phényl}-1-éthanesulfonamide, avec le bromoacétate de benzyle.

On obtient après salification avec de l'acide chlorhydrique 1N dans l'éther éthylique une poudre jaune (chlorhydrate, hémihydrate).
Rendement : 55%
Point de fusion : 95°C

### Exemple 214

### Chlorhydrate de l'acide 2-{{[2-(dibutylamino) éthyl] sulfonyl} 2-méthoxy 4-[(1-méthoxy 2-phényl indolizin-3-yl) carbonyl] anilino} acétique

Ce composé a été obtenu selon le même mode opératoire que l'exemple 212 étape B par hydrogénation de 2-{{[2-(dibutylamino) éthyl] sulfonyl} 2-méthoxy 4-[(1-méthoxy 2-phényl indolizin- 3-yl) carbonyl] anilino}acétate de benzyle composé de l'exemple 213, avec du cyclohexène en présence de Pd/C dans l'éthanol. On obtient après salification avec de l'acide chlorhydrique 1N dans l'éther éthylique une poudre jaune (chlorhydrate, 1.5H₂O)
Rendement : 75%
Point de fusion: 113°C

### Exemple 215

### Etude de la liaison ¹²⁵I - b-FGF au Récepteur purifié FGF R α IIIc par méthode de scintillation de proximité

Des plaques NBS (NBS plate 96 well solid white CORNING 3600) sont coatées avec 100 µl de gélatine 0.1 % par puit, 2 heures à 37°C. Au terme de l'incubation, on élimine le coating, on rinçe et on sèche bien les plaques. On distribue 100 µl de tampon de binding (Tampon Bis Tris 40 mM pH 7.0) dans les plaques.

Les dilutions des composés de l'invention sont réparties dans les puits à raison de 10 µl / puit. On distribue ensuite 10 µl / puit de b-FGF (AMERSHAM ARM 35050) et 10 µl / puit de FGF R α III c (R&D Systems 658 FR). On ajoute après 10 µl / puit de ¹²⁵I - bFGF (Dupont NEN NEX 268 - Activité spécifique > 70 µCi) et 50 µl / puit des billes SPA (AMERSHAM RPQN 00019). On agite quelques secondes la plaque et on l'incube 60 minutes à 37°C et à l'abri de la lumière.

Au terme de l'incubation, la plaque est lue dans un compteur de radioactivité MIBROBETA TRILUX (: WALLAC - PERKINELMER)

Les composés de l'invention ont démontré une activité spécifique comprise entre 10⁻⁶M et 10⁻⁹M.

### Exemple 216

### Effets des composés de la formule I sur la prolifération d'HUVECs versus 30 ng/ml de b-FGF ou 10 ng/ml de a-FGF

Coater les plaques 24 puits (FALCON PRIMARIA) avec 200 µl d'une solution de fibronectine (50 µg / ml préparée dans du PBS) / puits.

Ensemencer à raison de 30000 cellules / ml / puit dans un milieu RPMI 1640 +10 % SVF + 1 % Glutamine + mélange Héparine-ECGF (HE).

Incuber à 37 °C, 5 % CO2 le temps que les cellules adhèrent.

Dissoudre les produits et préparer des solutions dans le DMSO / milieu réactionnel ayant une concentration finale de 1 µM final à 10⁻⁷M.

Après adhésion des cellules pendant 6 heures à 37 °C en présence de 5% CO₂, le milieu est remplacé par du RPMI 1640 0.1 % SVF + Glutamine +HE.

Pour la derivatisation, on utilise comme contrôle négatif 0.1 % SVF, comme contrôle positif 0 % SVF et comme témoin 0.1 % SVF + 30 ng/ml de b-FGF ou 10 ng/ml de a-FGF. On incube ensuite 24 heures à 37 °C en présence de 5 % CO₂.

Le deuxième jour les cellules sont rincées par 1 ml de PBS et 200µl de trypsine, puis elles sont récupérées dans l'isoton. On procède à la numération (n> 9µm)

Dans ce test de prolifération des cellules endothéliales induite par le b-FGF ou le a-FGF les composés de l'invention ont démontré une activité spécifique comprise entre 10⁻⁵M et 10⁻⁹M.

### Exemple 217

### Modèle d'angiogenèse in-vitro

Préparer les gels en distribuant dans chaque puits de chamberslide (Biocoat Cellware rat tail collagen, Type I, 8-well culturesides: Becton Dickinson 354630) 160 µl de matrigel dilué au 1/6 (Growth factor reduced Matrigel: Becton Dickinson 356230) dans du collagen (Rat Tail Collagène, type I: Becton Dickinson 354236). Laisser gélifier pendant 1 heure à 37°C.

Ensemencer les cellules endothéliales veineuses humaines (HUVEC ref:C-015-10C - cascade Biologics, INC) ou artérielles de porc (PAEC) à 15.10³ cellules/puits dans 400 µl de milieu EBM (Clonetics C3121) + 2% FBS + hEGF 10 µg/ml pour les HUVEC et DMEM + 3% SVF + Glutamine 2 mM + Sodium pyruvate 1 mM + Acides Aminés Non Essentiels 1% (GIBCO) pour les PAEC.

Stimuler avec le b-FGF (TEBU/Peprotech) 10 ng/ml ou le a-FGF (TEBU/Peprotech) 10 ng/ml en présence ou non des produits de l'invention pendant 24h à 37°C en présence de 5% CO₂.

Après 24 heures, fixer les cellules et colorer la lame au trichrome de Masson avant observation au microscope objectif X4 et analyse d'image (BIOCOM- logiciel Visiolab 2000).

Pour le test d'angiogénèse in vitro induite par le b-FGF ou le a-FGF, les composés de l'invention ont démontré une activité spécifique comprise entre 10⁻⁷M et 10⁻¹¹M.

### Exemple 218

### Modèle d'angiogenèse inflammatoire chez la souris

L'angiogenèse est requise pour le développement des maladies inflammatoires chroniques comme l'arthrite rhumatoïde, les IBD, mais aussi pour le développement des tumeurs solides. La formation de nouveaux vaisseaux permet non seulement la perfusion des tissus pathologiques mais également le transport de cytokines responsables de l'établissement de la chronicité de la maladie.

Le modèle décrit par Colville-Nash P et al (*D. JPET.,* 1995, vol 274 n°3, pp1463-1472) permet d'étudier des agents pharmacologiques susceptibles de moduler l'apparition de l'angiogenèse.

Les animaux, souris blanches non consanguines de 25g environ sont anesthésiés avec du pentobarbital sodique (60 mg/kg ; Sanofi Nutrition Santé animale) par voie intrapéritonéale.

Une poche d'air est créée sur le dos de la souris par injection de 3 ml d'air en sous-cutanée.

Après le réveil, les animaux reçoivent un traitement en général par gavage et reçoivent une injection de 0.5 ml d'adjuvant de Freund (Sigma) avec 0.1 % d'huile de croton (Sigma) dans la poche.

Sept jours après, les souris sont à nouveau anesthésiées et placées sur une plaque chauffante à 40°C. Un ml de rouge carmin (5% dans 10 % de gélatine - Aldrich Chemicals) est injecté à la veine de la queue. Les animaux sont ensuite mis à 4°C pendant 2-3 heures.

Les peaux sont ensuite prélevées et mises à sécher pendant 48 heures dans une étuve à 56°C. Les tissus secs sont pesés et mis dans 1.8 ml de tampon digestif (dithiothreitol 2 mM, Na₂HPO₄ 20mM, EDTA 1 mM, papaïne 12U/ml) pendant 24 heures.

Le colorant est alors dissout dans 0.2 ml de NaOH 5M. Les peaux sont centrifugées à 2000g pendant 10 mn. Les surnageants sont filtrés sur membranes d'acétate de cellulose 0.2 µm. Les filtrats sont lus dans un spectrophotomètre à 492 nm. contre une gamme étalon de rouge carmin.

Deux paramètres sont étudiés : le poids sec du granulome et la quantité de colorant après digestion des tissus.

Les résultats sont exprimés en valeurs moyennes (± sem). Les différences entre les groupes sont testées avec une ANOVA suivie d'un test de Dunnet dont le groupe de référence est le groupe "témoin solvant".

Les composés de l'invention sont actifs par voie orale à des doses de 0.1 à 100mg/kg

### Exemple 219

### Modèle d'angiogenèse MATRIGEL chez la souris

Le modèle décrit par Passaniti and al. (*Laboratory Investigation* (1992) 67 (4) pp519-524) permet d'étudier des agents pharmacologiques susceptibles de moduler l'apparition de l'angiogenèse spécifiquement induite par le bFGF. A du Matrigel (Beckton Dickinson) maintenu sous forme liquide à 4°C est ajouté du FGF2 (Peprotecch) à raison de 300 ng/ml. Après homogénéisation, le mélange (0.5ml)est injecté par voie sous-cutanée dans le bas du dos de souris noires femelles(C57/B16) de 20g environ préalablement anesthésiées avec du pentobarbital sodique (60 mg/kg ; Sanofi Nutrition Santé animale) par voie intrapéritonéale. Les animaux sont traités par gavage. Après 5 jours les souris sont à nouveau anesthésiées et la peau du bas du dos est prélevée, à ce stade, les différences qualitatives de vascularisation du granulome sont évaluées (scorées) et les granulomes sont photographiés. Un dosage de DNA dans les granulomes est ensuite effectué pour quantifier la cellularité de celui-ci. Pour cela, les granulomes isolés sont digérés par de la collagénase (3mg/ml) toute une nuit à 37°C. Après centrifugation à 850 g durant 10 min, le surnageant est écarté et le culot est remis en solution dans 1.2 ml de tampon PBS contenant 1 mM CaCl₂, 1 mM MgCl₂ et 5 mM de glucose. La quantité de DNA présente est mesurée à l'aide d'un kit (Cyquant-GR®, Molecular probe) suivant les instructions du fournisseur.
Les résultats sont exprimés en valeurs moyennes (± sem). Les différences entre les groupes sont testées avec une ANOVA suivie d'un test de Dunnet dont le groupe de référence est le groupe "témoin solvant".

Pour les études histologiques, les granulomes sont prélevés avec le muscle et la peau, fixés une nuit dans une solution de formaldéhyde à 10% et inclus dans de la paraffine (Embedder Leica®). Les granulomes sont ensuite coupés à l'aide d'un microtome (Leica) et colorés avec le colorant Trichrome de Mason. La néo vascularisation des granulomes est alors évaluée. Les niveaux de vascularisation sont compris entre une valeur 0 et 5.

Les composés de l'invention sont actifs par voie orale à des doses de 0.1 à 100mg/kg

### Exemple 220

### Modèle d'angiogenèse tumorale chez la souris

Ce modèle permet d'étudier des agents pharmacologiques susceptibles de moduler l'apparition de l'angiogenèse spécifiquement induite par le développement tumoral. Des souris C56/B16 de 20g environ sont anesthésiées avec du pentobarbital sodique (60 mg/kg ; Sanofi Nutrition Santé animale) par voie intrapéritonéale. Les tumeurs sont établies par injection sous cutanée sur le dos de cellules Lewis Lung de souris à raison de 2 10⁵ cellules/souris. Après 5 jours, les souris sont traitées tous les jours par gavage. La taille des tumeurs est mesurée 2 fois par semaine durant 21 jours et le volume tumoral est calculé en utilisant la formule : [π/6(ω₁ x ω₂ x ω₂)], où ω₁ représente le plus grand diamètre et ω₂ représente le plus petit diamètre.

Les résultats sont exprimés en valeurs moyennes (± sem). Les différences entre les groupes sont testées avec une ANOVA suivie d'un test de Dunnet dont le groupe de référence est le groupe "témoin solvant".

Les composés de l'invention sont actifs par voie orale à des doses de 0.1 à 100mg/kg

## Revendications

1. Composés de formule I, 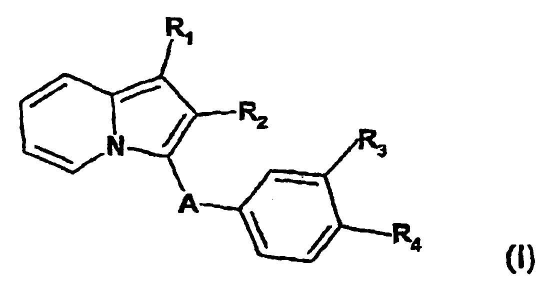 dans laquelle
- **R**_{**1**} représente un radical hydroxy, un radical alcoxy linéaire ou ramifié de 1 à 5 atomes de carbone, un radical carboxy, un radical alcoxycarbonyle de 2 à 6 atomes de carbone ou un radical de formule :
• -NR₅R₆
• -NH-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₈
• -O-Alk-OH
• -O-Alk-C(NH₂):NOH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
dans lesquelles
• Alk représente un radical alkyle ou un radical alkylène linéaire ou ramifié de 1 à 5 atomes de carbone
• cAlk représente un radical cycloalkyle de 3 à 6 atomes de carbone,
• n représente un nombre entier de 0 à 5,
• m représente un nombre entier de 1 à 5,
• R₅ et R₆ identiques ou différents représentent chacun un atome d'hydrogène, un radical alkyle linéaire ou ramifié de 1 à 5 atomes de carbone ou un radical benzyle,
• R₇ représente un atome d'hydrogène ou un radical alkyle de 1 à 5 atomes de carbone,
• R₈ représente un radical alkyle de 1 à 5 atomes de carbone ou un radical -CO-Alk,
• Ph représente un radical phényle éventuellement substitué par un ou plusieurs atomes d'halogène, par un ou plusieurs radicaux alcoxy de 1 à 5 atomes de carbone, un ou plusieurs radicaux carboxy ou par un ou plusieurs radicaux alcoxycarbonyle de 2 à 6 atomes de carbone,
- **R**_{**2**} représente un atome d'hydrogène, un radical alkyle de 1 à 5 atomes de carbone, un radical d'halogenure d'alkyle de 1 à 5 atomes de carbone comportant 3 à 5 atomes d'halogène, un radical cycloalkyle de 3 à 6 atomes de carbone ou un radical phényle éventuellement substitué par un ou plusieurs atomes d'halogène, par un ou plusieurs radicaux alcoxy de 1 à 5 atomes de carbone, un ou plusieurs radicaux carboxy ou par un ou plusieurs radicaux alcoxycarbonyle de 2 à 6 atomes de carbone,
- **A** représente un radical -CO-, -SO- ou -SO₂-,
- **R**_{**3**} et **R**_{**4**} identiques ou différents représentent chacun un atome d'hydrogène, un radical alcoxy de 1 à 5 atomes de carbone, un radical amino, un radical carboxy, un radical alcoxycarbonyle de 2 à 6 atomes de carbone, un radical hydroxy, un radical nitro, un radical hydroxyamino, un radical de formule
• -Alk-COOR₇
• -NR₅R₆
• -NH-Alk-COOR₇
• -NH-COO-Alk
• -N(R₁₁)-SO₂-Alk-NR₉R₁₀
• -N(R₁₁)-SO₂-Alk
• -N(R₁₁)-Alk-NR₅R₆
• -N(R₁₁)-CO-AlK-NR₉R₁₀
• -N(R₁₁)-CO-Alk
• -N(R₁₁)-CO-CF₃
• -NH-Alk-HetN
• -O-Alk-NR₉R₁₀
• -O-Alk-CO-NR₅R₆
• -O-Alk-HetN
dans lesquels n, m, Alk, R₅, R₆, et R₇, ont la signification donnée précédemment pour R₁ et
• R₉ et R₁₀ identiques ou différents représentent chacun un atome d'hydrogène ou un radical alkyle de 1 à 5 atomes de carbone,
• R₁₁ représente un atome d'hydrogène ou un radical -Alk-COOR₁₂ où R₁₂ représente un atome d'hydrogène, un radical alkyle de 1 à 5 atomes de carbone ou un radical benzyle,
• HetN représente un hétérocycle à 5 ou 6 chaînons comportant au moins un atome d'azote et éventuellement un autre hétéroatome choisi parmi l'azote et l'oxygène
ou R₃ et R₄ forment ensemble un hétérocycle insaturé de 5 à 6 chaînons, à conditions toutefois que lorsque R₃ représente un radical alcoxy et R₄ représente un radical -O-Alk-NR₉R₁₀ ou un radical hydroxy, R₁ ne représente pas un atome d'hydrogène ou un radical alcoxy,
éventuellement sous la forme de l'un de leurs sels pharmaceutiquement acceptables.

2. Composés de formule I, selon la revendication 1, dans laquelle
- **R**_{**1**} représente un radical hydroxy, un radical alcoxy linéaire ou ramifié de 1 à 5 atomes de carbone, un radical carboxy, un radical alcoxycarbonyle de 2 à 6 atomes de carbone ou un radical de formule:
• -NR₅R₆
• -NH-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₈
• -O-Alk-OH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
dans lesquelles
• Alk représente un radical alkyle ou un radical alkylène linéaire ou ramifié de 1 à 5 atomes de carbone
• cAlk représente un radical cycloalkyle de 3 à 6 atomes de carbone,
• n représente un nombre entier de 0 à 5,
• m représente un nombre entier de 1 à 5,
• R₅ et R₆ identiques ou différents représentent chacun un atome d'hydrogène, un radical alkyle linéaire ou ramifié de 1 à 5 atomes de carbone ou un radical benzyle,
• R₇ représente un atome d'hydrogène ou un radical alkyle de 1 à 5 atomes de carbone,
• R₈ représente un radical alkyle de 1 à 5 atomes de carbone ou un radical-CO-Alk,
• Ph représente un radical phényle éventuellement substitué par un ou plusieurs atomes d'halogène, par un ou plusieurs radicaux alcoxy de 1 à 5 atomes de carbone, par un ou plusieurs radicaux carboxy ou par un ou plusieurs radicaux alcoxycarbonyle de 2 à 6 atomes de carbone,
- **R**_{**2**} représente un radical alkyle de 1 à 5 atomes de carbone, un radical trifluorométhyle, un radical cycloalkyle de 3 à 6 atomes de carbone ou un radical phényle éventuellement substitué par un ou plusieurs atomes d'halogène, par un on plusieurs radicaux alcoxy de 1 à 5 atomes de carbone, par un ou plusieurs radicaux carboxy ou par un ou plusieurs radicaux alcoxycarbonyle de 2 à 6 atomes de carbone
- **A** représente un radical -CO-, -SO₂-,
- **R**_{**3**} et **R**_{**4**} identiques ou différents représentent chacun un atome d'hydrogène, un radical alcoxy de 1 à 5 atomes de carbone, un radical amino, un radical carboxy, un radical alcoxycarbonyle de 2 à 6 atomes de carbone, un radical nitro, un radical hydroxyamino, un radical de formule
• -Alk-COOR₇
• -NR₅R₆
• -NH-Alk-COOR₇
• -NH-COO-Alk
• -N(R₁₁)-SO₂-Alk-NR₉R₁₀
• -N(R₁₁)-SO₂-Alk
• -N(R₁₁)-Alk-NR₅R₆
• -N(R₁₁)-CO-Alk-NR₉R₁₀
• -N(R₁₁)-CO-Alk
• -N(R₁₁)-CO-CF₃
• -NH-Alk-HetN
dans lesquels n, m, Alk, R₅, R₆, et R₇, ont la signification donnée précédemment pour R₁ et
• R₉ et R₁₀ identiques ou différents représentent chacun un atome d'hydrogène ou un radical alkyle de 1 à 5 atomes de carbone,
• R₁₁ représente un atome d'hydrogène ou un radical -Alk-COOR₁₂ où R₁₂ représente un atome d'hydrogène, un radical alkyle de 1 à 5 atomes de carbone ou un radical benzyle,
• HetN représente un hétérocycle à 5 ou 6 chaînons comportant au moins un atome d'azote et éventuellement un autre hétéroatome choisi parmi l'azote et l'oxygène
éventuellement sous la forme de l'un de leurs sels pharmaceutiquement acceptables.

3. Composés de formule I, selon l'une quelconque des revendications 1 ou 2, dans laquelle
- **R**_{**1**} représente, un radical alcoxy de 1 à 5 atomes de carbone, un radical carboxy, un radical -O-Alk-COOH dans laquelle Alk représente un radical alkylène de 1 à 5 atomes de carbone, un radical de formule -O-Alk-Ph dans laquelle Alk représente un radical allcylène de 1 à 5 atomes de carbone et Ph représente un radical phényle éventuellement substitué par un ou plusieurs atomes d'halogène ou par un ou plusieurs radicaux alcoxy de 1 à 5 atomes de carbone ou par un ou plusieurs radicaux carboxy, un radical de formule -NH-CO-Ph, un radical de formule -NH-SO₂-Ph ou radical de formule -NH-CO-NH-Ph,
- **R**_{**2**} représente un radical alkyle de 1 à 5 atomes de carbone,
- **A** représente un radical -CO-,
- **R**_{**3**} et **R**_{**4**} différents représentent chacun un atome d'hydrogène, un radical alcoxy de 1 à 5 atomes de carbone, un radical amino, un radical carboxy un radical alcoxycarbonyle de 2 à 6 atomes de carbone,
éventuellement sous la forme de l'un de ses sels pharmaceutiquement acceptables.

4. Composé de formule I, selon la revendication 1, choisi parmi les composés suivants :
- (4-amino 3-méthoxy phényl) (1-méthoxy 2-méthyl indolizin-3-yl) méthanone
- acide 3-(4-amino 3-méthaxy benzoyl) 2-méthyl indolizin-1-yl carboxylique
- acide 2-{[3-(4-amino 3-méthoxybenzoyl) 2-méthyl indolizin-1-yl] oxy} acétique
- (4-amino 3-méthoxy phényl) {1-[(4-chlorobenzyl) oxy] 2-méthyl-indolizin-3-yi} méthanone
- (4-amino 3-méthoxy phényl) {1-[(3-méthoxybenzyl) oxy] 2-méthyl-indolizin-3-yl} méthanone
- acide 4-({[3-(4-amino 3-méthoxy benzoyl) 2-méthyl indolizin-1-yl] oxy} méthyl) benzoique
- acide 3-(4-carboxybenzoyl) 2-méthyl indolizin-1-yl carboxylique
- 3-[(1-méthoxy-2-méthyl indolizin-3-yl carbonyl] benzoate de méthyle
- acide 4-[(1-méthoxy-2-méthyl indolizin-3-yl) carbonyl] benzoïque
- acide 2-amino-5-[(1-méthoxy-2-méthyl indolizin-3-yl) carbonyl] benzoïque
- acide 2-amino-5-({1-[(3-méthoxybenzoyl)amino]-2-méthyl indolizin-3-yl} carbonyl) benzoïque
- acide 2-amino-5-({2-méthyl-1-[(3,4,5-triméthoxybenzoyl)amino] indolizut-3-yl} carbonyl) benzoïque
- acide 2-amino-5-({1-{[(3-méthoxyphényl) sulfonyl] amino}-2-méthyl indolizin-3-yl} carbonyl) benzoïque
éventuellement sous la forme de l'un de ses sels pharmaceutiquement acceptables.

5. Procédé de préparation des composés de formule **I** selon les revendications 1 à 4 **caractérisé en ce que**
**A)** l'on condense un dérivé d'indolizine de formule II, 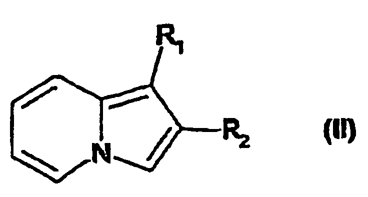 dans laquelle R₁ et R₂ ont la signification donnée pour la formule I mais R₂ ne représente pas un atome d'hydrogène ou un radical d'halogenure d'alkyle,
avec un dérivé de formule III, 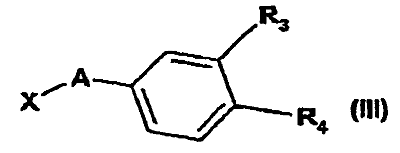 dans laquelle X représente un atome d'halogène et R₃ ou R₄ identiques ou différents représentent chacun un atome d'hydrogène, un radical nitro, un radical trifluoroacétamido, ou un radical alcoxycarbonyle de 2 à 6 atomes de carbone, pour obtenir les composés de formule Ia, Id ou Ik, 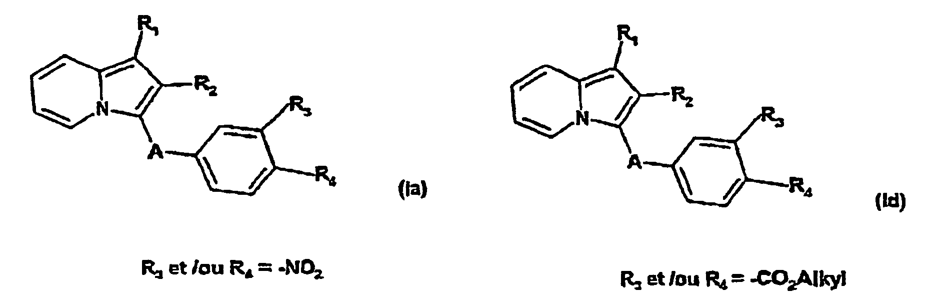 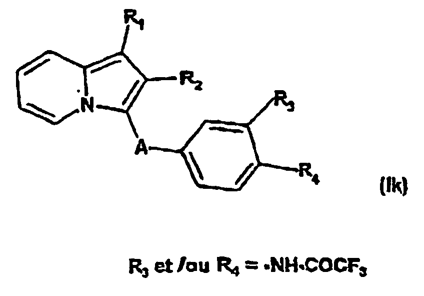 et ensuite,
**a)** on soumet les composés de formule Ia à une réduction pour obtenir les composés de formule Ib, 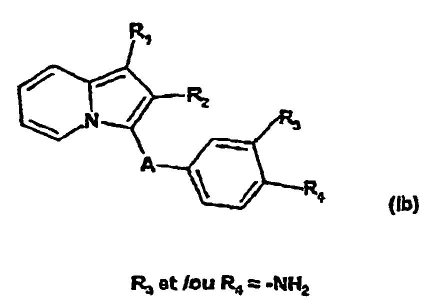 dans laquelle R₃ et / ou R₄ représentent un radical amino, lesquels ensuite composés de formule Ib
• sont soumis l'action d'un halogénure d'alkyle pour obtenir les composés de la formule I pour lesquels R₄ et / ou R₃ représentent un radical -NR₅R₆ (dans lequel R₅ représente un atome d'hydrogène et R₆ représente un radical alkyle de 1 à 5 atomes de carbone) un radical -NH-Alk-NR₅R₆ ou un radical -NH-Alk-COOR₇ (dans lequel R₇ ne représente pas un atome d'hydrogène) à partir duquel par une saponification ultérieure on obtient les composés de formule I pour lesquels R₄ et / ou R₃ représentent un radical -NH-Alk-COOR₇ dans laquelle R₇ représente un atome d'hydrogène,
**ou**
• sont soumis à une acylation pour obtenir les composés de formule I pour lesquels R₄ et / ou R₃ représentent un radical -NH-CO-Alk, ou un radical -NH-CO-Alk-NR₉R₁₀, lesquels ensuite sont soumis à une alkylation pour obtenir un radical -N(R₁₁)-CO-Alk ou un radical -N(R₁₁)-CO-Alk-NR₉R₁₀ où R₁₁ représente un radical -Alk-COOR₁₂ dans lequel R₁₂ ne représente pas un atome d'hydrogène, ces derniers composés sont ensuite éventuellement soumis à une saponification pour obtenir les composés de formule I pour lesquels R₄ et / ou R₃ représentent un radical -N(R₁₁)-CO-Alk ou un radical -N(R₁₁)-CO-Alk-NR₉R₁₀ où R₁₁ représente un radical -Alk-COOH,
**ou**
• sont soumis à une sulfonylation, pour obtenir les composés de formule I pour lesquels R₄ et / ou R₃ représentent un radical -NH-SO₂-Alk ou un radical -NH-SO₂-Alk-NR₉R₁₀, lesquels ensuite sont soumis à une alkylation pour obtenir un radical -N(R₁₁)-SO₂-Alk ou un radical -N(R₁₁)-SO₂-Alk-NR₉R₁₀ où R₁₁ représente un radical -Alk-COOR₁₂ dans lequel R₁₂ ne représente pas un atome d'hydrogène, ces derniers composés sont ensuite éventuellement soumis à une saponification pour obtenir les composés de formule I pour lesquels R₄ et / on R₃ représentent un radical -N(R₁₁)-SO₂-Alk ou un radical -N(R₁₁)- SO₂-Alk -NR₉R₁₀ où R₁₁ représente un radical -Alk-COOH
**b)** on soumet les composés de formule Id dans laquelle R₃ et / ou R₄ représentent un radical alcoxycarbonyle à une saponification pour obtenir les composés de formule I dans laquelle R₃ et / ou R₄ représente un radical carboxy, **ou**
**c)** on soumet lorsque R₁ représente un radical benzyloxy les composés de formule Ia à l'action de l'acide trifluoroacétique ou les composés de formule Id à une hydrogénation, pour obtenir les composés de formule If, 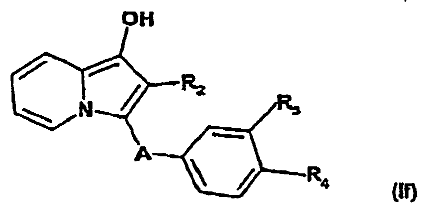 dans laquelle R₃ et / ou R₄ ont les significations données ci-dessus,
et ensuite que l'on soumet les composés de formule If à une O-alkylation pour obtenir les composés de formule Ig, 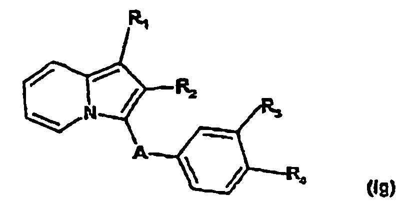 dans laquelle R₃ et / ou R₄ ont les significations données ci-dessus, et R₁ représente un radical alcoxy linéaire ou ramifié de 1 à 5 atomes de carbone, un radical -O-(CH₂)ₙ-cAlk, un radical -O-Alk-COOR₇, un radical -O-Alk-NR₅R₆ un radical -O-(CR₂)ₙ-Ph un radical -O-Alk-O-R₈, - qui lorsque R₈ représente un radical -COCH₃ peut donner par saponification ultérieure un radical -O-Alk-OH - ou un radical -O-Alk-CN qui par traitement avec de l'hydroxylamine conduit à un radical -O-Alk-C(NH₂)=NOH,
**ou**
**d)** on soumet lorsque R₁ représente un radical alcoxycarbonyle les composés de formule Ia à une saponification pour obtenir les composés de formule Ih, 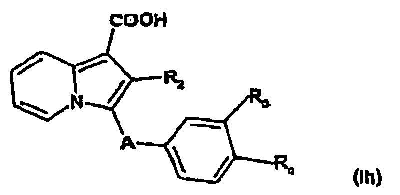 dans laquelle R₃ et / ou R₄ ont les significations données ci-dessus, lesquels ensuite sont soumis à l'action d'un dérivé d'amine pour obtenir les composés de formule I dans laquelle R₁ représente un radical -CO-NH-Alk ou à l'action d'un dérivé d'aminoacide pour obtenir les composés de formule I dans laquelle R₁ représente un radical -CO-NH-(CH₂)ₘ-COOR₇
**ou**
**e)** on soumet lorsque R₁ représente un radical -NH-CO₂tButyle les composés de formule Ia ou Id
• soit à une alkylation suivie d'une déprotection et d'une éventuelle deuxième alkylation pour obtenir les composés de formule Ii,
• soit à une déprotection suivie d'une acylation pour obtenir les composés de formule Ij dans laquelle R₅ représente un atome d'hydrogène, suivie d'une éventuelle alkylation pour obtenir les composés de formule Ij dans laquelle R₅ représente un radical alkyle 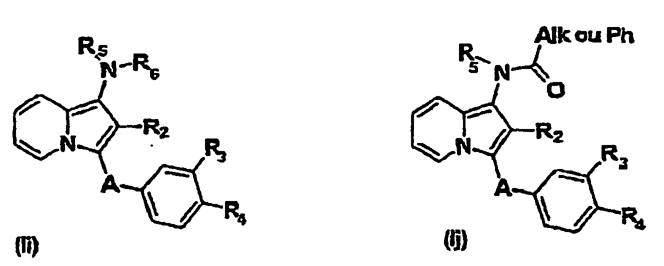
**ou**
**f)** on soumet lorsque R₁ représente un radical -NH-CO₂tButyle les composés de formule Ik
• soit à une déprotection suivie d'une acylation pour obtenir les composés de formule Il 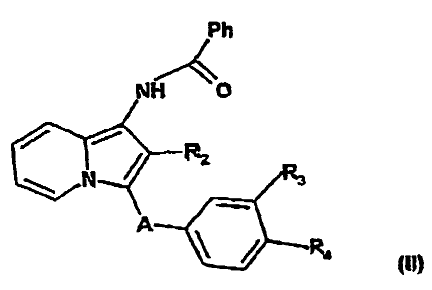
• soit à une déprotection suivie d'une sulfonylation pour obtenir les composés de formule Im 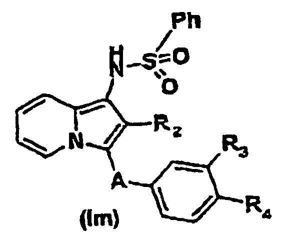
• soit à une déprotection suivie d'un traitement par un phénylisocyanate pour obtenir les composés de formule In 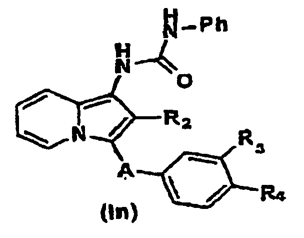
**OU**
**B)** lorsque R₁ représente un groupement électro-attracteur, R₂ représente un atome d'hydrogène ou un radical un radical d'halogenure d'alkyle et A représente un radical -CO-, on fait réagir la pyridine avec une bromoacétophénone de formule IV, 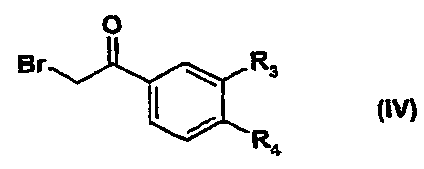 pour obtenir les composés de formule V, 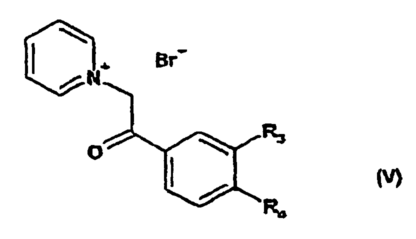 lesquels ensuite sont soumis à une cycloaddition 1,3-dipolaire avec l'acrylate d'éthyle ou un dérivé halogéné de crotonate d'éthyle en présence d'un oxydant pour obtenir les composés de formule la dans laquelle **R**_{**1**} représente un radical éthoxycarbonyle et **R**_{**2**} représente un atome d'hydrogène ou un radical un radical d'halogenure d'alkyle.

6. Composition pharmaceutique contenant en tant que principe actif un composé de formule I, selon l'une quelconque des revendications 1 à 4 éventuellement en association avec un ou plusieurs excipients inertes et appropriés,

7. Composition pharmaceutique selon la revendication 6 utile dans le traitement des maladies nécessitant une modulation des b-FGF's.

8. Composition pharmaceutique selon la revendication 6 utile dans le traitement des carcinomes ayant un degré de vascularisation important tels que le carcinomes de poumon, sein, prostate et oesophage, des cancers induisant des métastases tels que le cancer du colon et le cancer de l'estomac, des mélanomes, des gliomes, des lymphomes, et des leucémies.

9. Composition pharmaceutique selon la revendication 6 utile dans le traitement de maladies cardiovasculaires telles que l'athérosclérose, la resténose post angioplastie, des maladies liés aux complications apparaissant suite à la pose de prothèses endovasculaires et/ou de pontages aorto-coronariens ou d'autres greffes vasculaires de l'hypertropbie cardiaque, ou des complications vasculaires du diabète comme les rétinopathies diabétiques.

10. Composition pharmaceutique selon la revendication 6 utile dans le traitement de maladies inflammatoires chroniques comme l'arthrite rhumatoïde ou les IBD.

11. Composition pharmaceutique selon la revendication 6 utile dans le traitement des achondroplasies (ACH), des hypochondroplasies (HCH) et des TD (Thanatophoric dysplasia).

12. Utilisation d'un composé de formule I selon la revendication 1 pour la préparation d'une composition pharmaceutique utile dans le traitement des maladies nécessitant une modulation des b-FGF's.

## Patentansprüche

1. Verbindungen der Formel I 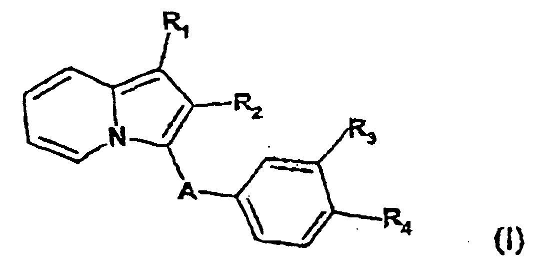 wobei
- R₁ eine Hydroxygruppe, einen linearen oder verzweigten Alkoxyrest von 1 bis 5 Kohlenstoffatomen, eine Carboxygruppe, einen Alkoxycarbonylrest von 2 bis 6 Kohlenstoffatomen oder einen Rest der Formel:
• -NR₅R₆
• -NE-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₈
• -O-Alk-OH
• -O-Alk-C(NH₂)NOH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
darstellt, wobei
• Alk einen linearen oder verzweigten Alkylrest oder einen linearen oder verzweigten Alkylenrest von 1 bis 5 Kohlenstoffatomen darstellt,
• cAlk einen Cycloalkylrest von 3 bis 6 Kohlenstoffatomen darstellt,
• n eine ganze Zahl von 0 bis 5 bedeutet,
• m eine ganze Zahl von 1 bis 5 bedeutet,
• R₅ und R₆, gleich oder verschieden, jeweils ein Wasserstoffatom, einen linearen oder verzweigten Alkylrest von 1 bis 5 Kohlenstoffatomen oder einen Benzylrest darstellen,
• R₇ ein Wasserstoffatom oder einen Alkylrest von 1 bis 5 Kohlenstoffatomen darstellt,
• R₈ einen Alkylrest von 1 bis 5 Kohlenstoffatomen oder einen Rest -CO-Alk darstellt,
• Ph einen Phenylrest darstellt, gegebenenfalls substituiert mit einem oder mehreren Halogenatomen, mit einem oder mehreren Alkoxyresten von 1 bis 5 Kohlenstoffatomen oder einem oder mehreren Carboxyresten oder mit einem oder mehreren Alkoxycarbonylresten von 2 bis 6 Kohlenstoffatomen,
- R₂ ein Wasserstoffatom, einen Alkylrest von 1 bis 5 Kohlenstoffatomen, einen Halogenalkylrest von 1 bis 5 Kohlenstoffatomen, umfassend 3 bis 5 Halogenatome, einen Cycloalkylrest von 3 bis 6 Kohlenstoffatomen oder einen Phenylrest, gegebenenfalls substituiert mit einem oder mehreren Halogenatomen, mit einem oder mehreren Alkoxyresten von 1 bis 5 Kohlenstoffatomen, einem oder mehreren Carboxyresten oder mit einem oder mehreren Alkoxycarbonylresten von 2 bis 6 Kohlenstoffatomen, darstellt,
- A einen Rest -CO-, -SO- oder -SO₂- darstellt,
- R₃ und R₄, gleich oder verschieden, jeweils ein Wasserstoffatom, einen Alkoxyrest von 1 bis 5 Kohlenstoffatomen, eine Aminogruppe, eine Carboxygruppe, einen Alkoxycarbonylrest von 2 bis 6 Kohlenstoffatomen, eine Hydroxygruppe, eine Nitrogruppe, eine Hydroxyaminogruppe, einen Rest der Formel
• -Alk-COOR₇
• -NR₅R₆
• -NH-Alk-COOR₇
• -NH-COO-Alk
• -N(R₁₁)-SO₂-Alk-NR₉R₁₀
• -N(R₁₁)-SO₂-Alk
• -N(R₁₁)-Alk-NR₅R₆
• -N(R₁₁)-CO-Alk-NR₉R₁₀
• -N(R₁₁)-CO-Alk
• -N(R₁₁)-CO-CF₃
• -NH-Alk-HetN
• -O-Alk-NR₉R₁₀
• -O-Alk-CO-NR₅R₆
• -O-Alk-HetN
darstellt, wobei n, m, Alk, R₅, R₆ und R₇ die zuvor für R₁ gegebene Bedeutung besitzen, und
• R₉ und R₁₀, gleich oder verschieden, jeweils ein Wasserstoffatom oder einen Alkylrest von 1 bis 5 Kohlenstoffatomen darstellt,
• R₁₁ ein Wasserstoffatom oder einen Rest -Alk-COOR₁₂ darstellt, wobei R₁₂ ein Wasserstoffatom, einen Alkylrest von 1 bis 5 Kohlenstoffatomen oder einen Benzylrest darstellt,
• HetN einen 5- oder 6-gliedrigen Heterocyclus darstellt, umfassend mindestens ein Stickstoffatom und gegebenenfalls ein weiteres Heteroatom, ausgewählt aus Stickstoff und Sauerstoff,
oder R₃ und R₄ zusammen einen ungesättigten 5- bis 6-gliedrigen Heterocyclus bilden, mit der Maßgabe, dass, wenn R₃ einen Alkoxyrest darstellt und R₄ einen Rest -O-Alk-NR₉R₁₀ oder eine Hydroxygruppe darstellt, R₁ kein Wasserstoffatom oder keinen Alkoxyrest darstellt,
gegebenenfalls in der Form von einem ihrer pharmazeutisch verträglichen Salze.

2. Verbindungen der Formel I nach Anspruch 1, wobei
- R₁ eine Hydroxygruppe, einen linearen oder verzweigten Alkoxyrest von 1 bis 5 Kohlenstoffatomen, eine Carboxygruppe, einen Alkoxycarbonylrest von 2 bis 6 Kohlenstoffatomen oder einen Rest der Formel
• -NR₅R₆
• -NH-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₈
• -O-Alk-OH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
darstellt, wobei
• Alk einen Alkylrest oder einen linearen oder verzweigten Alkylenrest von 1 bis 5 Kohlenstoffatomen darstellt,
• cAlk einen Cycloalkylrest von 3 bis 6 Kohlenstoffatomen darstellt,
• n eine ganze Zahl von 0 bis 5 bedeutet,
• m eine ganze Zahl von 1 bis 5 bedeutet,
• R₅ und R₆, gleich oder verschieden, jeweils ein Wasserstoffatom, einen linearen oder verzweigten Alkylrest von 1 bis 5 Kohlenstoffatomen oder einen Benzylrest darstellen,
• R₇ ein Wasserstoffatom oder einen Alkylrest von 1 bis 5 Kohlenstoffatomen darstellt,
• R₈ einen Alkylrest von 1 bis 5 Kohlenstoffatomen oder einen Rest -CO-Alk darstellt,
• Ph einen Phenylrest darstellt, gegebenenfalls substituiert mit einem oder mehreren Halogenatomen, mit einem oder mehreren Alkoxyresten von 1 bis 5 Kohlenstoffatomen oder mit einem oder mehreren Carboxyresten oder mit einem oder mehreren Alkoxycarbonylresten von 2 bis 6 Kohlenstoffatomen,
- R₂ einen Alkylrest von 1 bis 5 Kohlenstoffatomen, einen Trifluormethylrest, einen Cycloalkylrest von 3 bis 6 Kohlenstoffatomen oder einen Phenylrest, gegebenenfalls substituiert mit einem oder mehreren Halogenatomen, mit einem oder mehreren Alkoxyresten von 1 bis 5 Kohlenstoffatomen, mit einem oder mehreren Carboxyresten oder mit einem oder mehreren Alkoxycarbonylresten von 2 bis 6 Kohlenstoffatomen, darstellt,
- A einen Rest -CO-, -SO₂- darstellt,
- R₃ und R₄, gleich oder verschieden, jeweils ein Wasserstoffatom, einen Alkoxyrest von 1 bis 5 Kohlenstoffatomen, eine Aminogruppe, eine Carboxygruppe, einen Alkoxycarbonylrest von 2 bis 6 Kohlenstoffatomen, eine Nitrogruppe, eine Hydroxyaminogruppe, einen Rest der Formel
• -Alk-COOR₇
• -NR₅R₆
• -NH-Alk-COOR₇
• -NH-COO-Alk
• -N(R₁₁)-SO₂-Alk-NR₉R₁₀
• -N(R₁₁)-SO₂-Alk
• -N(R₁₁)-Alk-NR₅R₆
• -N(R₁₁)-CO-Alk-NR₉R₁₀
• -N(R₁₁)-CO-Alk
• -N(R₁₁)-CO-CF₃
• -NH-Alk-HetN
darstellen, wobei n, m, Alk, R₅, R₆ und R₇ die zuvor für R₁ gegebene Bedeutung besitzen, und
• R₉ und R₁₀, gleich oder verschieden, jeweils ein Wasserstoffatom oder einen Alkylrest von 1 bis 5 Kohlenstoffatomen darstellen,
• R₁₁ ein Wasserstoffatom oder einen Rest -Alk-COOR₁₂ darstellt, wobei R₁₂ ein Wasserstoffatom, einen Alkylrest von 1 bis 5 Kohlenstoffatomen oder einen Benzylrest darstellt,
• HetN einen 5- oder 6-gliedrigen Heterocyclus darstellt, umfassend mindestens ein Stickstoffatom und gegebenenfalls ein weiteres Heteroatom, ausgewählt aus Stickstoff und Sauerstoff
gegebenenfalls in der Form von einem ihrer pharmazeutisch verträglichen Salze.

3. Verbindungen der Formel I nach einem der Ansprüche 1 oder 2, wobei
- R₁ einen Alkoxyrest von 1 bis 5 Kohlenstoffatomen, eine Carboxygruppe, einen Rest -O-Alk-COOH, wobei Alk einen Alkylenrest von 1 bis 5 Kohlenstoffatomen darstellt, einen Rest der Formel -O-Alk-Ph, wobei Alk einen Alkylenrest von 1 bis 5 Kohlenstoffatomen darstellt und Ph einen Phenylrest, gegebenenfalls substituiert mit einem oder mehreren Halogenatomen oder mit einem oder mehreren Alkoxyresten von 1 bis 5 Kohlenstoffatomen oder mit einem oder mehreren Carboxyresten, darstellt, einen Rest der Formel -NH-CO-Ph, einen Rest der Formel -NH-SO₂-Ph oder einen Rest der Formel -NH-CO-NH-Ph darstellt,
- R₂ einen Alkylrest von 1 bis 5 Kohlenstoffatomen darstellt,
- A einen Rest -CO- darstellt,
- R₃ und R₄, die verschieden sind, jeweils ein Wasserstoffatom, einen Alkoxyrest von 1 bis 5 Kohlenstoffatomen, eine Aminogruppe, eine Carboxygruppe, einen Alkoxycarbonylrest von 2 bis 6 Kohlenstoffatomen darstellen,
gegebenenfalls in der Form eines ihrer pharmazeutisch verträglichen Salze.

4. Verbindung der Formel I nach Anspruch 1 ausgewählt unter den folgenden Verbindungen:
- (4-Amino-3-methoxyphenyl)(1-methoxy-2-methylindolizin-3-yl)methanon
- 3-(4-Amino-3-methoxybenzoyl)-2-methylindolizin-1-yl-carbonsäure
- 2-([3-(4-Amino-3-methoxybenzoyl)-2-methylindolizin-1-yl]oxy)essigsäure
- (4-Amino-3-methoxyphenyl) {2-[(4-chlorbenzyl)oxyl-2-methylindolizin-3-yl}methanon
- (4-Amino-3-methoxyphenyl){1-[(3-methoxybenzyl)oxy)-2-methylindolizin-3-yl}methanon
- 4-({[3-(4-Amino-3-methoxybenzoyl)-2-methylindolizin-1-yl]oxy}methyl) benzoesäure
- 3-(4-Carboxybenzoyl)-2-methylindolizin-1-yl-carbonsäure
- 3-[(1-Methoxy-2-methylindolizin-3-yl-carbonyl]methylbenzoat
- 4-[(1-Methoxy-2-methylindolizin-3-yl)carbonyl]benzoesäure
- 2-Amino-5-[(1-methoxy-2-methylindolizin-3-yl)carbonyl]benzoesäure
- 2-Amino-5-({1-[(3-methoxybenzoyl)amino]-2-methylindolizin-3-yl}carbonyl)benzoesäure
- 2-Amino-5-({2-methyl-1-[(3,4,5-trimethoxybenzoyl)amino]indolizin-3-yl} carbonyl)benzoesäure
- 2-Amino-5-({1-{[(3-methoxyphenyl)sulfonyl]amino}-2-methylindolizin-3-yl} carbonyl)benzoesäure
gegebenenfalls in der Form von einem ihrer pharmazeutisch verträglichen Salze.

5. Verfahren zur Herstellung der Verbindungen der Formel I nach den Ansprüchen 1 bis 4, **dadurch gekennzeichnet, dass**
A) ein Indolizinderivat der Formel II 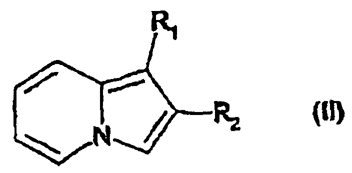 wobei R₁ und R₂ die für Formel (I) angegebene Bedeutung besitzen, R₂ allerdings kein Wasserstoffatom oder keinen Alkyhalogenidrest darstellt,
mit einem Derivat der Formel (III) 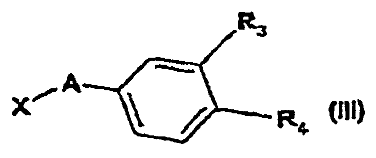 wobei X ein Halogenatom darstellt und R₃ oder R₄, gleich oder verschieden, jeweils ein Wasserstoffatom, eine Nitrogruppe, einen Trifluoracetamidorest oder einen Alkoxycarbonylrest mit 2 bis 6 Kohlenstoffatomen bedeuten, kondensiert wird, um die Verbindungen der Formel Ia, Id oder Ik zu erhalten 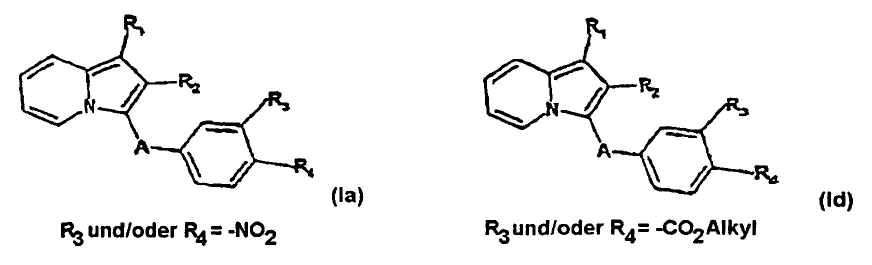 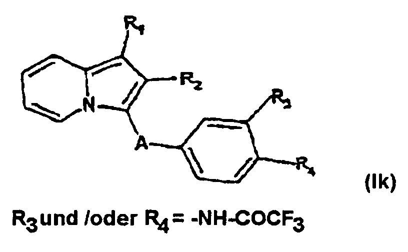 und anschließend
a) die Verbindungen der Formel Ia einer Reduktion unterzogen werden, um die Verbindungen der Formel Ib zu erhalten, 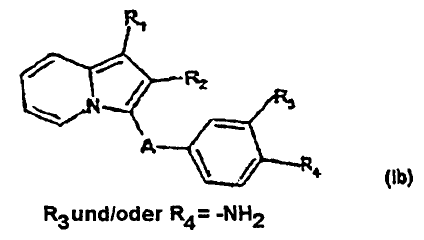 wobei R₃ und/oder R₄ eine Aminogruppe darstellen, wobei anschließend die Verbindungen der Formel Ib
• der Wirkung eines Alkylhalogenids unterzogen werden, um die Verbindungen der Formel I zu erhalten, für die R₄ und/oder R₃ einen Rest -NR₅R₆ (wobei R₅ ein Wasserstoffatom darstellt und R₆ einen Alkylrest mit 1 bis 5 Kohlenstoffatomen darstellt), einen Rest -NH-Alk-NR₅R₆ oder einen Rest -NH-Alk-COOR₇ (wobei R₇ kein Wasserstoffatom darstellt) darstellen, von denen ausgehend man durch spätere Verseifung die Verbindungen der Formel I erhält, für die R₄ und/oder R₃ einen Rest -NH-Alk-COOR₇ darstellen, wobei R₇ ein Wasserstoffatom darstellt,
oder
• einer Acylierung unterzogen werden, um die Verbindungen der Formel I zu erhalten, für die R₄ und/oder R₃ einen Rest -NH-CO-Alk oder einen Rest -NH-CO-Alk-NR₉R₁₀ darstellen, die anschließend einer Alkylierungsreaktion unterzogen werden, um einen Rest -N(R₁₁)-CO-Alk oder einen Rest -N(R₁₁)-CO-Alk-NR₉R₁₀ zu erhalten, wobei R₁₁ einen Rest -Alk-COOR₁₂ darstellt, wobei R₁₂ kein Wasserstoffatom darstellt, wobei die letzteren Verbindungen anschließend gegebenenfalls einer Verseifung unterzogen werden, um die Verbindungen der Formel I zu erhalten, für die R₄ und/oder R₃ einen Rest -N(R₁₁)-CO-Alk oder einen Rest -N(R₁₁)-CO-Alk-NR₉R₁₀ darstellen, wobei R₁₁ einen Rest -Alk-COOH darstellt
oder
• einer Sulfonylierung unterzogen werden, um die Verbindungen der Formel I zu erhalten, für die R₄ und/oder R₃ einen Rest -NH-SO₂-Alk oder einen Rest -NH-SO₂-Alk-NR₉R₁₀ darstellen, die anschließend einer Alkylierungsreaktion unterzogen werden, um einen Rest -N(R₁₁)-SO₂-Alk oder einen Rest -N(R₁₁)-SO₂-Alk-NR₉R₁₀ zu erhalten, wobei R₁₁ einen Rest -Alk-COOR₁₂ darstellt, wobei R₁₂ kein Wasserstoffatom darstellt, wobei die letzteren Verbindungen anschließend gegebenenfalls einer Verseifung unterzogen werden, um die Verbindungen der Formel I zu erhalten, für die R₄ und/oder R₃ einen Rest -N(R₁₁)-SO₂-Alk oder einen Rest -N(R₁₁)- SO₂-Alk -NR₉R₁₀ darstellen, wobei R₁₁ einen Rest -Alk-COOH darstellt.
b) die Verbindungen der Formel Id, wobei R₃ und/oder R₄ einen Alkoxycarbonylrest darstellen, einer Verseifung unterzogen werden, um die Verbindungen der Formel I zu erhalten, wobei R₃ und/oder R₄ eine Carboxygruppe darstellen,
oder
c) wenn R₁ einen Benzyloxyrest darstellt, die Verbindungen der Formel Ia der Wirkung von Trifluoressigsäure unterzogen werden oder die Verbindungen der Formel Id einer Hydrierung unterzogen werden, um die Verbindungen der Formel If zu erhalten 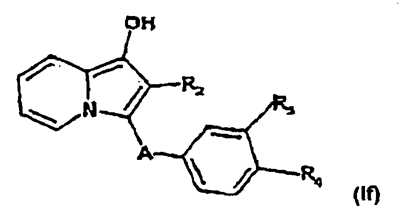 wobei R₃ und/oder R₄ die vorstehend gegebenen Bedeutungen aufweisen, und dass anschließend die Verbindungen der Formel If einer O-Alkylierung unterzogen werden, um die Verbindungen der Formel Ig zu erhalten, 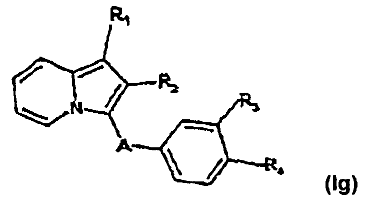 wobei R₃ und/oder R₄ die vorstehend angegebenen Bedeutungen besitzen und R₁ einen linearen oder verzweigten Alkoxyrest von 1 bis 5 Kohlenstoffatomen, einen Rest -O-(CH₂)ₙ-cAlk, einen Rest -O-Alk-COOR₇, einen Rest -O-Alk-NR₅R₆, einen Rest -O-(CH₂)ₙ-Ph, einen Rest -O-Alk-O-R₈ darstellen, die, wenn R₈ einen Rest -COCH₃ darstellt, durch spätere Verseifung einen Rest -O-Alk-OH oder einen Rest -O-Alk-CN ergeben können, der durch Behandlung mit Hydroxylamin zu einem Rest -O-Alk-C(NH₂)=NOH führt,
oder
d) wenn R₁ einen Alkoxycarbonylrest darstellt, die Verbindungen der Formel Ia einer Verseifung unterzogen werden, um die Verbindungen der Formel Ih zu erhalten 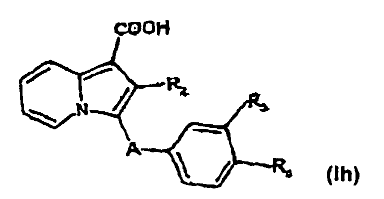 wobei R₃ und/oder R₄ die vorstehend gegebene Bedeutung besitzen, die anschließend der Wirkung eines Aminderivats unterzogen werden, um die Verbindungen der Formel I zu erhalten, wobei R₁ einen Rest -CO-NH-Alk darstellt, oder der Wirkung eines Aminosäurederivats ausgesetzt werden, um die Verbindungen der Formel I zu erhalten, wobei R₁ einen Rest -CO-NH-(CH₂)ₘ-COOR₇ darstellt,
oder
e) wenn R₁ einen Rest -NH-CO₂-tButyl darstellt, die Verbindungen der Formel Ia oder Id
• entweder einer Alkylierung und einer anschließenden Entschützung und gegebenenfalls einer zweiten Alkylierung unterzogen werden, um die Verbindungen der Formel Ii zu erhalten
• oder einer Entschützung und einer anschließenden Acylierung unterzogen werden, um die Verbindungen der Formel Ij zu erhalten, wobei R₅ ein Wasserstoffatom darstellt, und anschließend gegebenenfalls einer Alkylierung unterzogen werden, um die Verbindungen der Formel Ij zu erhalten, wobei R₅ einen Alkylrest darstellt 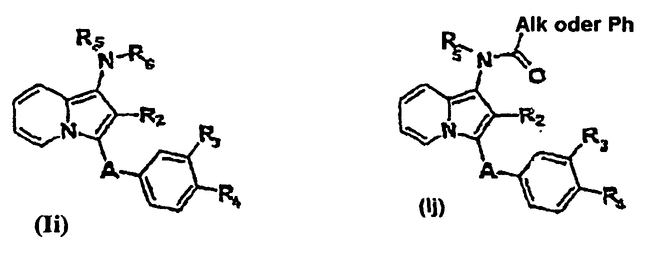
oder
f) wenn R₁ einen Rest -NH-CO₂-tButyl darstellt, die Verbindungen der Formel Ik
• entweder einer Entschützung und einer anschließenden Acylierung unterzogen werden, um die Verbindungen der Formel II zu erhalten 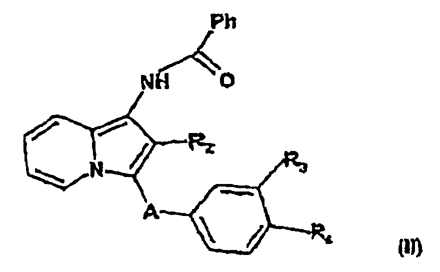
• oder einer Entschützung und anschließend einer Sulfonylierung unterzogen werden, um die Verbindungen der Formel Im zu erhalten 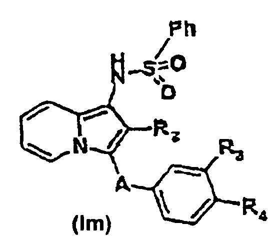
• oder einer Entschützung und anschließend einer Behandlung mit einem Phenylisocyanat unterzogen werden, um die Verbindungen der Formel In zu erhalten, 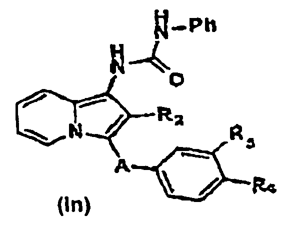
ODER
B) wenn R₁ eine elektronenziehende Gruppe darstellt, R₂ ein Wasserstoffatom oder einen Halogenalkylrest darstellt und A einen Rest -CO- darstellt, das Pyridin mit einem Bromacetophenon der Formel IV reagieren gelassen wird 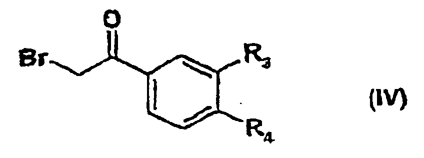 um die Verbindungen der Formel V zu erhalten 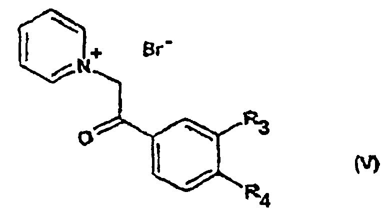 die anschließend einer 1,3-dipolaren Cycloaddition mit Ethylacrylat oder einem halogenierten Derivat von Ethylcrotonat in Gegenwart eines Oxidationsmittels unterzogen werden, um die Verbindungen der Formel Ia zu erhalten, wobei R₁ einen Ethoxycarbonylrest darstellt und R₂ ein Wasserstoffatom oder einen Halogenalkylrest darstellt.

6. Pharmazeutische Zubereitung, die als Wirkstoff eine Verbindung der Formel I nach einem der Ansprüche 1-4, gegebenenfalls in Verbindung mit einem oder mehreren inerten und geeigneten Exzipientien enthält.

7. Pharmazeutische Zubereitung nach Anspruch 6, die bei der Behandlung von Krankheiten geeignet ist, die eine Modulation der b-FGFs benötigen.

8. Pharmazeutische Zubereitung nach Anspruch 6, die bei der Behandlung von Karzinomen mit beträchtlichem Vaskularisationsgrad geeignet ist, wie die Karzinome von Lunge, Brust, Prostata und Ösophagus, Krebsarten, die Metastasen auslösen, wie Darm- und Magenkrebs, Melanome, Gliome, Lymphome und Leukämie.

9. Pharmazeutische Zubereitung nach Anspruch 6, die bei der Behandlung von kardiovaskulären Krankheiten geeignet ist, wie Atherosklerose, postangioplastische Restenose, Krankheiten im Zusammenhang mit Komplikationen, die nach der Einsetzung von endovaskulären Prothesen oder aorto-koronaren Shunts oder anderen Herzhypertrophie-Gefäßtransplantaten auftreten, oder vaskuläre Komplikationen des Diabetes, wie diabetische Retinopathien.

10. Pharmazeutische Zubereitung nach Anspruch 6, die bei der Behandlung von entzündlichen chronischen Erkrankungen, wie rheumatoide Arthritis oder IBD, geeignet ist.

11. Pharmazeutische Zubereitung nach Anspruch 6, die bei der Behandlung von Achondroplasien (ACH), Hypochondroplasien (HCH) und TD (thanatophorische Dysplasie) geeignet ist.

12. Verwendung einer Verbindung der Formel I nach Anspruch 1 zur Herstellung einer pharmazeutischen Zubereitung, die bei der Behandlung von Krankheiten geeignet ist, die eine Modulation der b-FGFs nötig machen.

## Claims

1. Compounds of formula I, 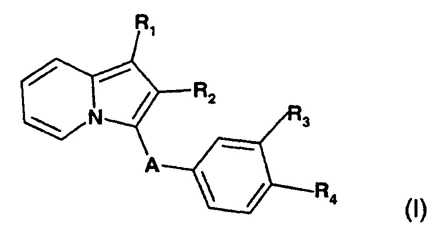 in which
- **R**_{**1**} represents a hydroxyl radical, a linear or branched alkoxy radical of 1 to 5 carbon atoms, a carboxyl radical, an alkoxycarbonyl radical of 2 to 6 carbon atoms or a radical of formula:
• -NR₅R₆
• -NH-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₈
• -O-Alk-OH
• -O-Alk-C(NH₂) :NOH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
in which
• Alk represents an alkyl radical or a linear or branched alkylene radical of 1 to 5 carbon atoms,
• cAlk represents a cycloalkyl radical of 3 to 6 carbon atoms,
• n represents an integer from 0 to 5,
• m represents an integer from 1 to 5,
• R₅ and R₆, which are identical or different, each represent a hydrogen atom, a linear or branched alkyl radical of 1 to 5 carbon atoms or a benzyl radical,
• R₇ represents a hydrogen atom or an alkyl radical of 1 to 5 carbon atoms,
• R₈ represents an alkyl radical of 1 to 5 carbon atoms or a radical -CO-Alk,
• Ph represents a phenyl radical which is optionally substituted with one or more halogen atoms, with one or more alkoxy radicals of 1 to 5 carbon atoms, with one or more carboxyl radicals or with one or more alkoxycarbonyl radicals of 2 to 6 carbon atoms,
- **R**_{**2**} represents a hydrogen atom, an alkyl radical of 1 to 5 carbon atoms, an alkyl halide radical of 1 to 5 carbon atoms containing 3 to 5 halogen atoms, a cycloalkyl radical of 3 to 6 carbon atoms or a phenyl radical which is optionally substituted with one or more halogen atoms, with one or more alkoxy radicals of 1 to 5 carbon atoms, with one or more carboxyl radicals or with one or more alkoxycarbonyl radicals of 2 to 6 carbon atoms,
- **A** represents a radical -CO-, -SO- or -SO₂-,
- **R**_{**3**} and **R**_{**4**}, which are identical or different, each represent a hydrogen atom, an alkoxy radical of 1 to 5 carbon atoms, an amino radical, a carboxyl radical, an alkoxycarbonyl radical of 2 to 6 carbon atoms, a hydroxyl radical, a nitro radical, a hydroxyamino radical, a radical of formula
• -Alk-COOR₇
• -NR₅R₆
• -NH-Alk-COOR₇
• -NH-COO-Alk
• -N (R₁₁)-SO₂-Alk-NR₉R₁₀
• -N(R₁₁)-SO₂-Alk
• -N (R₁₁)-Alk-NR₅R₆
• -N (R₁₁)-CO-Alk-NR₉R₁₀
• -N (R₁₁)-CO-Alk
• -N(R₁₁)-CO-CF₃
• -NH-Alk-HetN
• -O-Alk-NR₉R₁₀
• -O-Alk-CO-NR₅R₆
• -O-Alk-HetN
in which n, m, Alk, R₅, R₆ and R₇ have the meaning given above for R₁, and
• R₉ and R₁₀, which are identical or different, each represent a hydrogen atom or an alkyl radical of 1 to 5 carbon atoms,
• R₁₁ represents a hydrogen atom or a radical -Alk-COOR₁₂ where R₁₂ represents a hydrogen atom, an alkyl radical of 1 to 5 carbon atoms or a benzyl radical,
• HetN represents a 5- or 6-membered heterocycle containing at least one nitrogen atom and optionally another heteroatom chosen from nitrogen and oxygen,
or R₃ and R₄ form together a 5- to 6-membered unsaturated heterocycle, provided, however, that when R₃ represents an alkoxy radical and R₄ represents a radical -O-Alk-NR₉R₁₀ or a hydroxyl radical, R₁ does not represent a hydrogen atom or an alkoxy radical,
optionally in the form of one of their pharmaceutically acceptable salts.

2. Compounds of formula I, according to Claim 1, in which
- **R**_{**1**} represents a hydroxyl radical, a linear or branched alkoxy radical of 1 to 5 carbon atoms, a carboxyl radical, an alkoxycarbonyl radical of 2 to 6 carbon atoms or a radical of formula:
• -NR₅R₆
• -NH-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₈
• -O-Alk-OH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
in which
• Alk represents an alkyl radical or a linear or branched alkylene radical of 1 to 5 carbon atoms,
• cAlk represents a cycloalkyl radical of 3 to 6 carbon atoms,
• n represents an integer from 0 to 5,
• m represents an integer from 1 to 5,
• R₅ and R₆, which are identical or different, each represent a hydrogen atom, a linear or branched alkyl radical of 1 to 5 carbon atoms or a benzyl radical,
• R₇ represents a hydrogen atom or an alkyl radical of 1 to 5 carbon atoms,
• R₈ represents an alkyl radical of 1 to 5 carbon atoms or a radical -CO-Alk,
• Ph represents a phenyl radical which is optionally substituted with one or more halogen atoms, with one or more alkoxy radicals of 1 to 5 carbon atoms, with one or more carboxyl radicals or with one or more alkoxycarbonyl radicals of 2 to 6 carbon atoms,
- **R**_{**2**} represents an alkyl radical of 1 to 5 carbon atoms, a trifluoromethyl radical, a cycloalkyl radical of 3 to 6 carbon atoms or a phenyl radical which is optionally substituted with one or more halogen atoms, with one or more alkoxy radicals of 1 to 5 carbon atoms, with one or more carboxyl radicals or with one or more alkoxycarbonyl radicals of 2 to 6 carbon atoms,
- **A** represents a radical -CO- or -SO₂-,
- **R**_{**3**} and **R**_{**4**}, which are identical or different each represent a hydrogen atom, an alkoxy radical of 1 to 5 carbon atoms, an amino radical, a carboxyl radical, an alkoxycarbonyl radical of 2 to 6 carbon atoms, a nitro radical, a hydroxyamino radical, a radical of formula
• -Alk-COOR₇
• -NR₅R₆
• -NH-Alk-COOR₇
• -NH-COO-Alk
• -N (R₁₁)-SO₂-Alk-NR₉R₁₀
• -N (R₁₁)-SO₂-Alk
• -N (R₁₁)-Alk-NR₅R₆
• -N (R₁₁)-CO-Alk-NR₉R₁₀
• -N(R₁₁)-CO-Alk
• -N(R₁₁)-CO-CF₃
• -NH-Alk-HetN
in which n, m, Alk, R₅, R₆ and R₇ have the meaning given above for R₁, and
• R₉ and R₁₀, which are identical or different, each represent a hydrogen atom or an alkyl radical of 1 to 5 carbon atoms,
• R₁₁ represents a hydrogen atom or a radical -Alk-COOR₁₂ where R₁₂ represents a hydrogen atom, an alkyl radical of 1 to 5 carbon atoms or a benzyl radical,
• HetN represents a 5- or 6-membered heterocycle containing at least one nitrogen atom and optionally another heteroatom chosen from nitrogen and oxygen,
optionally in the form of one of their pharmaceutically acceptable salts.

3. Compounds of formula I, according to either of Claims 1 and 2, in which
- - **R**_{**1**} represents an alkoxy radical of 1 to 5 carbon atoms, a carboxyl radical, a radical -O-Alk-COOH in which Alk represents an alkylene radical of 1 to 5 carbon atoms, a radical of formula -O-Alk-Ph in which Alk represents an alkylene radical of 1 to 5 carbon atoms and Ph represents a phenyl radical which is optionally substituted with one or more halogen atoms or with one or more alkoxy radicals of 1 to 5 carbon atoms or with one or more carboxyl radicals, a radical of formula -NH-CO-Ph, a radical of formula -NH-SO₂-Ph or a radical of formula -NH-CO-NH-Ph,
- **R**_{**2**} represents an alkyl radical of 1 to 5 carbon atoms,
- **A** represents a radical -CO-,
- **R**_{**3**} and **R**_{**4**}, which are different, each represent a hydrogen atom, an alkoxy radical of 1 to 5 carbon atoms, an amino radical, a carboxyl radical or an alkoxycarbonyl radical of 2 to 6 carbon atoms,
optionally in the form of one of their pharmaceutically acceptable salts.

4. Compound of formula I, according to Claim 1, chosen from the following compounds:
- (4-amino-3-methoxyphenyl)(1-methoxy-2-methylindolizin-3-yl)methanone
- 3-(4-amino-3-methoxybenzoyl)-2-methylindolizin-1-yl carboxylic acid
- 2-{[3-(4-amino-3-methoxybenzoyl)-2-methylindolizin-1-yl]oxy}acetic acid
- (4-amino-3-methoxyphenyl){1-[(4-chlorobenzyl)oxy]-2-methylindolizin-3-yl}methanone
- (4-amino-3-methoxyphenyl){1-[(3-methoxybenzyl)oxy]-2-methylindolizin-3-yl}methanone
- 4-({[3-(4-amino-3-methoxybenzoyl)-2-methylindolizin-1-yl]oxy}methyl)benzoic acid
- 3-(4-carboxybenzoyl)-2-methylindolizin-1-yl carboxylic acid
- methyl 3-[(1-methoxy-2-methylindolizin-3-ylcarbonyl]benzoate
- 4-[(1-methoxy-2-methylindolizin-3-yl)carbonyl]benzoic acid
- 2-amino-5-[(1-methoxy-2-methylindolizin-3-yl)carbonyl]benzoic acid
- 2-amino-5-({1-[(3-methoxybenzoyl)amino]-2-methylindolizin-3-yl}carbonyl)benzoic acid
- 2-amino-5-({2-methyl-1-[(3,4,5-trimethoxybenzoyl)amino]indolizin-3-yl}carbonyl)benzoic acid
- 2-amino-5-({1-{[(3-methoxyphenyl)sulphonyl]amino}-2-methylindolizin-3-yl}carbonyl)benzoic acid
optionally in the form of one of its pharmaceutically acceptable salts.

5. Method for preparing the compounds of formula I according to Claims 1 to 4, **characterized in that**
**A)** an indolizine derivative of formula II, 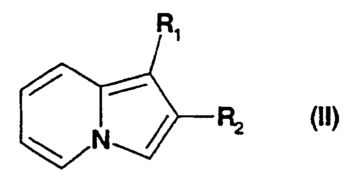 in which R₁ and R₂ have the meaning given for formula I, but R₂ does not represent a hydrogen atom or an alkyl halide radical,
is condensed with a derivative of formula III, 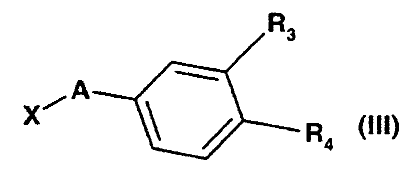 in which X represents a halogen atom and R₃ or R₄, which are identical or different, each represent a hydrogen atom, a nitro radical, a trifluoroacetamido radical or an alkoxycarbonyl radical of 2 to 6 carbon atoms, in order to obtain the compounds of formula Ia, Id or Ik, 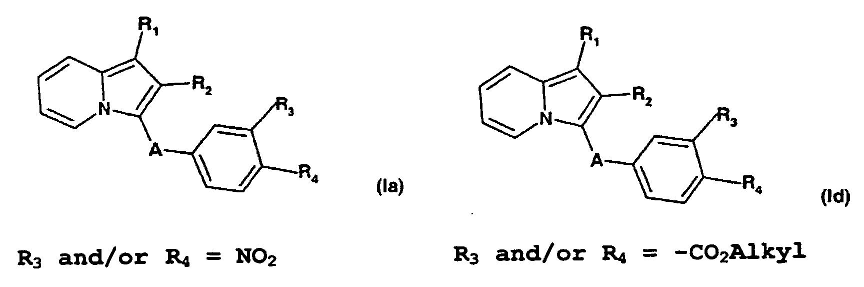 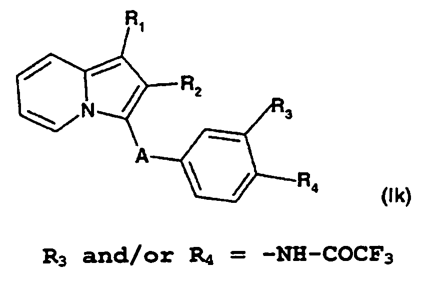 and then,
**a)** the compounds of formula Ia are subjected to a reduction in order to obtain the compounds of formula Ib, 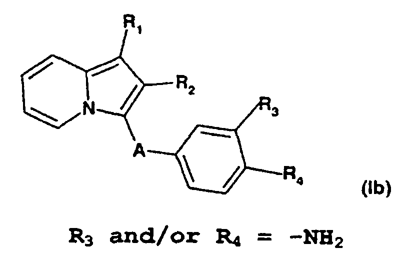 in which R₃ and/or R₄ represent an amino radical, which compounds of formula Ib then
• are subjected to the action of an alkyl halide in order to obtain the compounds of formula I for which R₄ and/or R₃ represent a radical -NR₅R₆ (in which R₅ represents a hydrogen atom and R₆ represents an alkyl radical of 1 to 5 carbon atoms) and a radical -NH-Alk-NR₅R₆ or a radical -NH-Alk-COOR₇ (in which R₇ does not represent a hydrogen atom) from which, by a subsequent saponification, the compounds of formula I are obtained for which R₄ and/or R₃ represent a radical -NH-Alk-COOR₇ in which R₇ represents a hydrogen atom,
or
• are subjected to acylation in order to obtain the compounds of formula I for which R₄ and/or R₃ represent a radical -NH-CO-Alk, or a radical -NH-CO-Alk-NR₉R₁₀, which are then subjected to alkylation in order to obtain a radical -N(R₁₁)-CO-Alk or a radical -N(R₁₁)-CO-Alk-NR₉R₁₀ where R₁₁ represents a radical -Alk-COOR₁₂ in which R₁₂ does not represent a hydrogen atom, the latter compounds are then optionally subjected to saponification in order to obtain the compounds of formula I for which R₄ and/or R₃ represent a radical -N (R₁₁)-CO-Alk or a radical -N(R₁₁)-CO-Alk-NR₉R₁₀ where R₁₁ represents a radical -Alk-COOH,
or
• are subjected to sulphonylation in order to obtain the compounds of formula I for which R₄ and/or R₃ represent a radical -NH-SO₂-Alk or a radical -NH-SO₂-Alk-NR₉R₁₀, which are then subjected to alkylation in order to obtain a radical -N(R₁₁)-SO₂-Alk or a radical -N(R₁₁)-SO₂-Alk-NR₉R₁₀ where R₁₁ represents a radical -Alk-COOR₁₂ in which R₁₂ does not represent a hydrogen atom, the latter compounds are then optionally subjected to saponification in order to obtain the compounds of formula I for which R₄ and/or R₃ represent a radical -N(R₁₁)-SO₂-Alk or a radical -N(R₁₁)-SO₂-Alk-NR₉R₁₀ where R₁₁ represents a radical -Alk-COOH
**b)** the compounds of formula Id in which R₃ and/or R₄ represent an alkoxycarbonyl radical are subjected to saponification in order to obtain the compounds of formula I in which R₃ and/or R₄ represent a carboxyl radical,
**or**
**c)** when R₁ represents a benzyloxy radical, the compounds of formula Ia are subjected to the action of trifluoroacetic acid or the compounds of formula Id to hydrogenation, in order to obtain the compounds of formula If, 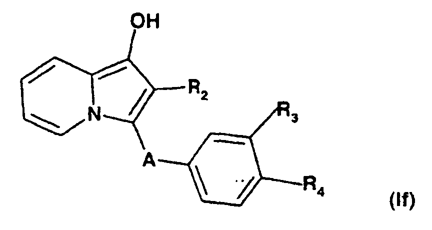 in which R₃ and/or R₄ have the meanings given above,
and then the compounds of formula If are subjected to O-alkylation in order to obtain the compounds of formula Ig, 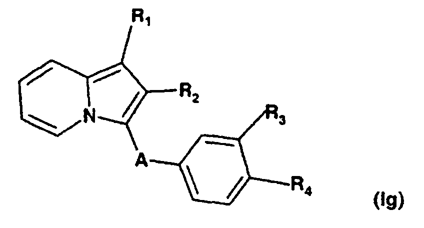 in which R₃ and/or R₄ have the meanings given above, and R₁ represents a linear or branched alkoxy radical of 1 to 5 carbon atoms, a radical -O-(CH₂)ₙ-cAlk, a radical -O-Alk-COOR₇, a radical -O-Alk-NR₅R₆, a radical -O-(CH₂)ₙ-Ph, or a radical -O-Alk-O-R₈ - which, when R₈ represents a radical -COCH₃, can give, by subsequent saponification, a radical -O-Alk-OH - or a radical -O-Alk-CN which, by treatment with hydroxylamine, gives a radical -O-Alk-C(NH₂)=NOH,
**or**
**d)** when R₁ represents an alkoxycarbonyl radical, the compounds of formula Ia are subjected to saponification in order to obtain the compounds of formula Ih, 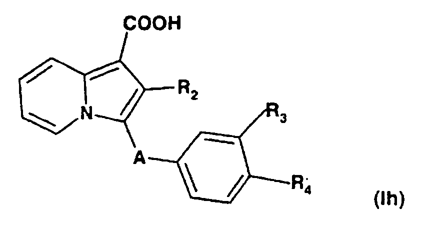 in which R₃ and/or R₄ have the meanings given above, which are then subjected to the action of an amine derivative in order to obtain the compounds of formula I in which R₁ represents a radical -CO-NH-Alk, or to the action of an amino acid derivative in order to obtain the compounds of formula I in which R₁ represents a radical -CO-NH- (CH₂)ₘ-COOR₇
**or**
**e)** when R₁ represents a radical -NH-CO₂tButyl, the compounds of formula Ia or Id are subjected
• either to alkylation followed by deprotection and an optional second alkylation in order to obtain the compounds of formula Ii,
• or to deprotection, followed by acylation in order to obtain the compounds of formula Ij in which R₅ represents a hydrogen atom, followed by an optional alkylation in order to obtain the compounds of formula Ij in which R₅ represents an alkyl radical 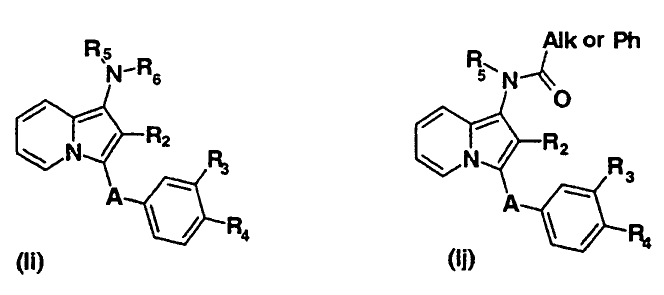
**or**
**f)** when R₁ represents a radical -NH-CO₂tButyl, the compounds of formula **Ik** are subjected
• either to deprotection, followed by acylation in order to obtain the compounds of formula II 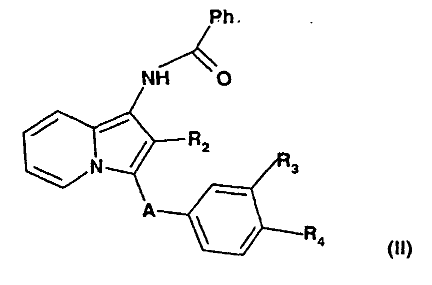
* or to deprotection followed by sulphonylation in order to obtain the compounds of formula Im 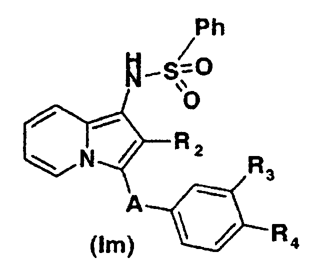
• or to deprotection, followed by a treatment with a phenyl isocyanate in order to obtain the compounds of formula In 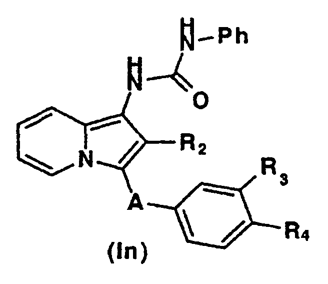
**OR**
**B)** when R₁ represents an electron-attracting group, R₂ represents a hydrogen atom or an alkyl halide radical and A represents a radical -CO-, pyridine is reacted with a bromoacetophenone of formula IV, 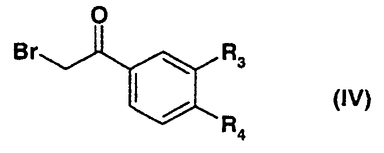 in order to obtain the compounds of formula V, 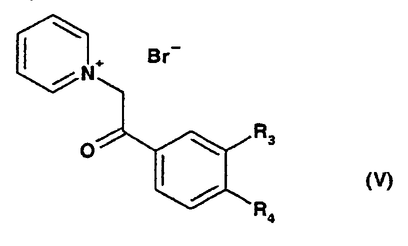 which are then subjected to a 1,3-dipolar cycloaddition with ethyl acrylate or a halogenated derivative of ethyl crotonate in the presence of an oxidizing agent in order to obtain the compounds of formula Ia in which **R**_{**1**} represents an ethoxycarbonyl radical and R₂ represents a hydrogen atom or an alkyl halide radical.

6. Pharmaceutical composition containing, as active ingredient, a compound of formula I, according to any one of Claims 1 to 4, optionally in combination with one or more inert and appropriate excipients.

7. Pharmaceutical composition according to Claim 6, which is useful in the treatment of diseases requiring modulation of b-FGFs.

8. Pharmaceutical composition according to Claim 6, which is useful in the treatment of carcinomas having a high degree of vascularization, such as carcinomas of the lung, breast, prostate and oesophagus, cancers which induce metastases, such as colon cancer and stomach cancer, melanomas, gliomas, lymphomas and leukaemias.

9. Pharmaceutical composition according to Claim 6, which is useful in the treatment of cardiovascular diseases such.as atherosclerosis, post-angioplasty restinosis, diseases linked to complications which appear following the fitting of endovascular prostheses and/or aorto-coronary artery by-pass surgery or other vascular transplants, cardiac hypertrophy, or vascular complications in diabetes such as diabetic retinopathies.

10. Pharmaceutical composition according to Claim 6, which is useful in the treatment of chronic inflammatory diseases such as rheumatoid arthritis or IBDs.

11. Pharmaceutical composition according to Claim 6, which is useful in the treatment of achondroplasia (ACH), hypochondroplasia (HCH) and TD (thanatophoric dysplasia).

12. Use of a compound of formula I according to Claim 1, for the preparation of a pharmaceutical composition which is useful in the treatment of diseases requiring modulation of b-FGFs.
